(19) Europäisches Patentamt
European Patent Office
Office européen des brevets

(11) Publication number: 0 170 006 B1

# (12) EUROPEAN PATENT SPECIFICATION

(45) Date of publication of patent specification: **08.07.92**

(51) Int. Cl.[5]: **C12P 1/06**, C07D 493/22, C07G 11/00, A61K 31/35, C12N 1/20, A01N 63/02, A23K 1/17, //(C12P1/06, C12R1:465,C12N1:20, C12R1:465),(C07D493/22, 313:00,311:00,311:00,307:00)

(21) Application number: **85106844.5**

(22) Date of filing: **03.06.85**

## (54) Method and compositions for helmintic, arthropod ectoparasitic and acaridal infections with novel agents.

(30) Priority: **05.06.84 US 617649**
**05.06.84 US 617650**

(43) Date of publication of application:
**05.02.86 Bulletin 86/06**

(45) Publication of the grant of the patent:
**08.07.92 Bulletin 92/28**

(84) Designated Contracting States:
**AT BE CH FR GB IT LI LU NL SE**

(56) References cited:
**EP-A- 0 058 518**
**EP-A- 0 073 660**
**EP-A- 0 102 721**

(73) Proprietor: **AMERICAN CYANAMID COMPANY**
**1937 West Main Street P.O. Box 60**
**Stamford Connecticut 06904-0060(US)**

(72) Inventor: **Wood, Irwin Bovden**
**211 Elm Avenue**
**Yardley Pennsylvania 19067(US)**
Inventor: **Pankavich, John Anthony**
**20 Sedwick Avenue**
**Trenton New Jersey 08690(US)**
Inventor: **Carter, Guy Thomas**
**8 Prairie Avenue**
**Suffern New York 10901(US)**
Inventor: **Torrey, Margaret Jennings**
**98 Haverstraw Road**
**Suffern New York 10901(US)**
Inventor: **Greenstein, Michael**
**45 Lorna Lane**
**Suffern New York 10901(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 170 006 B1

**CHEMICAL ABSTRACTS, vol. 85, 1976, page 196, abstract no. 118436e, Columbus, Ohio, US; G.T. CARTER: "I. Structures of oligomycin A and C. II. Structures of three isomeric octadecadienoic acids possessing divalent cation ionophoretic activity. III. Insecticidal components of dill and anise plants", && DISS. ABSTR. INT. B 1976, 37(2), 766-7**

**JOURNAL OF ANTIBIOTICS, vol. 36, no. 8, August 1983, pages 980-984, Tokyo, JP; H. MISHIMA et al.: "Milbemycins, a new family of macrolide antibiotics structure determination of milbemycins D,E,F,G,H,J and K"**

**CHEMICAL ABSTRACTS, vol. 106, 1987, page 511, abstract no. 32663s, Columbus, Ohio, US; G.T. CARTER: "Structure determination of oligomycins A and C", & J. ORG. CHEM. 1986, 51(22), 4264-71**

(74) Representative: **Wächtershäuser, Günter, Dr.**
**Tal 29**
**W-8000 München 2(DE)**

## Description

### BACKGROUND OF THE INVENTION

The present invention relates to new antibiotic compounds, collectively identified as LL-F28249, which are produced by the fermentation of a nutrient medium with the strain of the microorganism Streptomyces cyaneogriseus subsp. noncyanogenus LL-F28249, NRRL No. 15773 and to the pharmaceutically and pharmacologically-acceptable salts thereof. The present invention relates to compositions for preventing, treating or controlling helmintic, arthropod ectoparasitic and acaridal infections in warm-blooded animals by administering thereto an effective amount of the agents (compounds) designated LL-F28249$\alpha$, $\beta$, $\gamma$, $\delta$, $\epsilon$, $\zeta$, $\eta$, $\theta$, $\iota$, $\kappa$, $\lambda$, $\mu$, $\nu$ and $\omega$, or mixtures thereof, such as the fermentation broth, or whole mash or the pharmaceutically and pharmacologically-acceptable salts thereof. Plant nematodes also are effectively controlled by use of these agents, mixtures and/or salts. Further, these agents are effective as insecticidal agents, as well.

The diseases described above cause not only devastating effects but also serious economic problems and losses for farmers raising meat-producing animals such as swine, sheep, cattle, goats, rabbits, and poultry. Further, such diseases are a source of great concern for companion animals such as horses, dogs and cats. Although these diseases have been recognized for many years and drugs exist for the treatment and/or prevention of such diseases, the present invention utilizes an entirely new set of active agents, isolated from a previously unknown microorganism, for the prevention, treatment or control of those diseases.

The Journal of Antibiotics, 1983, p. 980 - 984; EP-A-0 102 721; and EP-A-0 058 518 disclose a family of macrolide antibiotics known as Milbemycins, which are obtained by culturing certain Streptomyces strains.

Further U.S. Patent 3,950,360, Aoki et al, April 13, 1976, discloses certain antibiotic substances obtained by culturing a Streptomyces microorganism, said compounds being useful as insecticides and acaracides. But as seen from the characteristics identifying such microorganism, the present microorganism is distinct, and its active components are derived from totally different microorganisms. Further, an entire series of U.S. patents relates to certain compounds produced by the fermentation of Streptomyces avermitilis, a organism distinct from the present one (U.S. Patent 4,171,314, Chabala et al, October 16, 1979; U.S. Patent 4,199,569, Chabala et al, April 22, 1980; U.S. Patent 4,206,205, Mrozik et al, June 3, 1980; U.S. Patent 4,310,519, Albers-Schonberg, January 12, 1982; U.S. Patent 4,333,925, Buhs et al, June 8, 1982). U.S. Patent 4,423,209, Mrozik, December 27, 1983 relates to the process of converting some of these less desirable components to more preferred ones. However, the present active agents identified as LL-F28249$\alpha$, $\beta$, $\gamma$,$\delta$, $\epsilon$, $\zeta$, $\eta$, $\theta$, $\iota$, $\kappa$ , $\lambda$, $\mu$, $\nu$and $\omega$, are derived from the fermentation of a newly discovered and previously uncultivated microorganism. Also, the present compounds and/or the fermentation broth or whole mash of microorganism Streptomyces cyaneogriseus ssp. noncyanogenus NRRL 15773, plus the pharmaceutically and pharmacologically-acceptable salts thereof (collectively referred to as active ingredient), exhibit excellent and effective treatments and/or prevention of these serious diseases of warm-blooded animals.

The full name of the microorganism LL-F28249, NRRL No. 15773, in terms of genus, species, and subspecies is Streptomyces cyaneogriseus noncyanogenus; however, for brevity it is referred to as above written throughout the specification and claims.

The strain is assigned to the genus Streptomyces based upon morphology and cell chemistry (content of the L isomer of diaminopimelic acid). The strain's morphology and physiological data place it close to S. cyaneogriseus, as represented by ISP 5534 (ATCC 27426). Then, comparisons of the formation of gray aerial mycelium soluble pigments on media (Table A) and coiled chains of smooth conidia (3-25 spores per chain) were made. The present strain is negative for blue soluble pigment wherein the comparison strain, ISR 5534, is positives The strains have similar reactions in the ISP carbohydrate utilization tests indicating positive for arabinose, fructose, glucose, rhamnose and xylose, while indicating negative for inositol, mannitol, raffinose and sucrose (ISP 5534 slightly positive). However, the strains differ in several characters (Table B) out of 53 in the Gordon tests. These differences support the creation of a subspecies of S. cyaneogenseus for the present microorganism.

### SUMMARY OF THE INVENTION

It is, therefore an object of this invention to provide novel compounds useful for the control of helmintic, arthropod ectoparasitic and acaridal infections in warm-blooded animals, particularly meat-producing animals, such as poultry, cattle, sheep, swine, rabbits, and companion animals such as horses, dogs and cats.

It is also an object of the present invention to provide novel compositions effective for the control of said diseases in warm-blooded animals.

It is a further object of the present invention to provide a novel method and compositions for the control of insect pests. These and further objects will become more apparent by the description of the invention.

The culture of Streptomyces cyaneogriseus noncyanogenus (LL-F28249 and deposited under NRRL No. 15773) which produces the agents $\alpha$, $\beta$, $\gamma$,$\delta$ ,$\epsilon$ ,$\zeta$ , $\eta$, $\theta$, $\iota$ , $\kappa$, $\lambda$,$\mu$ , $\nu$ and $\omega$components was isolated from mallee sand found in southern Australia.

It has been discovered that the agents useful in the methods and compositions of the present invention are produced by the fermentation of a nutrient medium containing the strain of microorganism, Streptomyces cyaneogriseus noncyanogenus,

TABLE A

| Comparison of F 28249 and ISP 5534 on ISP Morphology Test Media (Numbers are from NBS-ISCC) | | |
|---|---|---|
| Medium | F 28249 | ISP 5534 |
| Yeast-Malt (ISP 2) | A.m.[1] Medium gray (265)<br>V.m. Light tannish (75)<br>Deep yellow-brown<br>S.p. Light brown | Light to medium gray (264-265)<br>Light tannish-white to blackish-blue (188)<br><br>Light brown |
| Inorganic salts<br><br>starch (ISP 4) | A.m. Light olive-gray (112 to medium gray (265<br>V.m. Deep gray to black (266-267)<br>S.p. Grayish-yellowish-brown | Medium gray (265)<br><br>Gray-purplish-blue (204)<br><br>None |
| Glycerol-Asparagine<br><br>(ISP 5) | A.m. 263 (white) to yellowish-gray (93)<br>V.m. Black (267) to light olive brown (96)<br>S.p. Slight brownish | 263 (white) to light gray (264)<br><br>Gray-purplish-blue (203-204)<br><br>Light yellowish-gray |
| Oatmeal (ISP 3) | A.m. Yellow-gray (93)<br>V.m. Colorless<br>S.p. Slight yellowish | None<br>Colorless<br>None |

1 = A.m., aerial mycelium;
V.m. = vegetative mycelium;
S.p. = Soluble pigment

TABLE B

Comparison of Lederle F 28249 with ISP 5534 (Gordon Tests)

| | F28249 | ISP 5534 |
|---|---|---|
| Growth on/at | | |
| Salicin | ± | - |
| 10° | - | + |
| 45° | + | - |
| Production of | | |
| Urease | + | - |
| Decarboxylation of | | |
| Mucate | - | + |
| Acid Production | | |
| Raffinose | - | + |
| Sucrose | - | + |

Both strains have the following reactions:

Positive
- Hydrolysis of casein, hypoxanthine, xanthine, tyrosine, adrenine, potato starch, gelatin, and esculin;
- Production of phosphatase
- Sensitivity to lysozyme
- Decarboxylation of acetate, citrate, lactate, malate, oxalate and propionate
- Acid production from arabinose, cellobiose, dextrin, fructose, galactose, glucose, glycerol, lactose, maltose, mannose, α-methyl D-glucoside, rhamnose, salicin, trehalose.

Negative
- Production of nitrate reductase
- Decarboxylation of benzoate and tartrate
- Acid from adonitol, dulcitol, erythritol, inositol, mannitol, sorbitol, β-methyl-D-xyloside.
- Growth on 5% NaCl

NRRL 15773. These agents include not only the fermentation broth and whole mash of said microorganism but also include the agents, LL-F29249α, LL-F29249β, LL-F29249γ, LL-F29249δ, LL-F29249ε, LL-F29249ζ, LL-F29249η, LL-F29249θ, LL-F29249ι, LL-F29249κ, LL-F29249λ, LL-F29249μ, LL-F29249ν, and LL-F28249ω

The structure and stereochemistry of LL-F29249 have not been fully defined, but the proposed structures are shown below. Component LL-F29249 , is related to Hondamycin (Albimycin) which is disclosed in The Journal of Antibiotics, 22, No. 11, 521-526 (1969).

The structure and stereochemistry of LL-F28249, have not been fully defined, but the proposed structures are shown below. Component LL-F28249 is related to Hondamycin (Albimycin) which is disclosed in The Journal of Antibioticss, 22, No. 11, 521-526 (1969).

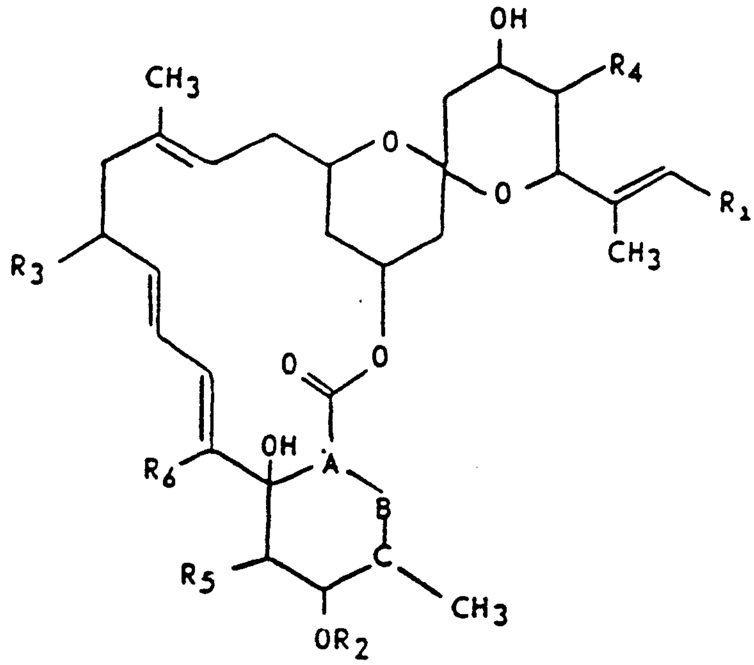

LL-F28249α-μ

| Component | $R_1$ | $R_2$ | $R_3$ | $R_4$ | $R_5$ | $R_6$ | $R_5$+$R_6$ | A-B | B-C |
|---|---|---|---|---|---|---|---|---|---|
| LL-F28249α | $CH(CH_3)_2$ | H | $CH_3$ | $CH_3$ | | | $-O-CH_2-$ | CH-CH | CH=C |
| LL-F28249β | $CH_3$ | H | $CH_3$ | $CH_3$ | | | $-O-CH_2-$ | CH-CH | CH=C |
| LL-F28249γ | $CH_3$ | $CH_3$ | $CH_3$ | $CH_3$ | | | $-O-CH_2-$ | CH-CH | CH=C |
| LL-F28249δ | $CH_3$ | $CH_3$ | $CH_3$ | $CH_3$ | OH | $CH_2OH$ | | CH-CH | CH=C |
| LL-F28249ε | $CH(CH_3)_2$ | H | H | $CH_3$ | | | $-O-CH_2-$ | CH-CH | CH=C |
| LL-F28249ζ | $CH_2CH_3$ | H | $CH_3$ | $CH_3$ | | | $-O-CH_2-$ | CH-CH | CH=C |
| LL-F28249η | $CH(CH_3)_2$ | H | $CH_3$ | $CH_3$ | | | $-O-CH_2-$ | C=CH | CH-CH |
| LL-F28249θ | $CH(CH_3)_2$ | H | $CH_3$ | $CH_2CH_3$ | | | $-O-CH_2-$ | CH-CH | CH=C |
| LL-F28249ι | $CH(CH_3)_2$ | H | $CH_2CH_3$ | $CH_3$ | | | $-O-CH_2-$ | CH-CH | CH=C |
| LL-F28249κ | $CH_3$ | $CH_3$ | $CH_3$ | $CH_3$ | H | $CH_3$ | | CH-CH | CH=C |
| LL-F28249λ | $CH(CH_3)_2$ | $CH_3$ | $CH_3$ | $CH_3$ | | | $-O-CH_2-$ | CH-CH | CH=C |
| LL-F28249μ | $CH(CH_3)_2$ | $CH_3$ | $CH_3$ | $CH_3$ | H | $CH_3$ | | CH-CH | CH=C |

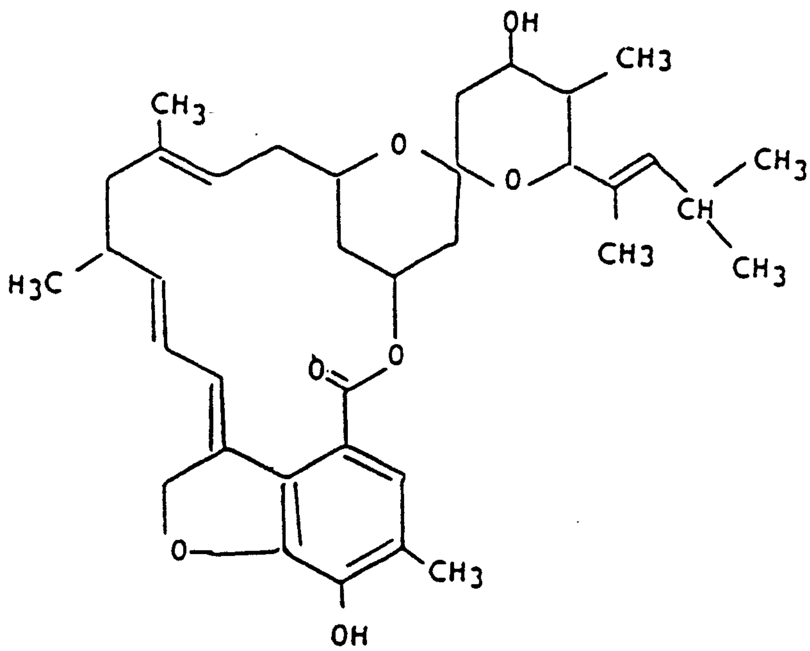

LL-F28249ν

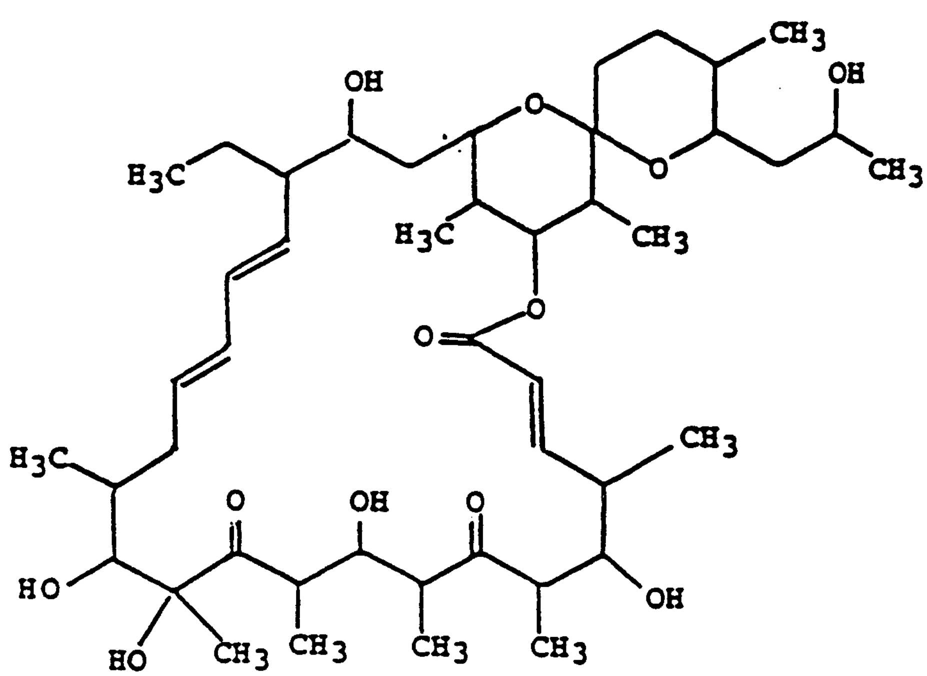

LL-F28249ω

DESCRIPTION OF THE DRAWINGS

FIGURE 1: Characteristic ultraviolet absorption spectrum of compound designated LL-F28249α, NRRL 15773.

FIGURE 2: Characteristic infrared absorption spectrum of compound designated LL-F28249α, NRRL 15773.

FIGURE 3: Characteristic proton nuclear magnetic resonance spectrum of compound designated LL-F28249α, NRRL 15773, in $CDCl_3$ solution.

FIGURE 4: Characteristic carbon-13 nuclear magnetic resonance spectrum of compound designated LL-F28249α, NRRL 15773, in $CDCl_3$ solution.

FIGURE 5: Characteristic electron impact mass spectrum of compound designated LL-F28249α, NRRL 15773.

FIGURE 6: Characteristic ultraviolet absorption spectrum of compound designated LL-F28249β, NRRL 15773.

FIGURE 7: Characteristic infrared absorption spectrum of compound designated LL-F28249β, NRRL 15773.

FIGURE 8: Characteristic proton nuclear magnetic resonance spectrum of compound designated LL-F28249β, NRRL 15773, in $CDCl_3$.

FIGURE 9: Characteristic electron impact mass spectrum of compound designated LL-F28249β, NRRL 15773.

FIGURE 10: Characteristic ultraviolet absorption spectrum of compound designated LL-F28249γ, NRRL 15773.

FIGURE 11: Characteristic infrared absorption spectrum of compound LL-F28249γ, NRRL 15773.

FIGURE 12: Characteristic proton nuclear magnetic resonance spectrum of compound LL-F28249γ,

NRRL 15773, in $CDCl_3$.

FIGURE 13: Characteristic carbon-13 nuclear magnetic resonance spectrum of compound designated LL-F28249$\gamma$, NRRL 15773, in $CDCl_3$.

FIGURE 14: Characteristic electron impact mass spectrum of compound designated LL-F28249$\gamma$, NRRL 15773.

FIGURE 15: Characteristic ultraviolet absorption spectrum of compound designated LL-F28249$\omega$, NRRL 15773.

FIGURE 16: Characteristic infrared absorption spectrum of compound designated LL-F28249$\omega$, NRRL 15773.

FIGURE 17: Characteristic proton nuclear magnetic resonance spectrum of compound designated LL-F28249$\omega$, NRRL 15773, in $CDCl_3$.

FIGURE 18: Characteristic nuclear magnetic resonance spectrum of compound designated LL-F28249$\omega$, NRRL 15773, in $CDCl_3$.

FIGURE 19: Characteristic electron impact mass spectrum of compound designated LL-F28249$\omega$, NRRL 15773.

FIGURE 20: Characteristic ultraviolet absorption spectrum of compound designated LL-F28249$\delta$, NRRL 15773.

FIGURE 21: Characteristic proton nuclear magnetic resonance spectrum of compound designated LL-F28249$\delta$, NRRL 15773, in $CDCl_3$.

FIGURE 22: Characteristic electron inpact mass spectrum of compound designated LL-F28249$\delta$, NRRL 15773.

FIGURE 23: Characteristic ultraviolet absorption spectrum of compound designated LL-F28249$\epsilon$, NRRL 15773.

FIGURE 24: Characteristic proton nuclear magnetic resonance spectrum of compound designated LL-F28249$\epsilon$, NRRL 15773, in $CDCl_3$.

FIGURE 25: Characteristic electron impact mass spectrum of compound designated LL-F28249$\epsilon$, NRRL 15773.

FIGURE 26: Characteristic ultraviolet absorption spectrum of compound designated LL-F28249$\zeta$, NRRL 15773.

FIGURE 27: Characteristic proton nuclear magnetic resonance spectrum of compound designated LL-F28249$\zeta$, NRRL 15773, in $CDCl_3$.

FIGURE 28: Characteristic electron impact mass spectrum of compound designated LL-F28249$\zeta$, NRRL 15773.

FIGURE 29: Characteristic ultraviolet absorption spectrum of compound designated LL-F28249$\eta$, NRRL 15773.

FIGURE 30: Characteristic proton nuclear magnetic resonance spectrum of compound designated LL-F28249$\eta$, NRRL 15773, in $CDCl_3$.

FIGURE 31: Characteristic electron impact mass spectrum of compound designated LL-F28249$\eta$, NRRL 15773.

FIGURE 32: Characteristic ultraviolet absorption spectrum of compound designated LL-F28249$\theta$, NRRL 15773.

FIGURE 33: Characteristic proton nuclear magnetic resonance spectrum of compound designated LL-F28249$\theta$, NRRL 15773, in $CDCl_3$.

FIGURE 34: Characteristic electron impact mass spectrum of compound designated LL-F28249$\theta$, NRRL 15773.

FIGURE 35: Characteristic ultraviolet absorption spectrum of compound designated LL-F28249$\iota$, NRRL 15773.

FIGURE 36: Characteristic proton nuclear magnetic resonance spectrum of compound designated LL-F28249$\iota$, NRRL 15773, in $CDCl_3$.

FIGURE 37: Characteristic electron impact mass spectrum of compound designated LL-F28249$\iota$, 15773.

FIGURE 38: Characteristic carbon - 13 nuclear magnetic resonance spectrum of compound designated LL-F28249$\beta$, NRRL 15773, in $CDCl_3$ solution.

FIGURE 39: Characteristic ultraviolet absorption spectrum of compound designated LL-F28249$\kappa$, NRRL 15773.

FIGURE 40: Characteristic infrared absorption spectrum of compound designated LL-F28249$\kappa$, NRRL 15773.

FIGURE 41: Characteristic electron impact mass spectrum of compound designated LL-F28249$\kappa$,

NRRL 15773.

FIGURE 42: Characteristic proton nuclear magnetic resonance spectrum of compound designated LL-F28249$\kappa$, NRRL 15773.

FIGURE 43: Characteristic carbon - 13 nuclear magnetic resonance spectrum of compound designated LL-F28249$\kappa$, NRRL 15773.

FIGURE 44: Characteristic ultraviolet absorption spectrum of compound designated LL-F28249$\lambda$, NRRL 15773.

FIGURE 45: Characteristic infrared absorption spectrum of compound designated LL-F28249$\lambda$, NRRL 15773.

FIGURE 46: Characteristic electron impact mass spectrum of compound designated LL-F28249$\lambda$, NRRL 15773.

FIGURE 47: Characteristic proton nuclear magnetic resonance spectrum of compound designated LL-F28249$\lambda$, NRRL 15773.

FIGURE 48: Characteristic carbon - 13 nuclear magnetic resonance spectrum of compound designated LL-F28249$\lambda$, NRRL 15773.

FIGURE 49: Characteristic ultraviolet absorption spectrum of compound designated LL-F28249$\mu$, NRRL 15773.

FIGURE 50: Characteristic infrared absorption spectrum of compound designated LL-F28249$\mu$, NRRL 15773.

FIGURE 51: Characteristic electron impact mass spectrum of compound designated LL-F28249$\mu$, NRRL 15773.

FIGURE 52: Characteristic proton nuclear magnetic resonance spectrum of compound designated LL-F28249$\mu$, NRRL 15773.

FIGURE 53: Characteristic ultraviolet absorption spectrum of compound designated LL-F28249$\nu$, NRRL 15773.

FIGURE 54: Characteristic infrared absorption spectrum of compound designated LL-F28249$\nu$, NRRL 15773.

FIGURE 55: Characteristic electron impact mass spectrum of compound designated LL-F28249$\nu$, NRRL 15773.

FIGURE 56: Characteristic proton nuclear magnetic resonance spectrum of compound designated LL-F28249$\nu$, NRRL 15773.

FIGURE 57: Characteristic carbon - 13 nuclear magnetic resonance spectrum of compound designated LL-F28249$\nu$, NRRL 15773.

## DETAILED DESCRIPTION OF THE INVENTION

It has been discovered that the above-mentioned agents, as well as the fermentation broth and whole mash of said microorganism, are especially effective for controlling helmintic, arthropod ectoparasitic and acaridal infections in meat-producing animals such as cattle, sheep, swine, rabbits, poultry, such as chickens, turkeys, ducks, geese, quail, and pheasants and companion animals.

In practice, the present invention involves the method of preventing, controlling or treating said infections, in warm-blooded animals by administering or-gally, parentally, or topically thereto, a prophylactically, pharmaceutically or therapeutically-effective amount of the fermentation broth or whole mash of microorganism Streptomyces cyaneogriseus noncyanogenus, NRRL 15773, the fermentation broth or whole mash of said microorganism containing compounds designated LL-F28249$\alpha$, $\beta$, $\gamma$, $\delta$, $\epsilon$, $\zeta$, $\eta$, $\theta$, $\iota$, $\kappa$, $\lambda$, $\mu$, $\nu$ and $\omega$, compounds designated as LL-F28249$\alpha$, LL-F28249$\beta$, LL-F28249$\gamma$, LL-F28249$\delta$, LL-F28249$\epsilon$, LL-F28249$\zeta$, LL-F28249$\eta$, LL-F28249$\theta$, LL-F28249 $\iota$, LL-F28249$\kappa$, LL-F28249$\lambda$, LL-F28249$\mu$, LL-F28249$\nu$, and LL-F28249$\omega$, as identified and characterized herein, or the pharmaceutically and pharmacologically-acceptable salts thereof (collectively referred to as active ingredient).

Although administration of the compound or fermentation broth/whole mash (hereinafter broth or mash) will generally be most practical in or with the feed or in the drinking water, the above-said compounds, broth or mash, or pharmaceutically and pharmacologically-acceptable salts thereof, may also be administered to individual hosts in the form of tablets, drenches, gels, capsules, or the like, or by injection in the form of a paste, gel, pellet, or solution. These latter methods of administration are, of course, less practical for the treatment of large groups of animals, but they are quite practical for use on a small scale or on an individual basis.

When the agents (antibiotics) LL-F28249$\alpha$, $\beta$, $\gamma$, $\delta$, $\epsilon$, $\zeta$, $\eta$, $\theta$, $\iota$, $\kappa$, $\lambda$, $\mu$, $\nu$ or $\omega$ or the fermentation broth or whole mash of Streptomyces cyaneogriseus noncyanogenus NRRL 15773 are used as prophylactic or

therapeutic treatments of helmintic, arthropod ectoparasitic and acaridal infections, in animals and poultry, generally about 0.05 ppm to 500.0 ppm, and preferably 0.1 ppm to 300 ppm of the agent or broth or mash above-described, administered in the diet or drinking water of the animal, is effective for preventing, controlling, or treating said infections in those animals.

Medicated feeds useful in the method of the present invention are usually prepared by thoroughly admixing about 0.00001% by weight to about 0.01% by weight of the agent (antibiotic) or above-described broth or mash with a nutritionally-balanced feed, as for example, the feed described in the examples hereinafter.

When using the compounds and/or broth or mash of the present invention for the prevention or control of helminths, arthropod ectoparasites and acarides, the active agent is generally first prepared as an animal feed premix. The premix usually contains a relatively high percentage of the active ingredient and is generally blended with the animal's feed just prior to administration. If desired, the feed premix may also be applied as a top dressing for the animal's daily ration.

Feed premixes or concentrates, useful in the practice of the present invention, may be prepared by admixing about 0.1% to 5.0% by weight of the above-identified agents, broth or mash, or pharmaceutically and pharmacologically-acceptable salts thereof, with about 99.9% to 95% by weight of a suitable carrier or diluent.

Carriers suitable for use to make up the feed supplement compositions include the following: alfalfa meal, soybean meal, cottonseed oil meal, linseed oil meal, sodium chloride, calcium carbonate, calcium sulfate, cornmeal, cane molasses, urea, bone meal, corncob meal, rice hull meal, and the like. The carrier promotes an essentially uniform distribution of the active ingredient in the finished feed into which the supplement is blended. It thus performs an important function by ensuring proper distribution of the active ingredient, i.e., about 0.1 ppm to 100 ppm thereof, throughout the feed. This is equivalent to 0.00001% to 0.01%, by weight, of the active ingredient in the finished feed. In practice, usually one or more pounds of premix is added per ton of feed to obtain the desired level of agent (antibiotic) or broth or mash in the finished feed.

If the supplement or premix is used as a top dressing for feed, it likewise helps to ensure uniformity of distribution of the active ingredient across the top of the dressed feed.

Since the compounds of this invention and their pharmaceutically and pharmacologically-acceptable salts are relatively insoluble in water, it is generally desirable, when administering any such compound in the animal's drinking water, to dissolve the active ingredient in an organic solvent such as methanol, ethanol, acetone, DMSO, oleic acid, linoleic acid, propylene glycol, or the like, and admix with the solution a small amount of surfactant and/or dispersing agent to assure solution and/or dispersion of the active ingredient in the animal's drinking water.

Advantageously, where the treatment of a small number of the larger meat-producing animals is required to control parasitic infection therein, the agents LL-F28249$\alpha$, $\beta$, $\gamma$, $\delta$, $\epsilon$, $\zeta$, $\eta$, $\theta$, $\iota$, $\kappa$, $\lambda$, $\mu$, $\nu$ and $\omega$, broth or mash, or pharmaceutically or pharmacologically-acceptable salts thereof may be orally administered, on a daily basis, to the host animal in the form of a medicated gel.

The active ingredients of the invention have also exhibited nematocidal activity against plant nematodes as demonstrated by effectiveness in controlling the free living soil nematode, C. elegans. Compositions containing these active ingredients for controlling plant nematodes can be formulated into either liquids or wettable powders. Liquid compositions include about 5% to 20%, w/w, of the active ingredient (active agent, fermentation broth, whole mash or salts) with appropriate amounts of a solvent such as methanol, ethanol, acetone, acetonitrile, and others, and the remainder water. Wettable powders include about 5% to 20%, w/w, of the active ingredient, about 1% to 10% of surfactant, and inert carriers, such as clays, vermiculite, carbon black or the like. About 0.1 to 1.4 kg per hectare is applied to the foilage of plants, the soil in which they are grown or into the trunks thereof.

These agents also are active as topical insecticides, stomach poisons and systemic insecticides and are especially effective for controlling insects of the orders Lepedoptera, Coleoptera, Homoptera, Deptera and Thysanoptera. Plant mites, acarids, additionally are controlled by the agents of the present invention.

These agents generally are applied as dilute, solid or liquid compositions to the breeding ground, food supply or habitat of such insects and/or acarids. The rate of application to such loci include about 0.01 kg/ha to about 8.0 kg/ha, preferably about 0.05 kg/ha to about 0.5 kg/ha.

Surfactants useful in wettable powders of the present invention include those commonly used for formulations of such wettable powders, preferably alkylbenzene sulfonate sodium salts. Bentonite, clay or mixtures thereof are preferred carriers.

Additionally, the active ingredients of the invention also have demonstrated systemic insecticidal activity against m. ovinus in sheep.

In practice, generally about 0.02mg/kg/day to about 3.0 mg/kg/day is effective for controlling parasitic infections in cattle, sheep, and swine and companion animals. For prolonged use, rates as low as 0.002 mg/kg of body weight/day may be employed.

Also in practice, about 0.1 mg per kg to 100 mg per kg is administered to animals infected with helminths.

The physiochemical characteristics for the $\alpha$, $\beta$, $\gamma$, $\delta$, $\epsilon$, $\zeta$, $\eta$, $\theta$, $\iota$, $\kappa$, $\lambda$, $\mu$, $\nu$ and $\omega$ components of LL-F28249 are described below:

DETAILED DESCRIPTION OF THE INVENTION

The physiochemical characteristics for the $\alpha$, $\beta$, $\gamma$, $\delta$, $\epsilon$, $\zeta$, $\eta$, $\theta$, $\kappa$, $\lambda$, $\mu$,$\nu$ and $\omega$ components of LL-F28249 are described below:

LL-F28249$\alpha$:

1) Molecular weight: 612 (FAB-MS);
2) Molecular formula: $C_{36}H_{52}O_8$;
3) Specific optical rotation: $[\alpha]_D^{26}$ = +133±3° (C 0.3, acetone);
4) Ultraviolet absorption spectrum: as shown in Figure I

$$UV_{MAX}^{CH_3OH} = 244\ nm\ (\varepsilon\ 28{,}000);$$

5) Infrared absorption spectrum: as shown in Figure II (KBr disc): 3439, 2960, 2925, 1714, 1454, 1374, 1338, 1171, 1120, 996, 967 $cm^{-1}$;
6) Proton nuclear magnetic resonance spectrum ($CDCl_3$): as shown in Figure III;
7) Carbon-13 nuclear magnetic resonance spectrum ($CDCl_3$): as shown in Figure IV and described in Table I; and
8) Electron impact mass spectrum: as shown in Figure V with accurate mass measurements and proposed elemental compositions indicated in Table II.

LL-F28249$\beta$:

1) Molecular weight: 584 (FAB-MS);
2) Molecular formula: $C_{34}H_{48}O_8$;
3) Specific optical rotation: $[\alpha]_D^{26}$ = +125° (C 0.30 acetone).
4) Ultraviolet absorption spectrum: as shown in Figure VI

$$UV_{MAX}^{CH_3OH} = 244\ nm\ (\varepsilon\ 25{,}600);$$

5) Infrared absorption spectrum: as shown in Figure VII (KBr disc): 3520, 2910, 1735, 1717, 1450, 1375, 1335, 1180, 1170, 1119, 993, 727 $cm^{-1}$;
6) Proton nuclear magnetic resonance spectrum ($CDCl_3$): as shown in Figure VIII;
7) Carbon-13 nuclear magnetic resonance spectrum ($CDCl_3$): as shown in Figure XXXVIII and described in Table II A; and
8) Electron impact mass spectrum: as shown in Figure IX with accurate mass measurements and proposed elemental compositions indicated in Table III.

LL-F28249$\gamma$:

1) Molecular weight: 598 (FAB-MS);
2) Molecular formula: $C_{35}H_{50}O_8$;
3) Specific optical rotation: $[\alpha]_D^{26}$ = +150±4° (C 0.3, acetone);
4) Ultraviolet absorption spectrum: as shown in Figure X

$$UV_{MAX}^{CH_3OH} = 244 \text{ nm } (\varepsilon\ 27,100);$$

5) Infrared absorption spectrum: as shown in Figure XI (KBr disc): 3510, 2910, 1735, 1715, 1452, 1375, 1338, 1182, 1172, 1119, 995 $cm^{-1}$;
6) Proton nuclear magnetic resonance spectrum ($CDCl_3$): as shown in Figure XII;
7) Carbon-13 nuclear magnetic resonance spectrum ($CDCl_3$): as shown in Figure XIII and described in Table IV; and
8) Electron impact mass spectrum: as shown in Figure XIV with accurate mass measurements and proposed elemental compositions indicated in Table V.

LL-F28249ω:

1) Molecular weight: 806 (FAB-MS);
2) Molecular formula: $C_{45}H_{74}O_{12}$;
3) Specific optical rotation: $[\alpha]_D^{26}$ = -49±3° (C 0.35, methanol);
4) Ultraviolet absorption spectrum: as shown in Figure XV

$$UV_{MAX}^{CH_3OH} = 225 \text{ nm } (\varepsilon 27,400) \quad 232 \text{ nm } (\varepsilon 25,700);$$

5) Infrared absorption spectrum: as shown in Figure XVI (KBr disc): 3480, 2965, 2935, 2880, 1703, 1647, 1458, 1380, 1292, 1223, 1135, 1098, 984 $cm^{-1}$;
6) Proton nuclear magnetic resonance spectrum ($CDCl_3$): as shown in Figure XVII;
7) Carbon-13 nuclear magnetic resonance spectrum ($CDCl_3$): as shown in Figure XVIII and described in Table VI; and
8) Electron impact mass spectrum: as shown in Figure XIX with accurate mass measurements and proposed elemental compositions indicated in Table VII.

LL-F28249δ:

1) Molecular weight: 616 (EI-MS)
2) Molecular formula: $C_{35}H_{52}O_9$
3) HPLC retention volume of 14.0 ml in the system indicated in Table VIII;
4) Ultraviolet absorption spectrum (methanol): as shown in Figure XX;
5) Proton nuclear magnetic resonance spectrum ($CDCL_3$): as shown in Figure XXI; and
6) Electron impact mass spectrum: as shown in Figure XXII.

LL-F28249ε:

1) Molecular weight: 598 (EI-MS)
2) Molecular formula: $C_{35}H_{50}O_8$
3) HPLC retention volume of 14.8 ml in the system indicated in Table VIII;
4) Ultraviolet absorption spectrum (methanol): as shown in Figure XXIII;
5) Proton nuclear magnetic resonance spectrum ($CDCl_3$): as shown in Figure XXIV; and
6) Electron impact mass spectrum: as shown in Figure XXV.

LL-F28249ζ:

1) Molecular weight: 598 (EI-MS)
2) Molecular formula: $C_{35}H_{50}O_8$
3) HPLC retention volume of 16.0 ml in the system indicated in Table VIII;
4) Ultraviolet absorption spectrum (methanol): as shown in Figure XXVI;
5) Proton nuclear magnetic resonance spectrum ($CDCL_3$): as shown in Figure XXVII; and
6) Electron impact mass spectrum: as shown in Figure XXVIII.

LL-F28249η:

1) Molecular weight: 612 (EI-MS)
2) Molecular formula: $C_{36}$ $H_{52}$ $O_8$
3) HPLC retention volume of 23.5 ml in the system indicated in Table VIII;
4) Ultraviolet absorption spectrum (methanol): as shown in Figure XXIX;
5) Proton nuclear magnetic resonance spectrum ($CDCl_3$): as shown in Figure XXX; and
6) Electron impact mass spectrum: as shown in Figure XXXI.

LL-F28249θ:

1) Molecular weight: 626 (EI-MS)
2) Molecular formula: $C_{37}H_{54}O_8$
3) HPLC retention volume of 24.5 ml in the system indicated in Table VIII;
4) Ultraviolet absorption spectrum (methanol): as shown in Figure XXXII;
5) Proton nuclear magnetic resonance spectrum ($CDCl_3$): as shown in Figure XXXIII; and
6) Electron impact mass spectrum: as shown in Figure XXXIV.

LL-F28249ι

1) Molecular weight: 626 (EI-MS)
2) Molecular formula: $C_{37}$ $H_{54}$ $O_8$
3) HPLC retention volume of 26.0 ml in the system indicated in Table VIII;
4) Ultraviolet absorption spectrum (methanol): as shown in Figure XXXV;
5) Proton nuclear magnetic resonance spectrum ($CDCl_3$): as shown in Figure XXXVI; and
6) Electron impact mass spectrum: as shown in Figure XXXVII.

LL-F28249κ:

1) Molecular weight: 584 (EI-MS);
2) Molecular formula: $C_{35}$ $H_{52}$ $O_7$;
3) Specific optical rotation: $[\alpha]^{26}_D = +189°$ -(C 0.165 acetone);
4) Ultraviolet absorption spectrum: as shown in Figure XXXIX

$$UV\ {}^{CH_3OH}_{MAX} = 241nm\ (E20,400);$$

5) Infrared absorption spectrum: as shown in Figure XL (KBr disc);
6) Electron impact mass spectrum: as shown in Figure XLI;
7) Proton nuclear magnetic resonance spectrum ($CDCl_3$); as shown in Figure XLII; and
8) Carbon-13 nuclear magnetic resonance spectrum ($CDCl_3$); as shown in Figure XLIII and described in Table IX.

LL-F28249λ:

1) Molecular weight: 626 (FAB-MS);
2) Molecular formula: $C_{37}$ $H_{54}$ $O_8$;
3) Specific optical rotation: $[\alpha]_D^{26} = +145°$ (C, 0.23 acetone);
4) Ultraviolet absorption spectrum: as shown in Figure XLIV

$$UV\ {}^{CH_3OH}_{MAX} = 244nm\ (E30,000);$$

5) Infrared absorption spectrum: as shown in Figure XLV (KBr disc);
6) Electron impact mass spectrum: as shown in Figure XLVI;
7) Proton nuclear magnetic resonance spectrum ($CDCl_3$); as shown in Figure XLVII; and

8) Carbon-13 nuclear magnetic resonance spectrum ($CDCl_3$); as shown in Figure XLVIII and described in Table X.

LL-F28249μ:

1) Molecular weight: 612 (EI-MS);
2) Molecular formula: $C_{37}H_{56}O_7$;
3) Ultraviolet absorption spectrum: as shown in Figure XLIX

$$UV_{MAX}^{CH_3OH} = 241nm\ (E16,800);$$

4) Infrared absorption spectrum: as shown in Figure L (KBr disc);
5) Electron impact mass spectrum: as shown in Figure LI;
6) Proton nuclear magnetic resonance spectrum ($CDCl_3$); as shown in Figure LII.

LL-F28249ν:

1) Molecular weight: 592 (EI-MS);
2) Molecular formula: $C_{36}H_{48}O_7$:
3) Specific optical rotation: $[\alpha]_D^{26}$ +131°- (C .325, acetone);
4) Ultraviolet absorption spectrum: as shown in Figure LIII

$$UV_{MAX}^{CH_3OH} = 256\ (E20,500);\ 358(E\ 8,830);$$

5) Infrared absorption spectrum: as shown in Figure LIV (KBr disc);
6) Electron impact mass spectrum: as shown in Figure LV;
7) Proton nuclear maagnetic resonance spectrum ($CDCl_3$); as shown in Figure LVI; and
8) Carbon-13 nuclear magnetic resonance spectrum ($CDCl_3$); as shown in Figure LVII, and described in Table XI.

**TABLE I**

**Carbon-13 NMR Data for LL-F28249α**

| Carbon | Chemical Shift[1] (ppm) | Proton Substitution | Carbon | Chemical Shift (ppm) | Proton Substitution |
|---|---|---|---|---|---|
| 1 | 173.4 | q[2] | 18 | 67.8 | CH |
| 2 | 142.8 | CH | 19 | 67.7 | CH |
| 3 | 139.4 | q | 20 | 48.4 | $CH_2$ |
| 4 | 137.7 | q | 21 | 45.7 | CH |
| 5 | 137.3 | q | 22 | 41.1 | $CH_2$ |
| 6 | 137.2 | CH | 23 | 40.7 | $CH_2$ |
| 7 | 130.6 | q | 24 | 36.1 | $CH_2$ |
| 8 | 123.3 | CH | 25 | 36.0 | CH |
| 9 | 120.3[3] | CH | 26 | 35.9 | CH |
| 10 | 118.0 | CH | 27 | 34.7 | $CH_2$ |
| 11 | 99.7 | q | 28 | 26.8 | CH |
| 12 | 80.2 | q | 29 | 22.8[4] | $CH_3$ |
| 13 | 79.3 | CH | 30 | 22.2 | $CH_3$ |
| 14 | 76.7 | CH | 31 | 19.9 | $CH_3$ |
| 15 | 69.3 | CH | 32 | 15.5 | $CH_3$ |
| 16 | 68.5 | CH | 33 | 13.9 | $CH_3$ |
| 17 | 68.4 | $CH_2$ | 34 | 11.0 | $CH_3$ |

[1] Downfield from TMS; $CDCl_3$ solution.

[2] q = quarternary carbon.

[3,4] Two unresolved signals.

TABLE II

| High Resolution Mass Measurements for LL-F28249α | |
|---|---|
| m/z | Elemental Composition |
| 612.3705 | $C_{36}H_{52}O_8$ |
| 594.3543 | $C_{36}H_{50}O_7$ |
| 576.3472 | $C_{36}H_{48}O_6$ |
| 484.3211 | $C_{30}H_{44}O_5$ |
| 482.2648 | $C_{29}H_{38}O_6$ |
| 466.3097 | $C_{30}H_{42}O_4$ |
| 448.2987 | $C_{30}H_{40}O_3$ |
| 442.2375 | $C_{26}H_{34}O_6$ |
| 425.2327 | $C_{26}H_{33}O_5$ |
| 354.2181 | $C_{23}H_{30}O_3$ |
| 314.1877 | $C_{20}H_{26}O_3$ |
| 278.1144 | $C_{15}H_{18}O_5$ |
| 265.1786 | $C_{16}H_{25}O_3$ |
| 248.1405 | $C_{15}H_{20}O_3$ |
| 247.1705 | $C_{16}H_{23}O_2$ |
| 237.1838 | $C_{15}H_{25}O_2$ |
| 219.1740 | $C_{15}H_{23}O$ |
| 151.0753 | $C_9H_{11}O_2$ |

TABLE IIa

| Carbon-13 NMR Data for LL-F28249β | | | |
|---|---|---|---|
| Carbon | Chemical Shift(ppm)* | Carbon | Chemical Shift(ppm) |
| 1 | 173.3 | 18 | 68.3 |
| 2 | 142.6 | 19 | 67.8 |
| 3 | 139.5 | 20 | 67.7 |
| 4 | 137.7 | 21 | 48.4 |
| 5 | 137.3 | 22 | 45.7 |
| 6 | 133.9 | 23 | 41.0 |
| 7 | 123.8 | 24 | 40.8 |
| 8 | 123.4 | 25 | 36.1 |
| 9 | 120.3 | 26 | 35.9 ** |
| 10 | 120.2 | 27 | 34.7 |
| 11 | 118.0 | 28 | 22.3 |
| 12 | 99.7 | 29 | 19.8 |
| 13 | 80.2 | 30 | 15.5 |
| 14 | 79.4 | 31 | 13.8 |
| 15 | 76.7 | 32 | 13.1 |
| 16 | 69.2 | 33 | 10.0 |
| 17 | 68.6 | | |

* Downfield from TMS; $CDCl_3$ solution
** Two unresolved signals

TABLE III

| High Resolution Mass Measurements for LL-F28249$\beta$ | |
|---|---|
| m/z | Elemental Composition |
| 584.3388 | $C_{34}H_{48}O_8$ |
| 566.3306 | $C_{34}H_{46}O_7$ |
| 456.2864 | $C_{28}H_{40}O_5$ |
| 442.2391 | $C_{26}H_{34}O_6$ |
| 438.2780 | $C_{28}H_{38}O_4$ |
| 425.2331 | $C_{26}H_{33}O_5$ |
| 354.2187 | $C_{23}H_{30}O_3$ |
| 314.1858 | $C_{20}H_{26}O_3$ |
| 278.1168 | $C_{15}H_{18}O_5$ |
| 237.1491 | $C_{14}H_{21}O_3$ |
| 219.1380 | $C_{14}H_{19}O_2$ |
| 209.1534 | $C_{13}H_{21}O_2$ |
| 191.1418 | $C_{13}H_{19}O$ |
| 151.0750 | $C_9H_{11}O_2$ |

TABLE IV

| Carbon-13 NMR Data for LL-F28249$\gamma$ | | | |
|---|---|---|---|
| Carbon | Chemical Shift[1] (ppm) | Carbon | Chemical Shift (ppm) |
| 1 | 173.6 | 19 | 68.3 |
| 2 | 142.4 | 20 | 67.9 |
| 3 | 139.9 | 21 | 57.7 |
| 4 | 137.3 | 22 | 48.5 |
| 5 | 136.0 | 23 | 45.8 |
| 6 | 134.0 | 24 | 41.2 |
| 7 | 123.8 | 25 | 40.8 |
| 8 | 123.6 | 26 | 36.2 |
| 9 | 120.4 | 27 | 36.1 |
| 10 | 119.6 | 28 | 36.0 |
| 11 | 118.5 | 29 | 34.8 |
| 12 | 99.8 | 30 | 22.3 |
| 13 | 80.5 | 31 | 19.9 |
| 14 | 77.8 | 32 | 15.5 |
| 15 | 77.0 | 33 | 13.8 |
| 16 | 76.8 | 34 | 13.1 |
| 17 | 69.3 | 35 | 10.8 |
| 18 | 68.6 | | |

[1]Downfield from TMS; $CDCl_3$ solution.

TABLE V

| High Resolution Mass Measurements for LL-F28249-γ | |
|---|---|
| m/z | Elemental Composition |
| 598.3543 | $C_{35}H_{50}O_8$ |
| 580.3422 | $C_{35}H_{48}O_7$ |
| 562.3292 | $C_{35}H_{46}O_6$ |
| 496.2824 | $C_{30}H_{40}O_6$ |
| 484.2440 | $C_{28}H_{36}O_7$ |
| 478.2687 | $C_{30}H_{38}O_5$ |
| 456.2576 | $C_{21}H_{36}O_6$ |
| 438.2772 | $C_{28}H_{38}O_4$ |
| 425.2341 | $C_{26}H_{33}O_5$ |
| 420.2651 | $C_{28}H_{36}O_3$ |
| 354.2199 | $C_{23}H_{30}O_3$ |
| 314.1875 | $C_{20}H_{26}O_3$ |
| 292.1307 | $C_{16}H_{20}O_5$ |
| 288.2075 | $C_{19}H_{28}O_2$ |
| 248.1397 | $C_{15}H_{20}O_3$ |
| 237.1490 | $C_{14}H_{21}O_3$ |
| 219.1382 | $C_{14}H_{19}O_2$ |
| 209.1544 | $C_{13}H_{21}O_2$ |
| 191.1435 | $C_{13}H_{19}O$ |
| 151.0759 | $C_9H_{11}O_2$ |

TABLE VI

| Carbon-13 NMR Data for LL-F28249ω | | | |
|---|---|---|---|
| Carbon | Chemical Shift[1] (ppm) | Carbon | Chemical Shift (ppm) |
| 1 | 220.7 | 23 | 42.2[2] |
| 2 | 219.6 | 24 | 40.4 |
| 3 | 165.2 | 25 | 38.3 |
| 4 | 148.7 | 26 | 37.6 |
| 5 | 133.1 | 27 | 36.1 |
| 6 | 132.3 | 28 | 34.8 |
| 7 | 132.1 | 29 | 33.5 |
| 8 | 130.2 | 30 | 30.1 |
| 9 | 122.3 | 31 | 26.6 |
| 10 | 100.0 | 32 | 25.4 |
| 11 | 82.9 | 33 | 24.5 |
| 12 | 75.9 | 34 | 23.0 |
| 13 | 73.0 | 35 | 21.1 |
| 14 | 72.7 | 36 | 17.9 |
| 15 | 72.6 | 37 | 14.3 |
| 16 | 72.1 | 38 | 14.2 |
| 17 | 69.0 | 39 | 12.1 |
| 18 | 67.3 | 40 | 11.5 |
| 19 | 63.6 | 41 | 10.9 |
| 20 | 51.4 | 42 | 8.7 |
| 21 | 46.2 | 43 | 8.3 |
| 22 | 45.7 | 44 | 5.7 |

[1]Downfield from TMS; $CDCl_3$ solution.
[2]Two unresolved signals.

TABLE VII

| High Resolution Mass Measurements for LL-F28249ω | |
|---|---|
| m/z | Elemental Composition |
| 462.3350 | $C_{28}H_{46}O_5$ |
| 444.3237 | $C_{28}H_{44}O_4$ |
| 425.2534 | $C_{23}H_{37}O_7$ |
| 407.2439 | $C_{23}H_{35}O_6$ |
| 406.3046 | $C_{25}H_{42}O_4$ |
| 387.2895 | $C_{25}H_{39}O_3$ |
| 337.2010 | $C_{19}H_{29}O_5$ |
| 297.2031 | $C_{17}H_{29}O_4$ |
| 279.1944 | $C_{17}H_{27}O_3$ |
| 261.1851 | $C_{17}H_{25}O_2$ |
| 253.1797 | $C_{15}H_{25}O_3$ |
| 235.1697 | $C_{15}H_{23}O_2$ |
| 224.1754 | $C_{14}H_{24}O_2$ |
| 209.1530 | $C_{13}H_{21}O_2$ |
| 207.1744 | $C_{14}H_{23}O$ |
| 184.1458 | $C_{11}H_{20}O_2$ |
| 179.1048 | $C_{11}H_{15}O_2$ |
| 173.1205 | $C_9H_{17}O_3$ |
| 167.1051 | $C_{10}H_{15}O_2$ |
| 155.1069 | $C_9H_{15}O_2$ |

TABLE VIII

HPLC Retention Volumes for LL-F28249α, δ, ε, ζ, η, θ and ι

| Compound | Retention Volume*(ml) |
|---|---|
| LL-F28249 α | 19.8 |
| LL-F28249 δ | 14.0 |
| LL-F28249 ε | 14.8 |
| LL-F28249 ζ | 16.0 |
| LL-F28249 η | 23.5 |
| LL-F28249 θ | 24.5 |
| LL-F28249 ι | 26.0 |

*System includes a column 3.9mm x 30cm packed with $C_{18}$ reverse phase packing developed with methanol:water (80:20) at 1.0 ml/minute, detection was by absorbance at 254 nm.

TABLE IX

| Carbon-13 NMR Data for LL-F28249χ | | | |
|---|---|---|---|
| Carbon | Chemical Shift(ppm)* | Carbon | Chemical Shift(ppm) |
| 1 | 173.9 | 19 | 56.7 |
| 2 | 140.7 | 20 | 48.4 |
| 3 | 138.3 | 21 | 47.7 |
| 4 | 136.6 | 22 | 41.1 |
| 5 | 136.5 | 23 | 40.6 |
| 6 | 133.8 | 14 | 37.1 |
| 7 | 124.7 | 25 | 36.3 |
| 8 | 124.4 | 26 | 36.0 |
| 9 | 123.8 | 27 | 35.9 |
| 10 | 120.1 | 28 | 34.6 |
| 11 | 118.5 | 29 | 22.0 |
| 12 | 99.7 | 30 | 19.3 |
| 13 | 77.2 | 31 | 16.0 |
| 14 | 76.6** | 32 | 13.8 |
| 15 | 76.5 | 33 | 13.3 |
| 16 | 69.3 | 34 | 13.1 |
| 17 | 68.6 | 35 | 10.7 |
| 18 | 67.3 | | |

* Downfield from TMS; $CDCl_3$ solution.
** Coincident with $CDCl_3$ signals.

TABLE X

| Carbon-13 NMR Data for LL-F28249λ | | | |
|---|---|---|---|
| Carbon | Chemical Shift(ppm)* | Carbon | Chemical Shift(ppm) |
| 1 | 173.6 | 19 | 68.3 |
| 2 | 142.5 | 20 | 67.9 |
| 3 | 139.8 | 21 | 57.8 |
| 4 | 137.4 | 22 | 48.6 |
| 5 | 137.2 | 23 | 45.8 |
| 6 | 136.0 | 24 | 41.2 |
| 7 | 130.7 | 25 | 40.9 |
| 8 | 123.6 | 26 | 36.1 ** |
| 9 | 120.3 | 27 | 36.0 |
| 10 | 119.7 | 28 | 34.9 |
| 11 | 118.6 | 29 | 26.9 |
| 12 | 99.8 | 30 | 23.0 ** |
| 13 | 80.5 | 31 | 22.4 |
| 14 | 77.7 | 32 | 20.0 |
| 15 | 77.6 | 33 | 15.7 |
| 16 | 76.7 | 34 | 14.0 |
| 17 | 69.3 | 35 | 11.1 |
| 18 | 68.6 | | |

* Downfield from TMS; $CDCl_3$ solution.
** Two unresolved signals.

TABLE XI

| Carbon-13 NMR Data for LL-F28249ν | | | |
|---|---|---|---|
| Carbon | Chemical Shift(ppm)* | Carbon | Chemical Shift(ppm) |
| 1 | 167.4 | 18 | 69.4 |
| 2 | 150.5 | 19 | 68.7 |
| 3 | 142.9 | 20 | 68.3 |
| 4 | 142.0 | 21 | 48.4 |
| 5 | 137.2 ** | 22 | 41.0 ** |
| 6 | 132.1 | 23 | 35.9 |
| 7 | 130.7 | 24 | 35.6 |
| 8 | 125.8 | 25 | 35.5 |
| 9 | 125.5 | 26 | 34.4 |
| 10 | 124.2 | 27 | 29.7 |
| 11 | 123.7 | 28 | 26.8 |
| 12 | 123.2 | 29 | 22.9 |
| 13 | 121.3 | 30 | 22.8 |
| 14 | 118.0 | 31 | 22.1 |
| 15 | 100.0 | 32 | 15.3 |
| 16 | 76.7 | 33 | 13.9 |
| 17 | 74.6 | 34 | 11.0 |

* Downfield from TMS; $CDCl_3$ solution.
** Two unresolved signals.

TABLE XII

| Chromatographic Data | | |
|---|---|---|
| Component | TLC * Relative Rf | HPLC ** Retention Time(minutes) |
| α | 1.00 | 13.8 |
| β | .797 | 9.3 |
| γ | 1.42 | 12.6 |
| δ | .758 | 10.4 |
| ε | 1.06 | 10.9 |
| ζ | 1.12 | 11.5 |
| η | 1.03 | 16.2 |
| θ | 1.27 | 17.3 |
| ι | 1.27 | 18.2 |
| κ | 1.83 | 24.7 |
| λ | 1.56 | 19.1 |
| μ | 1.92 | 38.0 |
| ν | 1.95 | 42.3 |
| ω | .212 | 7.1 |

* Analtech Silica Gel GHLF250μ developed with ethyl acetate:methylene chloride (1:3), detection by charring with $H_2SO_4$.
** Altex Ultrasphere ODS 5μ 4.6mmx25cm developed with 85% methanol in water at 1.0 ml/minute, detection by absorbance at 254 nm.

The new agents designated LL-F28249α,β ,γ ,δ ε, ζ, η, θ, ι, κ, λ, μ, νand ω are formed during the cultivation, under controlled conditions of Streptomyces cyaneogriseus noncyanogenus, NRRL 15773.

This organism is maintained in the culture collection of the Medical Research Division, American Cyanamid Company, Pearl River, New York as culture number LL-F28249. A viable culture of this new microorganism has been deposited with the Patent Culture Collection Laboratory, Northern Regional

Research Center, U. S. Department of Agriculture, Peoria, Illinois 61604, and has been added to its permanent collection. It is freely available to the public in this depository under its accession number NRRL 15773.

For the production of these new agents the present invention is not limited to this particular organism. In fact, it is desired and intended to include the use of naturally-occurring mutants of this organism, as well as induced mutants produced from this organism by various mutagenic means known to those skilled in the art, such as exposure to nitrogen mustard, X-ray radiation, ultraviolet radiation, N'-methyl-N'-nitro-N-nitrosoguanidine, actinophages and the like. It is also desired and intended to include inter- and intraspecific genetic recombinants produced by genetic techniques known to those skilled in the art such as for example, conjugation, transduction and genetic engineering techniques.

General Fermentation Conditions

Cultivation of Streptomyces cyaneogriseus non-cyaneogenus,NRRL 15773 may be carried out in a wide variety of liquid culture media. Media which are useful for the production of agents LL-F28249$\alpha$, $\beta$, $\gamma$, $\delta$,$\epsilon$ ,$\zeta$ ,$\eta$ ,$\theta$ , $\iota$, $\kappa$, $\gamma$,$\mu$,$\nu$ and $\omega$ include an assimilable source of carbon, such as dextrin, sucrose, molasses, glycerol, etc.; an assimilable source of nitrogen such as protein, protein hydrolysate, polypeptides, amino acids, corn steep liquor, etc.; and inorganic anions and cations, such as potassium, sodium, ammonium, calcium, sulfate, carbonate, phosphate, chloride, etc. Trace elements such as boron, molybdenum, copper, etc., are supplied as impurities of other constituents of the media. Aeration in tanks and bottles is supplied by forcing sterile air through or onto the surface of the fermenting medium: Further agitation in tanks is provided by a mechanical impeller. An antifoam agent such as silicone oil may be added as needed.

Example 1

Inoculum Preparation

A typical medium used to grow the various stages of inoculum was prepared according to the following formula:

| | |
|---|---|
| Dextrose | 1.0% |
| Dextrin | 2.0% |
| Yeast extract | 0.5% |
| NZ amine | 0.5% |
| Calcium carbonate | 0.1% |
| Water qs | 100% |

This medium was sterilized. A 100 ml portion of this sterile medium, in a flask, was inoculated with mycelial scrapings from an agar slant of Streptomyces cyaneogriseus noncyanogenus NRRRL 15773. The medium was then agitated vigorously on a rotary shaker for 48-72 hours at 28°C providing primary inoculum. This primary inoculum was then used to inoculate one liter of the above sterile medium, which was then grown aerobically at 28°C for 48 hours providing secondary inoculum.

Example 2

Fermentation

A fermentation medium of the following formulation was prepared.

| | |
|---|---|
| Dextrin | 1.0% |
| Soya peptone | 1.0% |
| Molasses | 2.0% |
| Calcium carbonate | 0.1% |
| Water qs | 100% |

This medium was-sterilized and then a 30 liter portion was inoculated with one liter of secondary

inoculum prepared as described in Example 1. The fermentation was conducted at 30 ° C, with a sterile air flow of 30 liters per minute, backpressure of 8 psig and agitation by an impeller operated at 500 rpm for 91 hours at which time the mash was harvested.

Example 3

Isolation of LL-F28249α, β andγ

A total of 26 liters of whole harvest mash, prepared as described in Example 2 was mixed with 1500 g of diatomaceous earth and filtered. The mycelial cake was washed with 5 liters of water and the filtrate and wash discarded. The mycelial cake was mixed with 10 liters of methanol for one hour, then filtered and washed with 5 liters of methanol. The methanol extract and methanol wash were combined and evaporated to an aqueous residue of about 1-2 liters. This aqueous residue was mixed with twice its volume of methylene chloride and mixed for 1/2 hour. The methylene chloride phase was separated and then concentrated to a syrup giving 27 g of crude material.

This 27 g of crude material was dissolved in a mixture of methylene chloride and methanol, filtered through cotton and anhydrous sodium sulfate and then evaporated, giving 7.0 g of an oil.

A 170 g portion of silica gel was slurried in 12.5% ethyl acetate in methylene chloride and poured to form a column 2.5x58 cm. The oil was dissolved in 12.5% ethyl acetate in methylene chloride and applied to the column. The column was developed with the same solvent mixture. The mobile phase was run at 1.3 ml/minute initially and 15 minute fractions were collected. The flow rate slowed to about 0.5 ml/minute after 10 fractions, so fractions 1-10 were 20 ml decreasing to about 10 ml uniformly and fractions 11-98 were about 7 ml. At fraction 99 the flow rate was increased to give 25 ml fractions in 10 minutes. A total of 105 fractions were collected. These fractions were tested by thin layer chromatography in ethyl acetate:methylene chloride (1:1).

Fractions 30-54 were combined and evaporated giving 1.08 g of an oil containing LL-F28249γ.

Fractions 55-62 were combined and evaporated giving 150 mg of solid containing LL-F28249α and β.

The 150 mg of solid containing LL-F28249 αand β was chromatographed by preparative HPLC using a reverse-phase column (Whatman C8, 2.2x50 cm) developed with 80% (v/v) methanol in water. The flow rate was about 10 ml/minute and 2 minute fractions were collected.

Fractions 58-69 were combined, the methanol was evaporated, t-butanol was added and the mixture was lyophilized, giving 60 mg of pure LL-F28249α.

Fractions 40-43 were combined, the methanol was evaporated and the residual aqueous suspension was extracted with methylene chloride which, upon evaporation, gave 10 mg of pure LL-F28249 β.

The 1.08 g of oil containing LL-F28249γ was dissolved in 10% ethyl acetate in methylene chloride and applied to a column (2.5x50 cm) packed with silica gel. The column was developed with 10% ethyl acetate in methylene chloride, eluting at a flow rate of 2 ml/minute and collecting 12 minute fractions. Fractions 19-29 were combined and evaporated to a residue. This residue was purified by preparative reverse-phase chromatography as described for the α and β components. Fractions 55-62 were combined, the methanol was evaporated *in vacuo*, t-butanol was added and the mixture was lyophilized giving 60 mg of pure LL-F28249 γ.

Example 4

Large Scale Fermentation

An inoculum of *Streptomyces cyaneogriseus noncyanogenus*, NRRL 15773 was prepared as described in Example 1, using 100 ml of primary inoculum to produce 10 liters of secondary inoculum.

Two 300 liter fermentations were conducted as described in Example 2 using 10 liters of the above secondary inoculum for each 300 liters of fermentation medium. At the end of 118 hours the mashes were harvested.

Example 5

Isolation of LL-F28249ω

A total of 450 liters of harvest mash from the two 300 liter fermentations described in Example 4 was treated as described in the first portion of Example 3 giving crude material as a syrup.

This syrupy residue was washed with hexane to remove non-polar materials and the remaining 9 g of insoluble material was subjected to Sephadex LH-20 partition chromatography.

The chromatographic column was prepared with 9 liters of Sephadex LH-20, previously swelled in methanol, to form a column 10x110 cm. The column was equilibrated by passing about 4800 ml of mobile phase [methylene chloride:hexane:methanol (10:10:1)] through it at a flow rate of 5 ml/minute. The 9 g of insoluble material was charged onto the column in 50 ml of the mobile phase. An initial forerun of 2150 ml was obtained at a flow rate of 5 ml/minute. The flow rate was then increased to 8 ml/minute and fractions were collected every 45 minutes. Fractions 9-12 were combined and the solvents evaporated in vacuo giving 4.9 g of residue.

This residue was dissolved in a 1:1 mixture of cyclohexane and ethyl acetate and allowed to evaporate slowly at room temperature. The addition of n-hexane gave a precipitate which was collected, giving 3.1 g of solid.

A 3.0 g portion of this solid was further purified by precipitation from 25 ml of methylene chloride using 50 ml of n-hexane.

The precipitate thus obtained was redissolved in 15 ml of methylene chloride and precipitated with 25 ml of n-hexane, giving 510 mg of pure LL-F28249ω.

Example 6

Isolation of LL-F28249δ,ε ,ζ ,η ,θ and ι

Fractions 4-7 from the Sephadex LH-20 column described in Example 5 were combined and the solvents evaporated in vacuo to give 1.9 g of residue.

This residue was chromatographed on a 200 g silica gel column (2.5cm x 83cm) using 10% ethyl acetate in methylene chloride as the eluant. The flow rate was approximately 2 ml/minute and fractions were collected every 12 minutes.

Fractions 65-67 and 73-79 were combined together and the solvents were evaporated in vacuo to yield 250 mg of residue.

This 250 mg of residue was subjected to preparative reverse-phase chromatography as described in Example 3 except using 75% methanol in water as the mobile phase. The flow rate was about 10 ml/minute. The first 2000 ml portion of eluate was diverted to waste then 72 fractions were collected at 2.0 minute intervals. After diverting another portion of eluate to waste (between 300-400 ml) fractions were collected again but at 2.5 minute intervals.

Fractions were combined as indicated below. The combined fractions were allowed to evaporate in a fume hood overnight, then the components were extracted into methylene chloride. Follwing evaporation of the solvent about 1 mg each of the pure components were obtained.

| Fractions Combined | Compound |
|---|---|
| 7-10 | LL-F28249δ |
| 19-22 | LL-F28249ε |
| 28-31 | LL-F28249ζ |
| 81-83 | LL-F28249η |
| 86-88 | LL-F28249θ |
| 93-95 | LL-F28249ι |

Example 7

Isolation of LL-F28249κ,λ , μ and ν

A total of 390 liters of fermentation mash, harvested from fermentations conducted as described in Example 2, was processed essentially as described in the first paragraph of Example 3, giving 120 ml of methylene chloride concentrate. This concentrate was diluted with 200 ml of hexane and chilled overnight at 4°C. The resulting precipitate was removed by filtration and discarded. The filtrate was diluted with 300 ml

of hexane. The resulting precipitate (A) was collected by filtration and saved. This filtrate was evaporated to dryness and the oily residue was then dissolved in 200 ml of methylene chloride and diluted with 1700 ml of hexane. The resulting precipitate (B) was collected by filtration and saved. This filtrate was concentrated to an oily residue which was then redissolved in 50 ml of methylene chloride, 950 ml of methanol was added and this solution was stored at 4°C for 3 days. The resulting precipitate was removed by filtration and discarded. The filtrate was evaporated to dryness and the residue (C) combined with (A) and (B) and subjected to chromatography as follows: The 5.0x109cm column was slurry-packed with Woelm TSC silica gel in ethyl acetate:methylene chloride (1:9). The column was developed with the same solvent mixture at a rate of 25 ml/minute. The first 2 liters of effluent were discarded, then sixteen 400 ml fractions were collected.

Fractions 2 and 3 were combined and evaporated giving 3.9 g of oily material (D).

Fractions 4 through 7 were combined and evaporated giving 9.5 g of oily material which was dissolved in hexane and chromatographed on a 2.5x110cm column slurry-packed with 300 g of Woelm silica gel in ethyl acetate:hexane (1:4). The column was developed with the same solvent system at a rate of 4 ml/minute, collecting fractions at 7 minute intervals.

Fractions 45-54 were combined and evaporated, giving 0.3 g of material (E).

Fractions 63-135 were combined, evaporated to dryness, then redissolved in t-butanol and lyophilized giving 4.6 g of off-white solid (F).

LL-F28249 $\kappa$ and$\mu$

Material (D) and (E) were combined and chromatographed on a 2.5x110cm column packed with 300 g of Woelm silica gel, developing with ethyl acetate:hexane (1:9). The flow rate was maintained at 4 ml/minute and fractions were collected at 7 minute intervals.

Fractions 67-115 were combined and evaporated to dryness, giving 920 mg of residue (G).

This residue (G) was chromatographed by preparative HPLC using a reverse phase column (Whatman C8, 2.2x50 cm) and developing with 85% (v/v) methanol in water. The flow rate was about 10ml/minute and fractions were collected at 2.5 minute intervals.

Fractions 33-40 were combined, concentrated to remove the methanol, then extracted with methylene chloride. The residue obtained upon evaporation was dissolved in t-butanol and then lyophilized, giving 60 mg of LLF28249$\kappa$.

Fractions 52-58 were similarly processed giving a small quantity of LL-F28249$\mu$.

LL-F28249$\lambda$

A one gram portion of material (F) was chromatographed by reverse phase HPLC, as described above, except that 80% (v/v)methanol in water was used as eluent.

Fractions 61-75 were combined and processed as above, giving 100 mg of LL-F28249$\lambda$.

LL-F28249$\nu$

A 396 g portion of material essentially the same as material (D) above, was dissolved in 500 ml of methanol and then chilled at 4° for several hours. The resulting precipitate was removed by filtration, washed with cold methanol and discarded. The combined filtrate and wash was evaporated. The residual oil was dissolved in hexane and charged on a 5x50 cm dry-packed silica gel column (Mallinkrodt SilicAR cc-7). The column was eluted with ethyl acetate:hexane (1.5:8.5) at a rate of about 50 ml/minute.

Four fractions were collected.

| Fraction | Volume(liters) |
|---|---|
| 1 | 1 |
| 2 | 4 |
| 3 | 1 |
| 4 | 2 |

Fraction 3 was evaporated, giving 5.0 g of residue which was purified by preparative reverse phase HPLC (Waters $C_{18}$, 5x60cm). The column was initially developed with 16 liters of 80% methanol in water (v/v) at 100 ml/minute, then with 6.4 liters of 84% methanol in water (v/v). The first liter of effluent was

discarded and then fractions of 400 ml were collected.

Fractions 44-47 were combined and processed as described above, giving 390 mg of LLF28249ν as a pale yellow solid.

Example 8

Anti-nematodal activity of LL-F28249, NRRL 15773

This in vitro assay is designed to utilize the free living nematode Caenorhabditis elegans (C. elegans) to detect the anti-nematodal activity of fermentation broths against microorganisms from the soil. The assay procedure consists of micropipetting 50 µl of each broth into one of 96 wells of a microculture plate and adding 10 µl of a three to four day-old culture of C. elegans (in all stages of development) suspended in C. briggsae Maintance Medium. The effects of the fermentation broths are observed and recorded at 48 hours after the initial mixing of broth and nematodes.

LL-F28249, NRRL 15773, broth killed all the adults and markedly reduced the survival and mobility of various larval stages in both the initial and in a replicate assay.

EXAMPLE 9

In vivo anthelmintic activity of LL-F28249, NRRL 15773

This in vivo system is designed to detect potential anthelmintic activity of all fermentation products found to have anti-nematodal action against C. elegans. Samples of LL-F28249, NRRL 15773 are mixed into feed, at concentrations of from 0.0031% to 2.0% (31 ppm to 20,000 ppm). Medicated diet containing the varying concentrations of LL-F28249, NRRL 15773 is given to gerbils infected with 400 third-stage larvae of Trichostrongylus colubriformis The medicated feed is fed ad libitum, starting when the infection is seven days old, for three and one-half to four days, at which time the gerbils are necropsied. The intestines are removed and placed in water in an incubator at 45°C for two hours to allow the parasites to migrate from the tissue. The efficacy of each treatment is determined by counting the number of T. colubriformis recovered compared to an untreated control. The results of these experiments, summarized in Table XIII below, demonstrate the anthelmintic activity of LL-F28249 as administered in feed, and when administered as a single oral drench, and by subcutaneous injection.

## TABLE XIII

Anthelmintic activity of active ingredients from LL-F28249, NRRL 15773 culture against Trichostrongylus colubriformis in the gerbil

| F28249 | | | | | | |
|---|---|---|---|---|---|---|
| | | **With medicated diet, Ad libitum** | | | | |
| Whole mash (lyophilized) | Conc. (ppm) | 500.0 | 250.0 | 125.0 | 62.5 | |
| | Efficacy % | 100.0 | 98.0 | 88.0 | 40.0 | |
| α | Conc. (ppm) | 20.0 | 0.5 | 0.1 | 0.05 | |
| | Efficacy % | 100.0 | 100.0 | 97.0 | 31.0 | |
| | | **With single oral drench** | | | | |
| Whole Mash (lyophilized) | Dose (mg/kg) | 200.0 | 100.0 | 50.0 | 25.0 | |
| | Efficacy % | 100.0 | 100.0 | 100.0 | 88.0 | |
| α | Dose (mg/kg) | 10.0 | 0.5 | 0.1 | 0.05 | 0.025 |
| | Efficacy % | 100.0 | 100.0 | 100.0 | 99.0 | 6.0 |
| γ | | - | - | 0.1 | 0.05 | 0.025 |
| | | | | 78.0 | 15.0 | 10.0 |
| ω | | - | - | 0.1 | - | - |
| | | | | 30.0 | | |
| | | **With subcutaneous injection** | | | | |
| Whole Mash (lyophilized) | Dose (mg/kg) | 200.0 | 100.0 | 50.0 | 25.0 | |
| | Efficacy % | 100.0 | 100.0 | 100.0 | 70.0 | |
| α | Dose (mg/kg) | 1.0 | 0.2 | 0.1 | | |
| | Efficacy % | 100.0 | 99.5 | 60.0 | | |

EXAMPLE 10

The anthelmintic activity of LL-F28249α against parasitic nematodes in sheep

This experiment is designed to evaluate the activity of LL-F28249α against the economically important parasites of sheep. The sheep are experimentally inoculated with infective larvae of Haemonchus contortus, Ostertagia circumcincta and Trichostrongylus coluriformis, to build up infections against which LL-F28249a will be challenged. Twenty-one days after inoculation, infection levels are determined by standard stoll count nematode counting procedures to determine the number of eggs of each species per gram of feces. The sheep are assigned randomly across three replicates of treatment and control groups based upon nematode egg counts. Twenty-two days after infection the sheep are treated with LL-F28249α using the doses and routes or administration shown in Table XIV below. Seven and eight days after treatment, the sheep are sacrificed and the worms are recovered using standard anthelmintic evaluation procedures. The efficacy of each treatment against each species is determined by comparing the number of worms at the respective dosage rate against the number of worms recovered in the three untreated control animals. The results of these evaluations, summarized in Table XIV below, demonstrate the high degree of effectiveness of LL-F28249α as an anthelmintic agent.

TABLE XIV

Anthelmintic efficacy of F28249 α against Haemonchus, Ostertagia and Trichostrongylus in sheep

| Dose mg/kg | Route of administration | Efficacy (%) against Haemonchus | Ostertagia | T. colubriformis |
|---|---|---|---|---|
| 1.0 | oral | 100.0 | 100.0 | 99.9 |
| 0.2 | oral | 100.0 | 100.0 | 99.9 |
| 0.1 | oral | 100.0 | 95.4 | 99.9 |
| 1.0 | IM | 100.0 | 100.0 | 100.0 |
| 0.2 | IM | 100.0 | 100.0 | 100.0 |
| | | Mean number of worms recovered (range) | | |
| 0.0 | - | 2683.0 | 881.0 | 16200.0 |

IM = Intermuscular

EXAMPLE 11

Efficacy of antibiotic LL-F28249α against the parasitic insect, Melophagus ovinus, (the sheep ked) on sheep

This experiment is conducted concurrently on the same sheep used for the determination of anthelmintic activity as reported in Example 10. During the handling of the sheep prior to treatment, said sheep are observed for harbouring of natural infestations of M. ovinus. One half of each sheep is inspected for the indications of anti-ectoparasitic activity at necropsy, seven days after treatment.

The left side of each sheep is slowly sheared with electric clippers and inspected for living and dead sheep keds. The degree of infestation is approximated by the numbers of pupae found in the wool during the inspection and are rated 0 through + + +, indicating no pupae to many pupae. The number of keds are recorded for each sheep, without knowledge of the treatment levels to eliminate bias. Initially, the keds were scored as alive or dead, but as experience was gained, some keds were scored as moribund because of abnormally-slow behavior.

Although there is a wide variation in the number of keds found on the sheep, the data summarized in Table XV below demonstrate that LL-F28249α is effective against M. ovinus and that said agent possesses systemic ectoparasiticide activity. In treated animals the numbers of live keds is effectively reduced and the number of dead keds increased in the intramuscularly-treated sheep.

TABLE XV

Efficacy of agent F28249α against *Melophagus ovinus* on sheep

| Dose mg/kg | Route of administration | Mean number of keds[a] Alive | Dead | % |
|---|---|---|---|---|
| 1.0 | Intramuscular | 1.67 | 1.67 | 78.22 |
| 0.2 | Intramuscular | 1.0 | 4.33 | 86.96 |
| 1.0 | Oral | 7.67 | 0.0 | 0.0 |
| 0.2 | Oral | 2.67 | 3.0 | 65.0 |
| 0.1 | Oral | 22.0 | 1.67 | 0.0 |
| Control | None | 7.67 | .67 | - |

a Three sheep per dose

b $\text{Efficacy \%} = 100 \times \frac{\text{Mean number in control - mean number in treated}}{\text{Mean number in control}}$

EXAMPLE 12

Insecticidal activity of the compounds of the invention

The insecticidal activity of the compounds of the present invention against a variety of insects at various concentrations of active ingredient in acetone-water solutions is determined by the following insecticidal test examples. The results of these tests are summarized in Table XVI.

A) Heliothis virescens, egg, tobacco budworm.

A young cotton leaf about 7-8 cm long is dipped and agitated in a test suspension for three seconds. Eggs are collected on cheesecloth that is cut into 10-20 mm squares containing about 50-100 eggs (6-30 hours old). A square of cheesecloth with eggs also is dipped in the test suspension and placed on the treated leaf. The combination is placed in the hood to dry. Following this, the combination is placed in an 8 ounce Dixie cup #2168-ST (240 mL, 6 cm tall, top diameter 9.5 cm, bottom diameter 8 cm) containing a 5 cm length of damp dental wick. A clear plastic lid is put on the top of the cup, and the treatments held for three (3) days before mortality counts are made.

B) Aphis fabae, mixed instars, bean aphids.

Pots containing single masturtium plant (Tropaeolum sp), about 5 cm tall, are infested with about 100 aphids one day before the test. In a hood, each plant is sprayed with the test suspension for 2 revolutions of a 4 rpm turntable using a #154 DeVilluss atomizer. The pots are set on their side on white enamel trays and held for two (2) days. After that time, mortality estimates of the aphids are made.

C) Empoasca abrupta, adult, western potato leafhopper.

A Sieva lima bean leaf about 5 cm long is dipped and agitated in the test suspension for three (3) seconds and then placed in a hood to dry. The leaf is placed in a 100 x 10 mm petri dish containing a moist filter paper on the bottom of the dish. Ten, adult leafhoppers are added to each dish, and the treatments are kept for three (3) days after which time mortality counts are made.

D) Trichoplusia ni, Third-instat larvae, cabbage looper.

The leaves of a Sieva lima bean plant expanded to 7-8 cm in length are dipped and agitated in a test suspension for three (3) seconds and then placed in a hood to dry. A leaf is then excised and placed in a 100 x 10 mm petri dish containing a damp filter paper on the bottom and ten third-instar larvae are placed therein. The dish is maintained for three (3) days before observations are made of mortality and reduced feeding.

E) Spodoptera eridanis, third-instar larvae, southern armyworm.

The leaves of a Sieva lima bean plant expanded to 7-8cm in length are dipped and agitated in the test suspension for three (3) seconds and placed in a hood to dry. A leaf is then excised and placed in a 100 x 10 mm petri dish containing a damp filter paper on the bottom and ten (10) third-instar larvae are added. The dish is maintained for five (5) days before observations are made of mortality, reduced feeding or any interference with normal moulting.

F) Heliothis virescens, third-instar larvae, tobacco budworm.

Cotton cotyledons are dipped in the test suspension and placed in a hood to dry. The cotyledon is cut into 4 sections, and each section is placed in a 30 ml plastic medicine cup containing a 5-7 mm piece of moist dental wick. One third-instar larvae are added to each cup and a cardboard lid placed on the cup. Treatments are maintained for three (3) days before mortality counts and estimates of reduction in feeding are made.

G) Musca domestica, house fly.

The desired concentration of the test compound is added to the standard CSMA alfalfa-bran larval medium. House flies' eggs, 0-4 hours of age, are added to the treated medium. The treated medium is maintained and observations on egg hatch, larval growth and adult emergence are made.

H) Tribolium confusum, confused flour beetle.

Confused flour beetles (Tribolium confusum) are obtained from laboratory colonies reared on a whole wheat and white flour mixture. For this test, white flour is treated with an acetone solution of the test material using 1 ml of solution per 5 grams of flour in a 30 ml wide-mouth jar. The acetone is evaporated off in a hood overnight. The contents are stirred with a spatula to break up lumps formed by the test solution. The jar is then placed on a VORTES-GENIE® vibrating mixer to thoroughly mix the test materials throughout the diet. Ten adult confused flour beetles are placed in each jar and the jar loosely capped. After five (5) days to allow oviposition, the beetles are removed and notations made of any mortality. At two (2) and four (4) weeks after initial infestation, observations are made of the number and size of trails produced by the developing larvae throughout the treated flour. Such observations give an indication of delayed growth, kill of eggs or larvae or any other interference in the normal growth pattern. After about nine (9) weeks at 27° C, the adult beetles emerge and the final observations are made by passing the contents of each jar through a 50-mesh screen sieve. These observations include the number of adults, pupae and larvae, as well as examination of the debris which did not pass through the screen in order to determine if there are any dead eggs or neonates.

I) Tetranychus urticae (P-resistant strain), 2-spotted spider mite.

Sieva lima bean plants with primary leaves expanded to 7-8 cm are selected and cut back to one plant per pot. A small piece is cut from a leaf taken from the main colony and placed on each leaf of the test plants. This is done about two (2) hours before treatment to allow the mites to move over to the test plant and to lay eggs. The size of the cut piece is varied to obtain about 100 mites per leaf. At the time of the treatment, the piece of leaf used to transfer the mites is removed and discarded. The mite-infested plants are dipped and agitated in the test formulation for three (3) seconds and set in the hood to dry. Plants are kept for two (2) days before estimates of adult kill are made by using the first leaf. The second leaf is kept on the plant for another five (5) days before observations are made of the kill of eggs and/or newly emerged nymphs.

J) Southern armyworm (Spodoptera eridania), third-instar, cut-stem systemic test.

The compound is formulated as an emulsion containing 0.1 gm of the test material, 0.1 gm of a polyethoxylated vegetable oil in 0.4 g water, 10 mL of acetone and 90 mL of water. This is diluted tenfold with water to give the 100 ppm emulsion for the test. Sieva lima bean plants with just the primary leaves expanded are used in this test. These leaves are cut off at least 2.5 cm above the soil level to avoid contamination with soil bacteria which may cause decay of the stem during the test. The cut stems are placed in the test emulsion. After three (3) days of uptake, a leaf is excised and placed in a 100 x 10 mm petri dish containing a moist filter paper on the bottom and ten third-instar larvae. Mortality counts and estimates of reduced feeding are made after three (3) days.

K) Thrips palmi, thrips.

Heavily infested leaves of cotton seedings are sprayed under field conditions at the desired concentrations. The number of thrips are counted before and after spraying. Percent control is based on these counts.

L) Tetranychus urticae (P-resistant strain), two spotted spider mite.

The compound is formulated as an emulsion containing 0.1 gm of the test material, 0.1 gm of a polyethoxylated vegetable oil in 0.4 g water, 10 mL of acetone and 90 mL of water. This is diluted tenfold with water to give the 100 ppm emulsion for the test. Sieva lima bean plants with just the primary leaves expanded are used in this test. They are cut off at least 2.5 cm above the soil level to avoid contamination with soil bacteria which may cause decay of the stem during the test. The cut stems are placed in the test emulsions. Each leaf is infested with approximately 100 adult mites and maintained for three (3) days at which time mortality counts are made.

TABLE XVI

Insecticidal and Miticidal Activity of F-28,249α and F-28,249γ

| | | Percent Mortality | | | | | | | | | | Plant Systemic Activity | |
|---|---|---|---|---|---|---|---|---|---|---|---|---|---|
| Compound | Concn. in ppm | Cabbage loopers | Southern army-worms | Tobacco bud-worms | Tobacco bud-worms eggs | Bean aphid | Western potato leaf hoppers | Confused flour beetle larvae and/or pupae | House fly larvae | Thrips | Mites | Southern army-Worms | Mites |
| F-28,249α | 1000 | 100 | 100 | 100 | - | - | 55* | 100 | 100 | 93 | - | - | - |
| F-28,249α | 300 | 100* | - | 100 | 100 | - | 50 | - | 100 | 89 | 100 | - | - |
| F-28,249α | 100 | - | 60* | 100* | 100 | 100 | - | 97 | - | - | 100 | 60 | 100 |
| F-28,249γ | 1000 | - | 40 | 50* | 100 | 100 | 20 | - | - | - | 100 | - | - |
| F-28,249γ | 100 | - | 0 | 0 | 0 | 100 | 0 | - | - | - | 90 | - | - |

* Feeding deterent (anti-feeding properties)

## Claims

1. The compound designated LL-F28249α useful for helminthic, arthropod, ectoparasitic, and acaridal and nematodal infections wherein the compound has:

a) a molecular weight of 612 (FAB-MS);
b) a molecular formula, $C_{36}H_{52}O_8$;
c) a specific optical rotation, $[\alpha]_D^{26}$ = +133±3° (C 0.3, acetone);
d) a characteristic ultraviolet absorption spectrum as shown in Figure I of the attached drawings;
e) a characteristic infrared absorption spectrum as shown in Figure II of the attached drawings;
f) a characteristic proton nuclear magnetic resonance spectrum as shown in Figure III of the attached drawings;
g) a characteristic carbon-13 nuclear magnetic resonance spectrum as shown in Figure IV of the attached drawings with significant peaks at, 173.4; 142.8; 139.4; 137.7; 137.3; 137.2; 130.6; 123.3; 120.3; 118.0; 99.7; 80.2; 79.3; 76.7; 69.3; 68.5; 68.4; 67.8; 67.7; 48.4; 45.7; 41.1; 40.7; 36.1; 36.0; 35.9; 34.7; 26.8; 22.8; 22.2; 19.9; 15.5; 13.9; 11.0; and
h) a characteristic electron impact mass spectrum as shown in Figure V of the attached drawings with measured m/z values and proposed elemental compositions as indicated below obtained by high resolution mass measurements,

| | | | |
|---|---|---|---|
| 612.3705 | $C_{36}H_{52}O_8$ | 354.2181 | $C_{23}H_{30}O_3$ |
| 594.3543 | $C_{36}H_{50}O_7$ | 314.1877 | $C_{20}H_{26}O_3$ |
| 576.3472 | $C_{36}H_{48}O_6$ | 278.1144 | $C_{15}H_{18}O_5$ |
| 484.3211 | $C_{30}H_{44}O_5$ | 265.1786 | $C_{16}H_{25}O_3$ |
| 482.2648 | $C_{29}H_{38}O_6$ | 248.1405 | $C_{15}H_{20}O_3$ |
| 466.3097 | $C_{30}H_{42}O_4$ | 247.1705 | $C_{16}H_{23}O_2$ |
| 448.2987 | $C_{30}H_{40}O_3$ | 237.1838 | $C_{15}H_{25}O_2$ |
| 442.2375 | $C_{26}H_{34}O_6$ | 219.1740 | $C_{15}H_{23}O$ |
| 425.2327 | $C_{26}H_{33}O_5$ | 151.0753 | $C_9H_{11}O_2$. |

**2.** The compound designated LL-F28249β useful for helminthic, arthropod, ectoparasitic, and acaridal and nematodal infections wherein the compound has:
a) a molecular weight of 584 (FAB-MS);
b) a molecular formula, $C_{34}H_{48}O_8$;
c) specific optical rotation: $[\alpha]_D^{26}$ = +125° (C 0.30, acetone).
d) a characteristic ultraviolet absorption spectrum as shown in Figure VI of the attached drawings;
e) a characteristic infrared absorption spectrum as shown in Figure VII of the attached drawings;
f) a characteristic proton nuclear magnetic resonance spectrum as shown in Figure VIII of the attached drawings;
g) a characteristic carbon-13 nuclear magnetic resonance spectrum as shown in Figure XXXVIII of the attached drawings, with significant peaks at 173.3; 142.6; 139.5; 137.7; 137.3; 133.9; 123.8; 123.4; 120.3; 120.2; 118.0; 99.7; 80.2; 79.4; 76.7; 69.2; 68.6; 68.3; 67.8; 67.7; 48.4; 45.7; 41.0; 40.8; 36.1; 35.9; 34.7; 22.3; 19.8; 15.5; 13.8; 13.1; 10.8; and
h) a characteristic electron impact mass spectrum as shown in Figure IX of the attached drawings with measured m/z values and proposed elemental compositions as indicated below obtained by high resolution mass measurements,

| | | | |
|---|---|---|---|
| 584.3388 | $C_{34}H_{48}O_8$ | 314.1858 | $C_{20}H_{26}O_3$ |
| 566.3306 | $C_{34}H_{46}O_7$ | 278.1168 | $C_{15}H_{18}O_5$ |
| 456.2864 | $C_{28}H_{40}O_5$ | 237.1491 | $C_{14}H_{21}O_3$ |
| 442.2391 | $C_{26}H_{34}O_6$ | 219.1380 | $C_{14}H_{19}O_2$ |
| 438.2780 | $C_{28}H_{38}O_4$ | 209.1534 | $C_{13}H_{21}O_2$ |
| 425.2331 | $C_{26}H_{33}O_5$ | 191.1418 | $C_{13}H_{19}O$ |
| 354.2187 | $C_{23}H_{30}O_3$ | 151.0750 | $C_9H_{11}O_2$. |

**3.** The compound designated LL-F28249γ useful for helminthic, arthropod, ectoparasitic, and acaridal and nematodal infections wherein the compound has:
a) a molecular weight of 598 (FAB-MS);
b) a molecular formula, $C_{35}H_{50}O_8$;

c) a specific optical rotation: $[\alpha]_D^{26}$ = +150±4° (C 0.3, acetone);
d) a characteristic ultraviolet absorption spectrum as shown in Figure X of the attached drawings;
e) a characteristic infrared absorption spectrum as shown in Figure XI of the attached drawings;
f) a characteristic proton nuclear magnetic resonance spectrum as shown in Figure XII of the attached drawings;
g) a characteristic carbon-13 nuclear magnetic resonance spectrum as shown in Figure XIII of the attached drawings with significant peaks at, 173.6; 142.4; 139.9; 137.3; 136.0; 134.0; 123.8; 123.6; 120.4; 119.6; 118.5; 99.8; 80.5; 77.8; 77.0; 76.8; 69.3; 68.6; 68.3; 67.9; 57.7; 48.5; 45.8; 41.2; 40.8; 36.2; 36.1; 36.0; 34.8; 22.3; 19.9; 15.5; 13.8; 13.1; 10.8; and
h) a characteristic electron impact mass spectrum as shown in Figure XIV of the attached drawings with measured m/z values and proposed elemental compositions as indicated below obtained by high resolution mass measurements,

| | | | |
|---|---|---|---|
| 598.3543 | $C_{35}H_{50}O_8$ | 354.2199 | $C_{23}H_{30}O_3$ |
| 580.3422 | $C_{35}H_{48}O_7$ | 314.1875 | $C_{20}H_{26}O_3$ |
| 562.3292 | $C_{35}H_{46}O_6$ | 292.1307 | $C_{16}H_{20}O_5$ |
| 496.2824 | $C_{30}H_{40}O_6$ | 288.2075 | $C_{19}H_{28}O_2$ |
| 484.2440 | $C_{28}H_{36}O_7$ | 248.1397 | $C_{15}H_{20}O_3$ |
| 478.2687 | $C_{30}H_{38}O_5$ | 237.1490 | $C_{14}H_{21}O_3$ |
| 456.2576 | $C_{27}H_{36}O_6$ | 219.1382 | $C_{14}H_{19}O_2$ |
| 438.2772 | $C_{28}H_{38}O_4$ | 209.1544 | $C_{13}H_{21}O_2$ |
| 425.2341 | $C_{26}H_{33}O_5$ | 191.1435 | $C_{13}H_{19}O$ |
| 420.2651 | $C_{28}H_{36}O_3$ | 151.0759 | $C_9H_{11}O_2$. |

**4.** The compound designated LL-F28249ω useful for helminthic, arthropod, ectoparasitic, and acaridal and nematodal infections wherein the compound has:
a) a molecular weight of 806 (FAB-MS);
b) a molecular formula, $C_{45}H_{74}O_{12}$;
c) a specific optical rotation: $[\alpha]_D^{26}$ = -49±4° (C 0.35, methanol);
d) a characteristic ultraviolet absorption spectrum as shown in Figure XV of the attached drawings;
e) a characteristic infrared absorption spectrum as shown in Figure XVI of the attached drawings;
f) a characteristic proton nuclear magnetic resonance spectrum as shown in Figure XVII of the attached drawings;
g) a characteristic carbon-13 nuclear magnetic resonance spectrum as shown in Figure XVIII of the attached drawings with significant peaks at, 220.7; 219.6; 165.2; 148.7; 133.1; 132.3; 130.2; 122.3; 100.0; 82.9; 75.9; 73.0; 72.7; 72.6; 72.1; 69.0; 67.3; 63.6; 51.4; 46.2; 45.7; 42.2; 40.4; 38.3; 37.6; 36.1; 34.8; 33.5; 30.1; 26.6; 25.4; 24.5; 23.0; 21.1; 17.9; 14.3; 14.2; 12.1; 11.5; 10.9; 8.7; 8.3; 5.7; and
h) a characteristic electron impact mass spectrum as shown in Figure XIX of the attached drawings with measured m/z values and proposed elemental compositions as indicated below obtained by high resolution mass measurements,

| | | | |
|---|---|---|---|
| 462.3350 | $C_{28}H_{46}O_5$ | 253.1797 | $C_{15}H_{25}O_3$ |
| 444.3237 | $C_{28}H_{44}O_4$ | 235.1697 | $C_{15}H_{23}O_2$ |
| 425.2534 | $C_{23}H_{37}O_7$ | 224.1754 | $C_{14}H_{24}O_2$ |
| 407.2439 | $C_{23}H_{35}O_6$ | 209.1530 | $C_{13}H_{21}O_2$ |
| 406.3046 | $C_{25}H_{42}O_4$ | 207.1744 | $C_{14}H_{23}O$ |
| 387.2895 | $C_{25}H_{39}O_3$ | 184.1458 | $C_{11}H_{20}O_2$ |
| 337.2010 | $C_{19}H_{29}O_5$ | 179.1048 | $C_{11}H_{15}O_2$ |
| 297.2031 | $C_{17}H_{29}O_4$ | 173.1205 | $C_9H_{17}O_3$ |
| 279.1944 | $C_{17}H_{27}O_3$ | 167.1051 | $C_{10}H_{15}O_2$ |
| 261.1851 | $C_{17}H_{25}O_2$ | 155.1069 | $C_9H_{15}O_2$. |

**5.** The compound designated LL-F28249 δ useful for helminthic, arthropod, ectoparasitic, and acaridal and nematodal infections wherein the compound has:

a) a molecular weight of 616 (EI-MS);
b) a molecular formula, $C_{35}H_{52}O_9$;
c) a HPLC retention volume of 14.0 ml;
d) a characteristic ultraviolet absorption spectrum shown in Figure XX of the attached drawings;
e) a characteristic proton nuclear magnetic resonance spectrum as shown in Figure XXI of the attached draw-
f) a characteristic electron impact mass spectrum as shown in Figure XXII of the attached drawings.

**6.** The compound designated LL-F28249$\epsilon$ useful for helminthic, arthropod, ectoparasitic, and acaridal and nematodal infections wherein the compound has:
a) a molecular weight of 598 (EI-MS);
b) molecular formula, $C_{35}H_{50}O_8$;
c) a HPLC retention volume of 14.8 ml;
d) a characteristic ultraviolet absorption spectrum as shown in Figure XXIII of the attached drawings;
e) a characteristic proton nuclear magnetic resonance spectrum as shown in Figure XXIV of the attached drawings; and
f) a characteristic electron impact mass spectrum as shown in Figure XXV of the attached drawings.

**7.** The compound designated LL-F28249 $\zeta$ useful for helminthic, arthropod, ectoparasitic, and acaridal and nematodal infections wherein the compound has:
a) a molecular weight of 598 (EI-MS);
b) a molecular formula, $C_{35}H_{50}O_8$;
c) a HPLC retention volume of 16.0 ml;
d) a characteristic ultraviolet absorption spectrum as shown in Figure XXVI of the attached drawings;
e) a characteristic proton nuclear magnetic resonance spectrum as shown in Figure XXVII of the attached drawings; and
f) a characteristic electron impact mass spectrum as shown in Figure XXVIII of the attached drawings.

**8.** The compound designated LL-F28249$\eta$ useful for helminthic, arthropod, ectoparasitic, and acaridal and nematodal infections wherein the compound has:
a) a molecular weight of 612 (EI-MS);
b) a molecular formula, $C_{36}H_{52}O_8$;
c) a HPLC retention value of 23.5 ml;
d) a characteristic ultraviolet absorption spectrum as shown in Figure XXIX of the attached drawings;
e) a characteristic proton nuclear magnetic resonance spectrum as shown in Figure XXX of the attached drawings; and
f) a characteristic electron impact mass spectrum as shown in Figure XXXI of the attached drawings.

**9.** The compound designated LL-F28249 $\theta$ useful for helminthic, arthropod, ectoparasitic, and acaridal and nematodal infections wherein the compound has:
a) a molecular weight of 626 (EI-MS);
b) a molecular formula, $C_{37}H_{54}O_8$;
c) a HPLC retention value of 24.5 ml;
d) a characteristic ultraviolet absorption spectrum as shown in Figure XXXII of the attached drawings;
e) a characteristic proton nuclear magnetic resonance spectrum as shown in Figure XXXIII of the attached drawings; and
f) a characteristic electron impact mass spectrum as shown in Figure XXXIV of the attached drawings.

**10.** The compound designated LL-F28249 $\iota$ useful for helminthic, arthropod, ectoparasitic, and acaridal and nematodal infections wherein the compound has:
a) a molecular weight of 626 (EI-MS);
b) a molecular formula, $C_{37}H_{54}O_8$;
c) a HPLC retention value of 26.0 ml;
d) a characteristic ultraviolet absorption spectrum as shown in Figure XXXV of the attached drawings;
e) a characteristic proton nuclear magnetic resonance spectrum as shown in Figure XXXVI of the

attached drawings; and

f) a characteristic electron impact mass spectrum as shown in Figure XXXVII of the attached drawings.

**11.** The compound designated LL-F28249*x* useful for helminthic, arthropod, ectoparasitic, and acaridal and nematodal infections wherein the compound has:

a) molecular weight 584 (EI-MS);

b) molecular formula: $C_{35}$ $H_{52}$ $O_7$;

c) Specific optical rotation: $[\alpha]_D^{26}$ = +189°-(C 0.165 acetone);

d) Ultraviolet absorption spectrum: as shown in Figure XXXIX

$$UV \frac{CH_3OH}{MAX} = 241nm\ (E20,400);$$

e) Infrared absorption spectrum: as shown in Figure XL (KBr disc);

f) Electron impact mass spectrum: as shown in Figure XLI;

g) Proton nuclear magnetic resonance spectrum ($CDCl_3$); as shown in Figure XLII; and

h) Carbon-13 nuclear magnetic resonance spectrum ($CDCl_3$); as shown in Figure XLIII and described in Table IX.

**12.** The compound designated LL-F28249λ useful for helminthic, arthropod, ectoparasitic, and acaridal and nematodal infections wherein the compound has:

a) molecular weight: 626 (FAB-MS);

b) Molecular formula: $C_{37}$ $H_{54}$ $O_8$;

c) specific optical rotation: $[\alpha]_D^{26}$ = +145° (C, 0.23 acetone);

d) Ultraviolet absorption spectrum: as shown in Figure XLIV

$$UV \frac{CH_3OH}{MAX} = 244nm\ (E30,000);$$

e) Infrared absorption spectrum: as shown in Figure XLV (KBr disc);

f) Electron impact mass spectrum: as shown in Figure XLVI;

g) Proton nuclear magnetic resonance spectrum ($CDCl_3$); as shown in Figure XLVII; and

h) Carbon-13 nuclear magnetic resonance spectrum ($CDCl_3$); as shown in Figure XLVIII and described in Table X.

**13.** The compound designated LL-F28249μ useful for helminthic, arthropod, ectoparasitic, and acaridal and nematodal infections wherein the compound has:

a) molecular weight: 612 (EI-MS);

b) molecular formula: $C_{37}$ $H_{56}$ $O_7$;

c) Ultraviolet absorption spectrum: as shown in Figure XLIX

$$UV \frac{CH_3OH}{MAX} = 241nm\ (E16,800);$$

d) Infrared absorption spectrum: as shown in Figure L (KBr disc);

e) Electron impact mass spectrum: as shown in Figure LI;

f) Proton nuclear magnetic resonance spectrum ($CDCl_3$); as shown in Figure LII.

**14.** The compound designated LL-F28249ν useful for helminthic, arthropod, ectoparasitic, and acaridal and nematodal infections wherein the compound has:

a) molecular weight: 592 (EI-MS);

b) molecular formula: $C_{36}$ $H_{48}$ $O_7$;

c) specific optical rotation: $[\alpha]_D^{26}$ +131° (C .325, acetone);

d) Ultraviolet absorption spectrum: as shown in Figure LIII

$$UV\ {}^{CH_3OH}_{MAX} = 256\ (E20,500);\ 358\ (E\ 8,830);$$

e) Infrared absorption spectrum: as shown in Figure LIV (KBr disc);
f) Electron impact mass spectrum: as shown in Figure LV;
g) Proton nuclear magnetic resonance spectrum ($CDCl_3$); as shown in Figure LVI; and
h) Carbon-13 nuclear magnetic resonance spectrum ($CDCl_3$); as shown in Figure LVII, and described in Table XI.

**15.** A process for producing agents LL-F28249α, LL-F28249β, LL-F28249γ, LL-F28249δ, LL-F28249ε,LL-F28249ζ, LL-F28249η, LL-F28249θ, LL-F28249ι, LL-F28249κ, LL-F28249λ, LL-F28249μ, LL-F28249ν, and LL-F28249ω as defined in one of Claims 1 to 14, which comprises aerobically fermenting the organism Streptomyces cyaneogriseus noncyanogenus, NRRL 15773 in a liquid medium containing assimilable sources of carbon, nitrogen and inorganic anions and cations, until a substantial amount of LL-F28249α,β , γ, δ,ε ,ζ ,η,θ , ι, κ ,λ ,μ ,ν and ω are produced in said medium; and then recovering the agents therefrom.

**16.** A process for producing agents LL-F28249α, LL-F28249β, LL-F28249γ, LL-F28249δ, LL-F28249ε, LL-F28249ζ, LL-F28249η, LL-F28249θ, LL-F28249ι, LL-F28249κ, LL-F28249λ, LL-F28249μ, LL-F28249ν, and LL-F28249ω as defined in one of Claims 1 to 14, which comprises aerobically fermenting a liquid medium containing assimilable sources of carbon, nitrogen and inorganic anions and cations, which medium has been inoculated with a viable culture of the organism Streptomyces cyaneogriseus noncyanogenus, NRRL 15773; maintaining said fermentation culture with sterile aeration and agitation at a temperature of 24°-32°C for a period of 80-200 hours; harvesting the mash; and extracting the agents.

**17.** A biologically pure culture of the microorganism Streptomyces cyaneogriseus noncyanogenus, NRRL 15773, said culture being capable of producing agents LL-F28249α, LL-F28249β, LL-F28249γ, LL-F28249δ, LL-F28249ε, LL-F28249ζ, LL-F28249η, LL-F28249θ, LL-F28249ι, LL-F28249κ, LL-F28249λ, LL-F28249μ, LL-F28249ν, and LL-F28249ω as defined in one of Claims 1 to 14, in recoverable quantities upon fermentation in an aqueous nutrient medium containing assimilable sources of carbon, nitrogen and inorganic anions and cations.

**18.** The compound according to Claim 1, 2, 3, 4, 5, 6, 7, 8, 9, 10, 11, 12, 13 or 14 additionally comprising: the pharmaceutically and pharmacologically-acceptable salts thereof.

**19.** A method for the control of plant nematodes, said method comprising: applying to the foliage of plants, the soil in which they are grown, or into the trunks thereof, a nematocidally-effective amount of the fermentation broth or whole mash of microorganism Streptomyces cyaneogriseus non-cyanogenus, having deposit accession number NRRL 15773.

**20.** A method for the control of plant nematodes, said method comprising: applying to the foliage of plants, the soil in which they are grown, or into the trunks thereof, a nematocidally-effective amount of the fermentation broth or whole mash of microorganism Streptomyces cyaneogriseus noncyanogenus, having deposit accession number NRRL 15773, containing agents designated LL-F28249α, LL-F28249β, LL-F28249γ, LL-F28249δ, LL-F28249ε, LL-F28249ζ, LL-F28249η, LL28249θ, LL-F28249ι, LL-F28249κ, LL-F28249λ, LL-F28249μ, LL-F28249ν, and LL-F28249ω as defined in one of Claims 1 to 14; or the pharmaceutically and pharmacologically acceptable salts thereof.

**21.** A method according to Claims 19 or 20, wherein about 0.1 to 1.4 kg per hectare is applied to thereof.

**22.** An animal feed composition for the prevention, treatment or control of helmintic, arthropod ectoparasitic or acaridal infections in meat-producing animals, said animal feed composition comprising: an edible solid carrier; and a phophylactically, therapeutically or pharmaceutically-effective amount of the fermentation broth or whole mash of microorganism Streptomyces cyaneogriseus noncyanogenus, having deposit accession number NRRL 15773.

**23.** An animal feed premix composition for the prevention, treatment or control of helmintic, arthropod ectoparasitic or acaridal infections in meat-producing animals, said animal feed premix composition comprising: an edible carrier; and a prophylactically, therapeutically or pharmaceutically-effective amount of the fermentation broth or whole mash of microorganism Streptomyces cyanogriseus noncyanogenus, having deposit accession number NRRL 15773, containing agents designated LL-F28249α, LL-F28249β, LLF28249 γ, LL-F28249δ, LL-F28249ε, LL-F28249ζ, LL-F28249η, LL-F28249 θ, Ll-F28249 ι, LL-F28249κ, LL-F28249λ, LL-F28249μ, LL-F28249 νand LL-F28249ω as defined in one of Claims 1 to 14; or the pharmaceutically and pharmacologically acceptable salts thereof.

**24.** A composition according to Claims 22 or 23, wherein said effective amount is about 0.00001% to 5%, by weight, of said composition.

**Revendications**

**1.** Composé dénommé LL-F28249α, utile contre les infections par des helminthes, des arthropodes, des ectoparasites, des acariens et des nématodes, ce composé ayant :

a) un poids moléculaire de 612 (spectrométrie de masse FAB) ;

b) une formule moléculaire correspondant à $C_{36}H_{52}O_8$ ;

c) une rotation optique spécifique correspondant à $[\alpha]_D^{26}$ = +133±3° (C : 0,3 ; acétone) ;

d) un spectre d'absorption caractéristique en ultraviolet tel qu'illustré sur la figure I des dessins annexés ;

e) un spectre d'absorption caractéristique en infrarouge tel qu'illustré sur la figure II des dessins annexés ;

f) un spectre caractéristique de résonance magnétique nucléaire du proton tel qu'illustré sur la figure III des dessins annexés ;

g) un spectre caractéristique de résonance magnétique nucléaire du carbone 13 tel qu'illustré sur la figure IV des dessins annexés, avec des pics significatifs à 173,4 ; 142,8 ; 139,4 ; 137,7 ; 137,3 ; 137,2 ; 130,6 ; 123,3 ; 120,3 ; 118,0 ; 99,7 ; 80,2 ; 79,3 ; 76,7 ; 69,3 ; 68,5 ; 68,4; 67,8 ; 67,7 ; 48,4 ; 45,7 ; 41,1 ; 40,7 ; 36,1 ; 36,0 ; 35,9 ; 34,7 ; 26,8 ; 22,8 ; 22,2 ; 19,9 ; 15,5 ; 13,9 ; 11,0 ; et

h) un spectre de masse à impact électronique caractéristique tel qu'illustré sur la figure V des dessins annexés, ayant les valeurs m/z mesurées et les compositions élémentaires proposées mentionnées ci-dessous, obtenues par des mesures de masse à haute résolution :

| | | | |
|---|---|---|---|
| 612,3705 | $C_{36}H_{52}O_8$ | 354,2181 | $C_{23}H_{30}O_3$ |
| 594,3543 | $C_{36}H_{50}O_7$ | 314,1877 | $C_{20}H_{26}O_3$ |
| 576,3472 | $C_{36}H_{48}O_6$ | 278,1144 | $C_{15}H_{18}O_5$ |
| 484,3211 | $C_{30}H_{44}O_5$ | 265,1786 | $C_{16}H_{25}O_3$ |
| 482,2648 | $C_{29}H_{38}O_6$ | 248,1405 | $C_{15}H_{20}O_3$ |
| 466,3097 | $C_{30}H_{42}O_4$ | 247,1705 | $C_{16}H_{23}O_2$ |
| 448,2987 | $C_{30}H_{40}O_3$ | 237,1838 | $C_{15}H_{25}O_2$ |
| 442,2375 | $C_{26}H_{34}O_6$ | 219,1740 | $C_{15}H_{23}O$ |
| 425,2327 | $C_{26}H_{33}O_5$ | 151,0753 | $C_9H_{11}O_2$ |

**2.** Composé dénommé LL-F28249β utile contre les infections par des helminthes, des arthropodes, des ectoparasites, des acariens et des nématodes, ce composé ayant :

a) un poids moléculaire de 584 (spectrométrie de masse FAB) ;

b) une formule moléculaire correspondant à $C_{34}H_{48}O_8$ ;

c) une rotation optique spécifique correspondant à $[\alpha]_D^{26}$ = +125°(C : 0,30, acétone) ;

d) un spectre d'absorption caractéristique en ultraviolet tel qu'illustré sur la figure VI des dessins annexés ;

e) un spectre d'absorption caractéristique en infrarouge tel qu'illustré sur la figure VII des dessins annexés ;

f) un spectre caractéristique de résonance magnétique nucléaire du proton tel qu'illustré sur la figure VIII des dessins annexés ;

g) un spectre caractéristique de résonance magnétique nucléaire du carbone 13 tel qu'illustré sur la figure XXXVIII des dessins annexés, comportant des pics importants à 173,3 ; 142,6 ; 139,5 ; 137,7 ;

137,3 ; 133,9 ; 123,8 ; 123,4 ; 120,3 ; 120,2 ; 118,0 ; 99,7 ; 80,2 ; 79,4 ; 76,7 ; 69,2 ; 68,6 ; 68,3 ; 67,8 ; 67,7 ; 48,4 ; 45,7 ; 41,0 ; 40,8 ; 36,1 ; 35,9 ; 34,7 ; 22,3 ; 19,8 ; 15,5 ; 13,8 ; 13,1 ; 10,8 ; et

h) un spectre de masse à impact électronique caractéristique tel qu'illustré sur la figure IX des dessins annexés, avec des valeurs m/z mesurées et des compositions élémentaires proposées telles que mentionnées ci-dessous, obtenues par des mesures de masse à haute résolution :

| | | | |
|---|---|---|---|
| 584,3388 | $C_{34}H_{48}O_8$ | 314,1858 | $C_{20}H_{26}O_3$ |
| 566,3306 | $C_{34}H_{46}O_7$ | 278,1168 | $C_{15}H_{18}O_5$ |
| 456,2864 | $C_{28}H_{40}O_5$ | 237,1491 | $C_{14}H_{21}O_3$ |
| 442,2391 | $C_{26}H_{34}O_6$ | 219,1380 | $C_{14}H_{19}O_2$ |
| 438,2780 | $C_{28}H_{38}O_4$ | 209,1534 | $C_{13}H_{21}O_2$ |
| 425,2331 | $C_{26}H_{33}O_5$ | 191,1418 | $C_{13}H_{19}O$ |
| 354,2187 | $C_{23}H_{30}O_3$ | 151,0750 | $C_9H_{11}O_2$ |

**3.** Composé dénommé LL-F28249$\gamma$ utile contre les infections par des helminthes, des arthropodes, des ectoparasites, des acariens et des nématodes, ce composé ayant :

a) un poids moléculaire de 598 (spectrométrie de masse FAB) ;

b) une formule moléculaire correspondant à $C_{35}H_{50}O_8$ ;

c) une rotation optique spécifique correspondant à $[\alpha]_D^{26}$ = +150±4° (C : 0,3, acétone) ;

d) un spectre d'absorption caractéristique en ultraviolet tel qu'illustré sur la figure X des dessins annexés ;

e) un spectre d'absorption caractéristique en infrarouge tel qu'illustré sur la figure XI des dessins annexés ;

f) un spectre caractéristique de résonance magnétique nucléaire du proton caractéristique tel qu'illustré sur la figure XII des dessins annexés ;

g) un spectre caractéristique de résonance magnétique nucléaire du carbone 13 caractéristique tel qu'illustré sur la figure XIII des dessins annexés, comportant des pics importants à 173,6 ; 142,4 ; 139,9 ; 137,3 ; 136,0 ; 134,0 ; 123,8 ; 123,6 ; 120,4 ; 119,6 ; 118,5 ; 99,8 ; 80,5 ; 77,8 ; 77,0 ; 76,8 ; 69,3 ; 68,3 ; 67,9 ; 57,7 ; 48,5 ; 45,8 ; 41,2 ; 40,8 ; 36,2 ; 36,1 ; 36,0 ; 34,8 ; 22,3 ; 19,9 ; 15,5 ; 13,8 ; 13,1 ; 10,8 ; et

h) un spectre de masse à impact électronique caractéristique tel qu'illustré sur la figure XIV des dessins annexés, avec des valeurs m/z mesurées et des compositions élémentaires proposées telles que mentionnées ci-dessous, obtenues par des mesures de masse à haute résolution :

| | | | |
|---|---|---|---|
| 598,3543 | $C_{35}H_{50}O_8$ | 354,2199 | $C_{23}H_{30}O_3$ |
| 580,3422 | $C_{35}H_{48}O_7$ | 314,1875 | $C_{20}H_{26}O_3$ |
| 562,3292 | $C_{35}H_{46}O_6$ | 292,1307 | $C_{16}H_{20}O_5$ |
| 496,2824 | $C_{30}H_{40}O_6$ | 288,2075 | $C_{19}H_{28}O_2$ |
| 484,2440 | $C_{28}H_{36}O_7$ | 248,1397 | $C_{15}H_{20}O_3$ |
| 478,2687 | $C_{30}H_{38}O_5$ | 237,1490 | $C_{14}H_{21}O_3$ |
| 456,2576 | $C_{27}H_{36}O_6$ | 219,1382 | $C_{14}H_{19}O_2$ |
| 438,2772 | $C_{28}H_{38}O_4$ | 209,1544 | $C_{13}H_{21}O_2$ |
| 425,2341 | $C_{26}H_{33}O_5$ | 191,1435 | $C_{13}H_{19}O$ |
| 420,2651 | $C_{28}H_{36}O_3$ | 151,0759 | $C_9H_{11}O_2$ |

**4.** Composé dénommé LL-F28249$\omega$ utile contre les infections par des helminthes, des arthropodes, des ectoparasites, des acariens et des nématodes, ce composé ayant :

a) un poids moléculaire de 806 (spectrométrie de masse FAB) ;

b) une formule moléculaire correspondant à $C_{45}H_{74}O_{12}$ ;

c) une rotation optique spécifique correspondant à $[\alpha]_D^{26}$ = +49±4° (C : 0,35, méthanol) ;

d) un spectre d'absorption caractéristique en ultraviolet tel qu'illustré sur la figure XV des dessins annexés ;

e) un spectre d'absorption caractéristique en infrarouge tel qu'illustré sud la figure XVI des dessins annexés ;

f) un spectre caractéristique de résonance magnétique nucléaire du proton tel qu'illustré sur la figure XVII des dessins annexés ;

g) un spectre caractéristique de résonance magnétique nucléaire du carbone 13 tel qu'illustré sur la figure XVIII des dessins annexés, comportant des pics importants à 220,7 ; 219,6 ; 165,2 ; 148,7 ; 133,1 ; 132,3 ; 130,2 ; 122,3 ; 100,0 ; 82,9 ; 75,9 ; 73,0 ; 72,7 ; 72,6 ; 72,1 ; 69,0 ; 67,3 ; 63,6 ; 51,4 ; 46,2 ; 45,7 ; 42,2 ; 40,4 ; 38,3 ; 37,6 ; 36,1 ; 34,8 ; 33,5 ; 30,1 ; 26,6 ; 25,4 ; 24,5 ; 23,0 ; 21,1 ; 17,9 ; 14,3 ; 14,2 ; 12,1 ; 11,5 ; 10,9 ; 8,7 ; 8,3 ; 5,7 ; et

h) un spectre de masse à impact électronique caractéristique tel qu'illustré sur la figure XIX des dessins annexés, avec des valeurs m/z mesurées et des compositions élémentaires proposées telles que mentionnées ci-dessous, obtenues par des mesures de masse à haute résolution :

| | | | |
|---|---|---|---|
| 462,3350 | $C_{28}H_{46}O_5$ | 253,1797 | $C_{15}H_{25}O_3$ |
| 444,3237 | $C_{28}H_{44}O_4$ | 235,1697 | $C_{15}H_{23}O_2$ |
| 425,2534 | $C_{23}H_{37}O_7$ | 224,1754 | $C_{14}H_{24}O_2$ |
| 407,2439 | $C_{23}H_{35}O_6$ | 209,1530 | $C_{13}H_{21}O_2$ |
| 406,3046 | $C_{25}H_{42}O_4$ | 207,1744 | $C_{14}H_{23}O$ |
| 387,2895 | $C_{25}H_{39}O_3$ | 184,1458 | $C_{11}H_{20}O_2$ |
| 337,2010 | $C_{19}H_{29}O_5$ | 179,1048 | $C_{11}H_{15}O_2$ |
| 297,2031 | $C_{17}H_{29}O_4$ | 173,1205 | $C_9H_{17}O_3$ |
| 279,1944 | $C_{17}H_{27}O_3$ | 167,1051 | $C_{10}H_{15}O_2$ |
| 261,1851 | $C_{17}H_{25}O_2$ | 155,1069 | $C_9H_{15}O_2$ |

**5.** Composé dénommé LL-F28249δ utile dans le cas d'infections par des helminthes, des arthropodes, des ectoparasites, des acariens et des nématodes, ce composé ayant :

a) un poids moléculaire de 616 (spectrométrie de masse à impact électronique) ;

b) une formule moléculaire correspondant à $C_{35}H_{52}O_9$ ;

c) un volume de rétention par chromatographie HPLC de 14,0 ml ;

d) un spectre d'absorption caractéristique en ultraviolet tel qu'illustré sur la figure XX des dessins annexés ;

e) un spectre caractéristique de résonance magnétique nucléaire du proton tel qu'illustré sur la figure XXI des dessins annexés ; et

f) un spectre de masse à impact électronique caractéristique tel qu'illustré sur la figure XXII des dessins annexés.

**6.** Composé dénommé LL-F28249ε utile dans le cas d'infections par des helminthes, des arthropodes, des ectoparasites, des acariens et des nématodes, ce composé ayant :

a) un poids moléculaire de 598 (spectrométrie de masse à impact électronique) ;

b) une formule moléculaire correspondant à $C_{35}H_{50}O_8$ ;

c) un volume de rétention par chromatographie HPLC de 14,8 ml ;

d) un spectre d'absorption caractéristique en ultraviolet tel qu'illustré sur la figure XXIII des dessins annexés ;

e) un spectre caractéristique de résonance magnétique nucléaire du proton tel qu'illustré sur la figure XXIV des dessins annexés ; et

f) un spectre de masse à impact électronique caractéristique tel qu'illustré sur la figure XXV des dessins annexés.

**7.** Composé dénommé LL-F28249ζ utile dans le cas d'infections par des helminthes, des arthropodes, des ectoparasites, des acariens et des nématodes, ce composé ayant :

a) un poids moléculaire de 598 (spectrométrie de masse à impact électronique) ;

b) une formule moléculaire correspondant à $C_{35}H_{50}O_8$ ;

c) un volume de rétention par chromatographie HPLC de 16,0 ml ;

d) un spectre d'absorption caractéristique en ultraviolet tel qu'illustré sur la figure XXVI des dessins annexés ;

e) un spectre caractéristique de résonance magnétique nucléaire du proton tel qu'illustré sur la figure XXVII des dessins annexés ; et

f) un spectre de masse à impact électronique caractéristique tel qu'illustré sur la figure XXVIII des

dessins annexés.

**8.** Composé dénommé LL-F28249η utile dans le cas d'infections par des helminthes, des arthropodes, des ectoparasites, des acariens et des nématodes, ce composé ayant :
a) un poids moléculaire de 612 (spectrométrie de masse à impact électronique) ;
b) une formule moléculaire correspondant à $C_{36}H_{52}O_8$ ;
c) un volume de rétention par chromatographie HPLC de 23,5 ml ;
d) un spectre d'absorption caractéristique en ultraviolet tel qu'illustré sur la figure XXIX des dessins annexés ;
e) un spectre caractéristique de résonance magnétique nucléaire du proton tel qu'illustré sur la figure XXX des dessins annexés ; et
f) un spectre de masse à impact électronique caractéristique tel qu'illustré sur la figure XXXI des dessins annexés.

**9.** Composé dénommé LL-F28249θ utile dans le cas d'infections par des helminthes, des arthropodes, des ectoparasites, des acariens et des nématodes, ce composé ayant :
a) un poids moléculaire de 626 (spectrométrie de masse à impact électronique) ;
b) une formule moléculaire correspondant à $C_{37}H_{54}O_8$ ;
c) un volume de rétention par chromatographie HPLC de 24,5 ml ;
d) un spectre d'absorption caractéristique en ultraviolet tel qu'illustré sur la figure XXXII des dessins annexés ;
e) un spectre caractéristique de résonance magnétique nucléaire du proton tel qu'illustré sur la figure XXXIII des dessins annexés ; et
f) un spectre de masse à impact électronique caractéristique tel qu'illustré sur la figure XXXIV des dessins annexés.

**10.** Composé dénommé LL-F28249 ι utile dans le cas d'infections par des helminthes, des arthropodes, des ectoparasites, des acariens et des nématodes, ce composé ayant :
a) un poids moléculaire de 626 (spectrométrie de masse à impact électronique) ;
b) une formule moléculaire correspondant à $C_{37}H_{54}O_8$ ;
c) un volume de rétention par chromatographie HPLC de 26,0 ml ;
d) un spectre d'absorption caractéristique en ultraviolet tel qu'illustré sur la figure XXXV des dessins annexés ;
e) un spectre caractéristique de résonance magnétique nucléaire du proton tel qu'illustré sur la figure XXXVI des dessins annexés ; et
f) un spectre de masse à impact électronique caractéristique tel qu'illustré sur la figure XXXVII des dessins annexés.

**11.** Composé dénommé LL-28249κ utile dans le cas d'infections par des helminthes, des arthropodes, des ectoparasites, des acariens et des nématodes, ce composé ayant :
a) un poids moléculaire de 584 (spectrométrie de masse à impact électronique) ;
b) formule moléculaire : $C_{35}H_{52}O_7$ ;
c) rotation optique spécifique : $[\alpha]^{26}_D$ = +189° ; (C : 0,165, acétone) ;
d) spectre d'absorption ultraviolet : tel qu'illustré sur la figure XXXIX ; $UV_{MAX}$ dans $CH_3OH$ = 241 nm (E = 20 400) ;
e) spectre d'absorption infrarouge : tel qu'illustré sur la figure XL (disque de KBr) ;
f) spectre de masse à impact électronique : tel qu'illustré sur la figure XLI ;
g) spectre de résonance magnétique nucléaire du proton ($CDCl_3$) : tel qu'illustré sur la figure XLII ; et
h) spectre de résonance magnétique nucléaire de carbone 13 ($CDCl_3$) : tel qu'illustré sur la figure XLIII,et décrit dans le tableau IX.

**12.** Composé dénommé LL-28249λ utile dans le cas d'infections par des helminthes, des arthropodes, des ectoparasites, des acariens et des nématodes, ce composé ayant les caractéristiques suivantes :
a) poids moléculaire : 626 (spectrométrie de masse FAB) ;
b) formule moléculaire : $C_{37}H_{54}O_8$ ;
c) rotation optique spécifique : $[\alpha]^{26}_D$ = +145° ; (C : 0,23 ; acétone) ;
d) spectre d'absorption ultraviolet : tel qu'illustré sur la figure XLIV ; $UV_{MAX}$ dans $CH_3OH$ = 244 nm

(E = 30 000) ;
e) spectre d'absorption infrarouge : tel qu'illustré sur la figure XLV (disque de KBr) ;
f) spectre de masse à impact électronique : tel qu'illustré sur la figure XLVI ;
g) spectre de résonance magnétique nucléaire du proton ($CDCl_3$) : tel qu'illustré sur la figure XLVII ; et
h) spectre de résonance magnétique nucléaire de carbone 13 ($CDCl_3$) : tel qu'illustré sur la figure XLVIII, et décrit dans le tableau X.

**13.** Composé dénommé LL-28249μ utile dans le cas d'infections par des helminthes, des arthropodes, des ectoparasites, des acariens et des nématodes, ce composé ayant les caractéristiques suivantes :
a) poids moléculaire : 612 (spectrométrie de masse à impact électronique) ;
b) formule moléculaire : $C_{37}H_{56}O_7$ ;
c) spectre d'absorption ultraviolet : tel qu'illustré sur la figure XLIX ; $UV_{MAX}$ dans $CH_3OH$ = 241 nm (E = 16 800) ;
d) spectre d'absorption infrarouge : tel qu'illustré sur la figure L (disque de KBr) ;
e) spectre de masse à impact électronique : tel qu'illustré sur la figure LI ;
f) spectre de résonance magnétique nucléaire du proton ($CDCl_3$) : tel qu'illustré sur la figure LII.

**14.** Composé dénommé LL-28249ν utile dans le cas d'infections par des helminthes, des arthropodes, des ectoparasites, des acariens et des nématodes, ce composé ayant les caractéristiques suivantes :
a) poids moléculaire : 592 (spectrométrie de masse à impact électronique) ;
b) formule moléculaire : $C_{36}H_{48}O_7$ ;
c) rotation optique spécifique : $[\alpha]^{26}_D$ = +131° ; (C : 0,325, acétone) ;
d) spectre d'absorption ultraviolet : tel qu'illustré sur la figure LIII : $UV_{MAX}$ dans $CH_3OH$ = 256 nm (E = 20 500) ; 358 (E = 8 830) ;
e) spectre d'absorption infrarouge : tel qu'illustré sur la figure LIV (disque de KBr) ;
f) spectre de masse à impact électronique : tel qu'illustré sur la figure LV ;
g) spectre de résonance magnétique nucléaire du proton ($CDCl_3$) : tel qu'illustré sur la figure LVI ; et
h) spectre de résonance magnétique nucléaire du carbone 13 ($CDCl_3$) : tel qu'illustré sur la figure LVII, et décrit dans le tableau XI.

**15.** Procédé de préparation des agents LL-F28249α, LL-F28249β, LL-F28249γ, LL-F28249δ, LL-F28249ε, LL-F28249ζ, LL-F28249n, LL-F28249θ, LL-F28249ι, LL-F28249κ, LL-28249λ, LL-F28249μ, LL-28249ν, LL-F28249ω tels que définis dans l'une des revendications 1 à 14, selon lequel on fermente dans des conditions aerobies, l'organisme Streptomyces cyaneogriseus noncyanogenus, NRRL 15773, dans un milieu liquide contenant des sources assimilables de carbone, d'azote, ainsi que d'anions et de cations inorganiques, jusqu'à ce que l'on ait produit une importante quantité de LL-F28249α, β, γ, δ, ε, ζ, n, θ, ι, κ, λ, μ, ν et ω dans ce milieu ; et on récupère ensuite les agents à partir de ce dernier.

**16.** Procédé de préparation des agents LL-F28249α, LL-F28249β, LL-F28249γ, LL-F28249δ, LL-F28249ε, LL-F28249ζ, LL-F28249n, LL-F28249θ, LL-F28249ι, LL-F28249κ, LL-28249λ, LL-28249μ, LL-28249ν et LL-28249ω tels que définis dans l'une des revendications 1 à 14, selon lequel on fermente, dans des conditions aérobies, un milieu liquide contenant des sources assimilables de carbone, d'azote, ainsi que d'anions et de cations inorganiques, ce milieu ayant été inoculé avec une culture viable de l'organisme Streptomyces cyaneogriseus noncyanogenus, NRRL 15773 ; on maintient cette culture de fermentation dans des conditions d'aération stérile et d'agitation à une température de 24 à 32 °C pendant 80 à 200 heures ; on récupère le mélange, et on extrait les agents.

**17.** Culture biologiquement pure du microorganisme Streptomyces cyaneogriseus noncyanogenus, NRRL 15773, cette culture étant capable de produire les agents LL-F28249α, LL-F28249β, LL-F28249γ, LL-F28249δ, LL-F28249ε, LL-F28249ζ, LL-F28249n, LL-F28249θ, LL-F28249ι, LL-F28249κ, LL-28249λ, LL-F28249μ, LL-28249ν et LL-F28249 ω tels que définis dans l'une des revendications 1 à 14, selon des quantités récupérables, par fermentation dans un milieu nutritif aqueux contenant des sources assimilables de carbone, d'azote, ainsi que d'anions et de cations inorganiques.

**18.** Composé selon la revendication 1, 2, 3, 4, 5, 6, 7,8, 9, 10, 11, 12, 13 ou 14, comprenant en outre des sels pharmaceutiquement et pharmacologiquement acceptables de ceux-ci.

**19.** Procédé de lutte contre les nématodes des végétaux, selon lequel on applique sur le feuillage des végétaux, le substrat sur lequel ils sont cultivés, ou leurs tiges, une quantité à effet nématicide du bouillon ou du mélange complet de fermentation du micro-organisme Streptomyces cyaneogriseus noncyanogenus correspondant au numéro de dépôt NRRL 15773.

**20.** Procédé de lutte contre les nématodes des végétaux, selon lequel on applique sur le feuillage des végétaux, le substrat sur lequel ils sont cultivés, ou leurs tiges, une quantité à effet nématicide du bouillon ou du mélange complet de fermentation du micro-organisme Streptomyces cyanogriseus noncyanogenus correspondant au numéro de dépôt NRRL 15773, contenant les agents dénommés LL-F28249α, LL-F28249β, LL-F28249γ, LL-F28249δ, LL-F28249ε, LL-F28249ζ, LL-F28249n, LL-F28249θ, LL-F28249ι, LL-F28249κ, LL-28249λ, LL-28249μ, LL-28249ν et LL-28249ω tels que définis dans l'une des revendications 1 à 14 ; ou leurs sels pharmaceutiquement ou pharmacologiquement acceptables.

**21.** Procédé selon la revendication 19 ou 20, dans lequel l'application est effectuée selon une dose de 0,1 à 1,4 kg par ha.

**22.** Composition alimentaire pour animaux destinée à prévenir, à traiter ou à lutter contre les infections par les helminthes, les arthropodes, les ectoparasites ou les acariens chez les animaux producteurs de viande, cette composition alimentaire pour animaux comprenant : un véhicule solide comestible ; et une quantité à effet prophylactique, thérapeutique ou pharmaceutique du bouillon ou de l'ensemble du mélange de fermentation du microorganisme Streptomyces cyaneogriseus noncyanogenus correspondant au numéro de dépôt NRRL 15773.

**23.** Composition formant prémélange alimentaire pour animaux destinée à la prévention, au traitement ou la lutte contre les infections par les helminthes, les arthropodes, les ectoparasites ou les acariens, chez les animaux producteurs de viande, cette composition formant prémélange alimentaire pour animaux comprenant un véhicule comestible ; et une quantité à effet prophylactique, thérapeutique ou pharmaceutique du bouillon ou de l'ensemble du mélange de fermentation du micro-organisme Streptomyces cyanogriseus noncyanogenus correspondant au numéro de dépôt NRRL 15773, contenant les agents dénommés LL-F28249α, LL-F28249β, LL-F28249γ, LL-F28249δ, LL-F28249ε, LL-F28249ζ, LL-F28249n, LL-F28249θ, LL-F28249ι, LL-F28249κ, LL-28249λ, LL-F28249μ, LL-28249ν et et LL-F28249ω tels que définis dans l'une des revendications 1 à 14 ; ou les sels pharmaceutiquement ou pharmacologiquement acceptables de ceux-ci.

**24.** Composition selon la revendication 22 ou 23, dans laquelle cette quantité efficace est d'environ 0,00001 % à 5 % par rapport au poids de cette composition.

**Patentansprüche**

**1.** Die mit LL-F28249α bezeichnete Verbindung, die brauchbar ist bei Infektionen durch Helminthen, Arthropoden, Ektoparasiten und bei Infektionen durch Milben und Nematoden, gekennzeichnet durch folgende Eigenschaften:

(a) ein Molekulargewicht von 612 (FAB-MS);
(b) eine Molekülformel $C_{36}H_{52}O_8$;
(c) eine spezifische optische Drehung $[\alpha]^{26}D = +133\pm3°$ (C 0,3 Aceton);
(d) ein charakteristisches UV-Absorptionsspektrum gemäß der beigefügten Fig. I;
(e) ein charakteristisches IR-Absorptionsspektrum gemäß der beigefügten Fig. II;
(f) ein charakteristisches Protonen-kernmagnetisches Resonanz(PMR)-Spektrum gemäß der beigefügten Fig. III;
(g) ein charakteristisches Kohlenstoff-13-kernmagnetisches Resonanz($^{13}$C-NMR)-Spektrum gemäß der beigefügten Fig. IV mit signifikanten Signalen bei 173,4, 142,8, 139,4, 137,7, 137,3, 137,2, 130,6, 123,3, 120,3, 118,0, 99,7, 80,2, 79,3, 76,7, 69,3, 68,5, 68,4, 67,8, 67,7, 48,4, 45,7, 41,1, 40,7, 36,1, 36,0, 35,9, 34,7, 26,8, 22,8, 22,2, 19,9, 15,5, 13,9, 11,0,; und
(h) ein charakteristisches Elektronenstoß-Massen-(EM)-Spektrum gemäß der beigefügten Fig. V und mit den nachstehend angegeben, durch hochauflösende Massenbestimmung erhaltenen, gemessenen m/z-Werten und zugeordneten Elementarzusammensetzungen

| | | | |
|---|---|---|---|
| 612.3705 | $C_{36}H_{52}O_8$ | 354.2181 | $C_{23}H_{30}O_3$ |
| 594.3543 | $C_{36}H_{50}O_7$ | 314.1877 | $C_{20}H_{26}O_3$ |
| 576.3472 | $C_{36}H_{48}O_6$ | 278.1144 | $C_{15}H_{18}O_5$ |
| 484.3211 | $C_{30}H_{44}O_5$ | 265.1786 | $C_{16}H_{25}O_3$ |
| 482.2648 | $C_{29}H_{38}O_6$ | 248.1405 | $C_{15}H_{20}O_3$ |
| 466.3097 | $C_{30}H_{42}O_4$ | 247.1705 | $C_{16}H_{23}O_2$ |
| 448.2987 | $C_{30}H_{40}O_3$ | 237.1838 | $C_{15}H_{25}O_2$ |
| 442.2375 | $C_{26}H_{34}O_6$ | 219.1740 | $C_{15}H_{23}O$ |
| 425.2327 | $C_{26}H_{33}O_5$ | 151.0753 | $C_9H_{11}O_2$ |

**2.** Die mit LL-F28249$\beta$ bezeichnete Verbindung, die brauchbar ist bei Infektionen durch Helminthen, Arthropoden, Ektoparasiten und bei Infektionen durch Milben und Nematoden, gekenzeichnet durch folgende Eigenschaften:

(a) ein Molekulargewicht von 584 (FAB-MS);
(b) eine Molekülformel $C_{34}H_{48}O_8$;
(c) eine spezifische optische Drehung $[\alpha]^{26}{}_D$ = +125° (C 0,30, Aceton)
(d) ein charakteristisches UV-Absorptionsspektrum gemäß der beigefügten Fig. VI;
(e) ein charakteristisches IR-Absorptionsspektrum gemäß der beigefügten Fig. VII;
(f) ein charakteristisches PMR-Sektrum gemäß der beigefügten Fig. VIII;
(g) ein charakteristisches $^{13}$C-NMR-Spektrum gemäß der beigefügten Fig. XXXVIII mit signifikanten Signalen bei 173,3, 142,6, 139,5, 137,7, 137,3, 133,9, 123,8, 123,4, 120,3, 120,2, 118,0, 99,7, 80,2, 79,4, 76,7, 69,2, 68,6, 68,3, 67,8, 67,7, 48,4, 45,7, 41,0, 40,8, 36,1, 35,9, 34,7, 22,3, 19,8, 15,5, 13,8, 13,1, 10,8; und
(h) ein charakteristisches EM-Spektrum gemäß der beigefügten Fig. IX und mit den nachstehend angegebenen, durch hochauflösende Massenbestimmung erhaltenen, gemessenen m/z-Werten und zugeordneten Elementarzusammensetzungen

| | | | |
|---|---|---|---|
| 584.3388 | $C_{34}H_{48}O_8$ | 314.1858 | $C_{20}H_{26}O_3$ |
| 566.3306 | $C_{34}H_{46}O_7$ | 278.1168 | $C_{15}H_{18}O_5$ |
| 456.2864 | $C_{28}H_{40}O_5$ | 237.1491 | $C_{14}H_{21}O_3$ |
| 442.2391 | $C_{26}H_{34}O_6$ | 219.1380 | $C_{14}H_{19}O_2$ |
| 438.2780 | $C_{28}H_{38}O_4$ | 209.1534 | $C_{13}H_{21}O_2$ |
| 425.2331 | $C_{26}H_{33}O_5$ | 191.1418 | $C_{13}H_{19}O$ |
| 354.2187 | $C_{23}H_{30}O_3$ | 151.0750 | $C_9H_{11}O_2$. |

**3.** Die mit LL-F28249$\gamma$ bezeichnete Verbindung, die brauchbar ist bei Infektionen durch Helminthen, Arthropoden, Ektoparasiten und bei Infektionen durch Milben und Nematoden, gekenzeichnet durch folgende Eigenschaften:

(a) ein Molekulargewicht von 598 (FAB-MS);
(b) eine Molekülformel $C_{35}H_{50}O_8$;
(c) eine spezifische optische Drehung $[\alpha]^{26}{}_D$ = +150±4° (C 0,3, Aceton);
(d) ein charakteristisches UV-Absorptionsspektrum gemäß der beigefügten Fig. X;
(e) ein charakteristisches IR-Absorptionsspektrum gemäß der beigefügten Fig. XI;
(f) ein charakteristisches PMR-Spektrum gemäß der beigefügten Fig. XII;
(g) ein charakteristisches $^{13}$C-NMR-Spektrum gemäß der bei gefügten Fig. XIII mit signifikanten Signalen bei 173,6, 142,4, 139,9, 137,3, 136,0, 134,0, 123,8, 123,6, 120,4, 119,6, 118,5, 99,8, 80,5, 77,8, 77,0, 76,8, 69,3, 68,6, 68,3, 67,9, 57,7, 48,5, 45,8, 41,2, 40,8, 36,2, 36,1, 36,0, 34,8, 22,3, 19,9, 15,5, 13,8, 13,1, 10,8; und
(h) ein charakteristisches EM-Spektrum gemäß der beigefügten Fig. XIV und mit den nachstehend angegebenen, durch hochauflösende Massenbestimmung erhaltenen, gemessenen m/z-Werten und zugeordneten Elementarzusammensetzungen

| | | | |
|---|---|---|---|
| 598.3543 | $C_{35}H_{50}O_8$ | 354.2199 | $C_{23}H_{30}O_3$ |
| 580.3422 | $C_{35}H_{48}O_7$ | 314.1875 | $C_{20}H_{26}O_3$ |
| 562.3292 | $C_{35}H_{46}O_6$ | 292.1307 | $C_{16}H_{20}O_5$ |
| 496.2824 | $C_{30}H_{40}O_6$ | 288.2075 | $C_{19}H_{28}O_2$ |
| 484.2440 | $C_{28}H_{36}O_7$ | 248.1397 | $C_{15}H_{20}O_3$ |
| 478.2687 | $C_{30}H_{38}O_5$ | 237.1490 | $C_{14}H_{21}O_3$ |
| 456.2576 | $C_{27}H_{36}O_6$ | 219.1382 | $C_{14}H_{19}O_2$ |
| 438.2772 | $C_{28}H_{38}O_4$ | 209.1544 | $C_{13}H_{21}O_2$ |
| 425.2341 | $C_{26}H_{33}O_5$ | 191.1435 | $C_{13}H_{19}O$ |
| 420.2651 | $C_{28}H_{36}O_3$ | 151.0759 | $C_9H_{11}O_2$. |

**4.** Die mit LL-F28249ω bezeichnete Verbindung, die brauchbar ist bei Infektionen durch Helminthen, Arthropoden, Ektoparasiten und bei Infektionen durch Milben und Nematoden, gekennzeichnet durch folgende Eigenschaften:

(a) ein Molekulargewicht von 806 (FAB-MS);
(b) eine Molekülformel $C_{45}H_{74}O_{12}$;
(c) eine spezifische optische Drehung $[\alpha]^{26}_D$ = -49±4° (C 0,35, Methanol);
(d) ein charakteristisches UV-Absorptionsspektrum gemäß der beigefügten Fig. XV;
(e) ein charakteristisches IR-Absorptionsspektrum gemäß der beigefügten Fig. XVI;
(f) ein charakteristisches PMR-Spektrum gemäß der beigefügten Fig. XVII;
(g) ein charakteristisches $^{13}$C-NMR-Spektrum gemäß der beigefügten Fig. XVIII mit signifikanten Signalen bei 220,7, 219,6, 165,2, 148,7 133,1, 132,3, 130,2, 122,3, 100,0, 82,9, 75,9, 73,0, 72,7, 72,6, 72,1, 69,0, 67,3, 63,6, 51,4, 46,2, 45,7, 42,2, 40,4, 38,3, 37,6, 36,1, 34,8, 33,5, 30,1, 26,6, 25,4, 24,5, 23,0, 21,1, 17,9, 14,3, 14,2, 12,1, 11,5, 10,9, 8,7, 8,3, 5,7; und
(h) ein charakteristisches EM-Spektrum gemäß der beigefügten Fig. XIX und mit den nachstehend angegebenen, durch hochauflösende Massenbestimmung erhaltenen, gemessenen m/z-Werten und zugeordneten Elementarzusammensetzungen

| | | | |
|---|---|---|---|
| 462.3350 | $C_{28}H_{46}O_5$ | 253.1797 | $C_{13}H_{25}O_3$ |
| 444.3237 | $C_{28}H_{44}O_4$ | 235.1697 | $C_{15}H_{23}O_2$ |
| 425.2534 | $C_{23}H_{37}O_7$ | 224.1754 | $C_{14}H_{24}O_2$ |
| 407.2439 | $C_{23}H_{35}O_6$ | 209.1530 | $C_{13}H_{21}O_2$ |
| 406.3046 | $C_{25}H_{42}O_4$ | 207.1744 | $C_{14}H_{23}O$ |
| 387.2895 | $C_{25}H_{39}O_3$ | 184.1458 | $C_{11}H_{20}O_2$ |
| 337.2010 | $C_{19}H_{29}O_5$ | 179.1048 | $C_{19}H_{15}O_2$ |
| 297.2031 | $C_{17}H_{29}O_4$ | 173.1205 | $C_9H_{17}O_3$ |
| 279.1944 | $C_{17}H_{27}O_3$ | 167.1051 | $C_{10}H_{15}O_2$ |
| 261.1851 | $C_{17}H_{25}O_2$ | 155.1069 | $C_9H_{15}O_2$ |

**5.** Die mit LL-F28249δ bezeichnete Verbindung, die brauchbar ist bei Infektionen durch Helminthen, Arthropoden, Ektoparasiten und bei Infektionen durch Milben und Nematoden, gekennzeichnet durch folgende Eigenschaften:

(a) ein Molekulargewicht von 616 (EMS);
(b) eine Molekülformel $C_{35}H_{52}O_9$;
(c) ein HPLC-Retentionsvolumen von 14,0 ml;
(d) ein charakteristisches UV-Absorptionsspektrum gemäß der beigefügten Fig. XX
(e) ein charakteristisches PMR-Spektrum gemäß der beigefügten Fig. XXX; und
(f) ein charakteristisches EM-Spektrum gemäß der beigefügten Fig. XXII.

**6.** Die mit LL-F28249ε bezeichnete Verbindung, die brauchbar ist bei Infektionen durch Helminthen, Arthropoden, Ektoparasiten und bei Infektionen durch Milben und Nematoden, gekennzeichnet durch folgende Eigenschaften:

(a) ein Molekulargewicht von 598 (EMS);
(b) eine Molekülformel $C_{35}H_{50}O_8$;

(c) ein HPLC-Retentionsvolumen von 14,8;
(d) ein charakteristisches UV-Absorptionsspektrum gemäß der beigefügten Fig. XXIII;
(e) ein charakteristisches PMR-Spektrum gemäß der beigefügten Fig. XXIV; und
(f) ein charakteristisches EM-Spektrum gemäß der beigefügten Fig. XXV.

**7.** Die mit LL-F28249ζ bezeichnete Verbindung, die brauchbar ist bei Infektionen durch Helminthen, Arthropoden, Ektoparasiten und bei Infektionen durch Milben und Nematoden, gekennzeichnet durch folgende Eigenschaften:
(a) ein Molekulargewicht von 598 (EMS);
(b) eine Molekülformel $C_{35}H_{50}O_8$,
(c) ein HPLC-Retentionsvolumen von 16,0 ml;
(d) ein charakteristisches UV-Absorptionsspektrum gemäßder beigefügten Fig. XXVI;
(e) ein charakteristisches PMR-Spektrum gemäß der beigefügten Fig. XXVII; und
(f) ein charakteristisches EM-Spektrum gemäß der beigefügten Fig. XXVIII.

**8.** Die mit LL-F28249η bezeichnete Verbindung, die brauchbar ist bei Infektionen durch Helminthen, Arthropoden, Ektoparasiten und bei Infektionen durch Milben und Nematoden, gekennzeichet durch folgende Eigenschaften:
(a) ein Molekulargewicht von 612 (EMS);
(b) eine Molekülformel $C_{36}H_{52}O_8$;
(c) ein HPLC-Rtentionsvolumen von 23,5 ml;
(d) ein charakteristisches UV-Absorptionsspektrum gemäß der beigefügten Fig. XXIX;
(e) ein charakteristisches PMR-Spektrum gemäß der beigefügten Fig. XXX; und
(f) ein charakteristisches EM-Spektrum gemäß der beigefügten Fig. XXXI.

**9.** Die mit LL-F28249θ bezeichnete Verbindung, die brauchbar ist bei Infektionen durch Helminthen, Arthropoden, Ektoparasiten und bei Infektionen durch Milben und Nematoden, gekennzeichnet durch folgende Eigenschaften:
(a) ein Molekulargewicht von 626 (EMS);
(b) eine Molekülformel $C_{37}H_{54}O_8$;
(c) ein HPLC-Retentionsvolumen von 24,5 ml;
(d) ein charakteristisches UV-Absorptiosspektrum gemäß der beigefügten Fig. XXXII;
(e) ein charakteristisches PMR-Spektrum gemäß der beigefügten Fig. XXXIII; und
(f) ein charakteristisches EM-Spektrum gemäß der beigefügten Fig. XXXIV.

**10.** Die mit LL-F28249ι bezeichnete Verbindung, die brauchbar ist bei Infektionen durch Helminthen, Arthropoden, Ektoparasiten und bei Infektionen durch Milben und Nematoden, gekennzeichnet durch folgende Eigenschaften:
(a) ein Molekulargewicht von 626 (EMS);
(b) eine Molekülformel $C_{37}H_{54}O_8$;
(c) ein HPLC-Retentionsvolumen von 26,0 ml;
(d) ein charakteristisches UV-Absorptionsspektrum gemäß der beigefügten Fig. XXXV;
(e) ein charakteristisches PMR-Spektrum gemäß der beigefügten Fig. XXXVI; und
(f) ein charakteristisches EM-Spektrum gemäß der beigefügten Fig. XXXVII

**11.** Die mit LL-F28249κ bezeichnete Verbindung, die brauchbar ist bei Infektionen durch Helminthen, Arthropoden, Ektoparasiten und bei Infektionen durch Milben und Nematoden, gekennzeichnet durch folgende Eigenschaften:
(a) ein Molekulargewicht von 584 (EMS);
(b) eine Molekülformel $C_{35}H_{52}O_7$;
(c) eine spezifische optische Rotation $[\alpha]^{26}_D = +189°$ (C 0,165, Aceton);
(d) ein UV-Absorptionsspektrum gemäß Fig. XXXIX

$$UV^{CH_3OH}_{max} = 241\ nm\ (E\ 20\ 400)$$

(e) ein IR-Absorptionsspektrum gemäß Fig. XL (KBr-Scheibe);

(f) ein EM-Spektrum gemäß Fig. XLI;
(g) ein PMR-Spektrum ($CDCl_3$ wie in Fig. XLIII gezeigt und in Tabelle IX beschrieben.

**12.** Die mit LL-F28249λ bezeichnete Verbindung, die brauchbar ist bei Infektionen durch Helminthen, Arthropoden, Ektoparasiten und bei Infektionen durch Milben und Nematoden, gekennzeichnet durch folgende Eigenschaften:
(a) ein Molekulargewicht von 626 (FAB-MS);
(b) eine Molekülformel $C_{37}H_{54}O_8$;
(c) eine spezifische optische Drehung $[\alpha]^{26}_D$ = +145° (C 0,23, Aceton);
(d) ein UV-Absorptionsspektrum gemäß Fig. XLIV

$$UV^{CH_3OH}_{max} = 244nm\ (E\ 30\ 000);$$

(e) ein IR-Absorptionsspektrum gemäß Fig. XLV (KBr-Scheibe);
(f) ein EM-Spektrum gemäß Fig. XLVI;
(g) ein PMR-Spektrum ($CDCl_3$) gemäß Fig. XLVII; und
(h) ein $^{13}C$-NMR-Spektrum ($CDCl_3$) wie in Fig. XLVIII gezeigt und in Tabelle X beschrieben.

**13.** Die mit LL-F28249μ bezeichnete Verbindung, die brauchbar ist bei Infektionen durch Helminthen, Arthropoden, Ektoparasiten und bei Infektionen durch Milben und Nematoden, gekennzeichnet durch folgende Eigenschaften:
(a) ein Molekulargewicht von 612 (EMS);
(b) eine Molekülformel $C_{37}H_{56}O_7$;
(c) ein UV-Absorptionsspektrum gemäß Fig. XLIX

$$UV^{CH_3OH}_{max} = 241\ nm\ (E\ 16\ 800);$$

(d) ein IR-Absorptionsspektrum gemäß Fig. L (KBr-Scheibe);
(e) ein EM-Spektrum gemäß Fig. LI; und
(f) ein PMR-Spektrum ($CDCl_3$ gemäß Fig. LII.

**14.** Die mit LL-F28249ν bezeichnete Verbindung, die brauchbar ist bei Infektionen durch Helminthen, Arthropoden, Ektoparasiten und bei Infektionen durch Milben und Nematoden, gekennzeichnet durch folgende Eigenschaften:
(a) ein Molekulargewicht von 592 (EMS);
(b) eine Molekülformel $C_{36}H_{48}O_7$;
(c) eine spezifische optische Drehung $[\alpha]^{26}_D$ = + 131° (C 0,325, Aceton);
(d) ein UV-Absorptionsspektrum gemäß Fig. LIII

$$UV^{CH_3OH}_{max} = 256\ (E\ 20\ 500);$$

(e) ein IR-Absorptionsspektrum gemäß Fig. LIV (KBr-Scheibe);
(f) ein EM-Spektrum gemäß Fig. LV;
(g) ein PMR-Spektrum ($CDCl_3$) gemäß Fig. LVI; und
(h) ein $^{13}C$-NMR-Spektrum ($CDCl_3$) wie in Fig. LVII gezeigt und in Tabelle XI beschrieben.

**15.** Verfahren zur Herstellung der Wirkstoffe LL-F28249α, LL-F28249β, LL-F28249γ, LL-F28249δ, LL-F28249ε, LL-F28249ζ, LL-F28249η, LL-F28249θ, LL-F28249 ι, LL-F28249κ, LL-F28249λ, LL-F28249μ, LL-F28249ν und LL-F28249ω, wie in einem der Ansprüche 1 bis 14 definiert, umfassend die Kultivierung des Organismus Streptomyces cyaneogriseus noncyanogenus, NRRL 15773, in einem flüssigen Medium, das assimilierbare Quellen von Kohlenstoff, Stickstoff und anorganischen Anionen und Kationen enhält, unter aerobischen Bedingungen, bis eine wesentliche Menge an LL-F28249α, β, γ, δ, ε,

ζ, η, θ, ι, κ, λ, μ, ν und ω in dem Medium gebildet wurde, und nachfolgende Isolierung der Wirkstoffe aus dem Medium.

**16.** Verfahren zur Herstellung der Wirkstoffe LL-F28249α, LL-F28249β, LL-F28249γ, LL-F28249δ, LL-F28249ε, LL-F28249ζ, LL-F28249η, LL-F28249θ, LL-F28249ι, LL-F28249κ, LL-F28249λ, LL-F28249μ, LL-F28249ν und LL-F28249ω, wie in einem der Ansprüche 1 bis 14 definiert, umfassend die Kultivierung des Organismus Streptomyces cyaneogriseus noncyanogenus, NRRL 15773 in einem flüssigen Medium, das assimilierbare Quellen von Kohlenstoff, Stickstoff und anorganischen Anionen und Kationen enhält, unter aerobischen Bedingungen, das Halten der Fermentationskultur unter steriler Belüftung und Rühren bei einer Temperatur von 24 bis 32 °C während eines Zeitraums von 80 bis 200 h; Ernten der Maische; und Extraktion der Wirkstoffe.

**17.** Biologisch reine Kultur des Mikroorganismus Streptomyces cyaneogriseus noncyanogenus, NRRL 15773, wobei die Kultur in der Lage ist, die Wirkstoffe LL-F28249α, LL-F28249β, LL-F28249γ, LL-F28249δ, LL-F28249ε, LL-F28249ζ, LL-F28249η, LL-F28249θ, LL-F28249ι, LL-F28249κ, LL-F28249λ, LL-F28249μ, LL-F28249ν und LL-F28249ω, wie in einem der Ansprüche 1 bis 14 definiert, bei Fermentierung in einem wässrigen Nährstoffmedium, das assimilierbare Quellen von Kohlenstoff, Stickstoff und anorganischen Anionen und Kationen enthält, in isolierbaren Mengen zu erzeugen.

**18.** Verbindung gemäß Anspruch 1, 2, 3, 4, 5, 6, 7, 8, 9, 10, 11, 12, 13 oder 14, zusätzlich umfassend die pharmazeutisch und pharmakologisch akzeptablen Salze derselben.

**19.** Verfahren zur Bekämpfung von Pflanzennematoden, dadurch gekennzeichnet, daß man auf die Blätter der Pflanzen, den Boden, in dem die Pflanzen wachsen, oder in den Hauptteil der Pflanzen eine nematozid wirksame Menge der Fermentationsbrühe oder Gesamtmaische des Mikroorganismus Streptomyces cyaneogriseus noncyanogenus mit der Hinterlegungsnummer NRRL 15773 appliziert.

**20.** Verfahren zur Bekämpfung von Pflanzennematoden, dadurch gekennzeichnet, daß man auf die Blätter der Pflanzen, den Boden, in dem die Pflanzen wachsen, oder in den Hauptteil der Pflanzen eine nematozid wirksame Menge der Fermentationsbrühe oder Gesamtmaische des Mikroorganismus Streptomyces cyaneogriseus noncyanogenus mit der Hinterlegungsnummer NRRL 15773, enthaltend die Wirkstoffe LL-F28249α, LL-F28249β, LL-F28249γ, LL-F28249δ, LL-F28249ε, LL-F28249ζ, LL-F28249η, LL-F28249θ, LL-F28249ι, LL-F28249κ, LL-F28249λ, LL-F28249μ, LL-F28249ν und LL-F28249ω, wie in einem der Ansprüche 1 bis 14 definiert; oder die pharmazeutisch und pharmakologisch akzeptablen Salze derselben appliziert.

**21.** Verfahren nach einem der Ansprüche 19 oder 20, wobei man 0,1 bis 1,4 kg/ha appliziert.

**22.** Tierfuttermittel-Zusammensetzung zur Verhinderung, Behandlung oder Bekämpfung von durch Helminthen, arthropoide Ektoparasiten oder Milben verursachte Infektionen bei fleischerzeugenden Tieren, dadurch gekennzeichnet, daß die Tierfuttermittel-Zusammensetzung folgende Komponenten umfaßt: einen eßbaren, festen Träger sowie eine prophylaktische, therapeutische oder pharmazeutisch wirksame Menge der Fermentationsbrühe oder Gesamtmaische des Mikroorganismus Streptomyces cyaneogriseus noncyanogenus mit der Hinterlegungsnummer NRRL 15773.

**23.** Tierfuttermittel-Prämixzusammensetzung zur Verhinderung, Behandlung oder Bekämpfung von durch Helminthen, arthropoide Ektoparasiten oder Milben verursachte Infektionen bei fleischerzeugenden Tieren, dadurch gekennzeichnet, daß die Tierfuttermittel-Prämixzusammensetzung folgende Komponenten umfaßt: einen eßbaren, festen Träger sowie eine prophylaktische, therapeutische oder pharmazeutisch wirksame Menge der Fermentationsbrühe oder Gesamtmaische des Mikroorganismus Streptomyces cyaneogriseus noncyanogenus mit der Hinterlegungsnummer NRRL 15773, enthaltend die Wirkstoffe LL-F28249α, LL-F28249β, LL-F28249γ, LL-F28249δ, LL-F28249ε, LL-F28249ζ, LL-F28249η, LL-F28249θ, LL-F28249ι, LL-F28249κ, LL-F28249λ, LL-F28249μ, LL-F28249ν und LL-F28249ω, wie in einem der Ansprüche 1 bis 14 definiert; oder die pharmazeutisch und pharmakologisch akzeptablen Salze derselben.

**24.** Zusammensetzung gemäß Anspruch 22 oder 23, wobei die wirksame Menge etwa 0,00001 bis 5 Gew.% der Zusammensetzung beträgt.

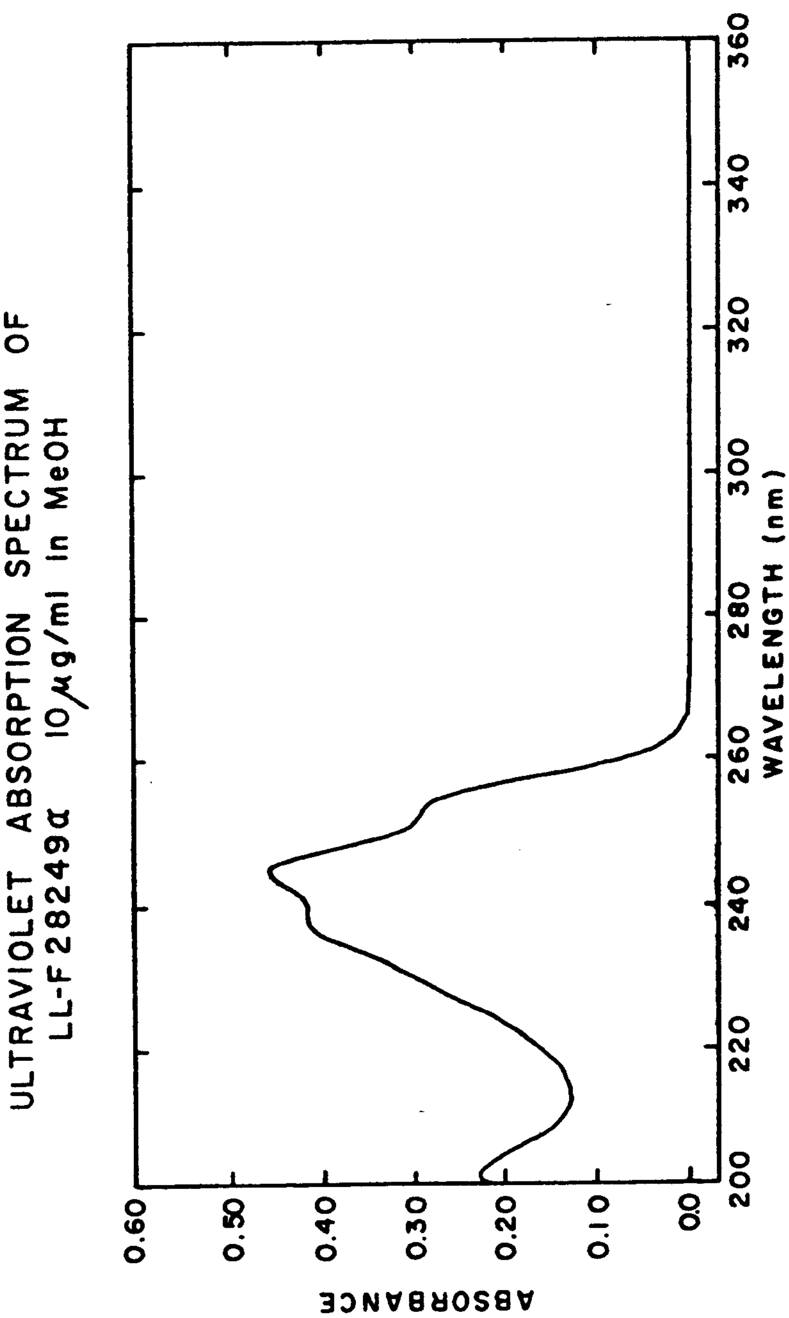
ULTRAVIOLET ABSORPTION SPECTRUM OF
LL-F28249α 10μg/ml in MeOH
ABSORBANCE
0.60
0.50
0.40
0.30
0.20
0.10
0.0
200
220
240
260
280
300
320
340
360
WAVELENGTH (nm)

FIGURE 1

INFRARED ABSORPTION SPECTRUM
OF LL-F28249α (KBr disc)

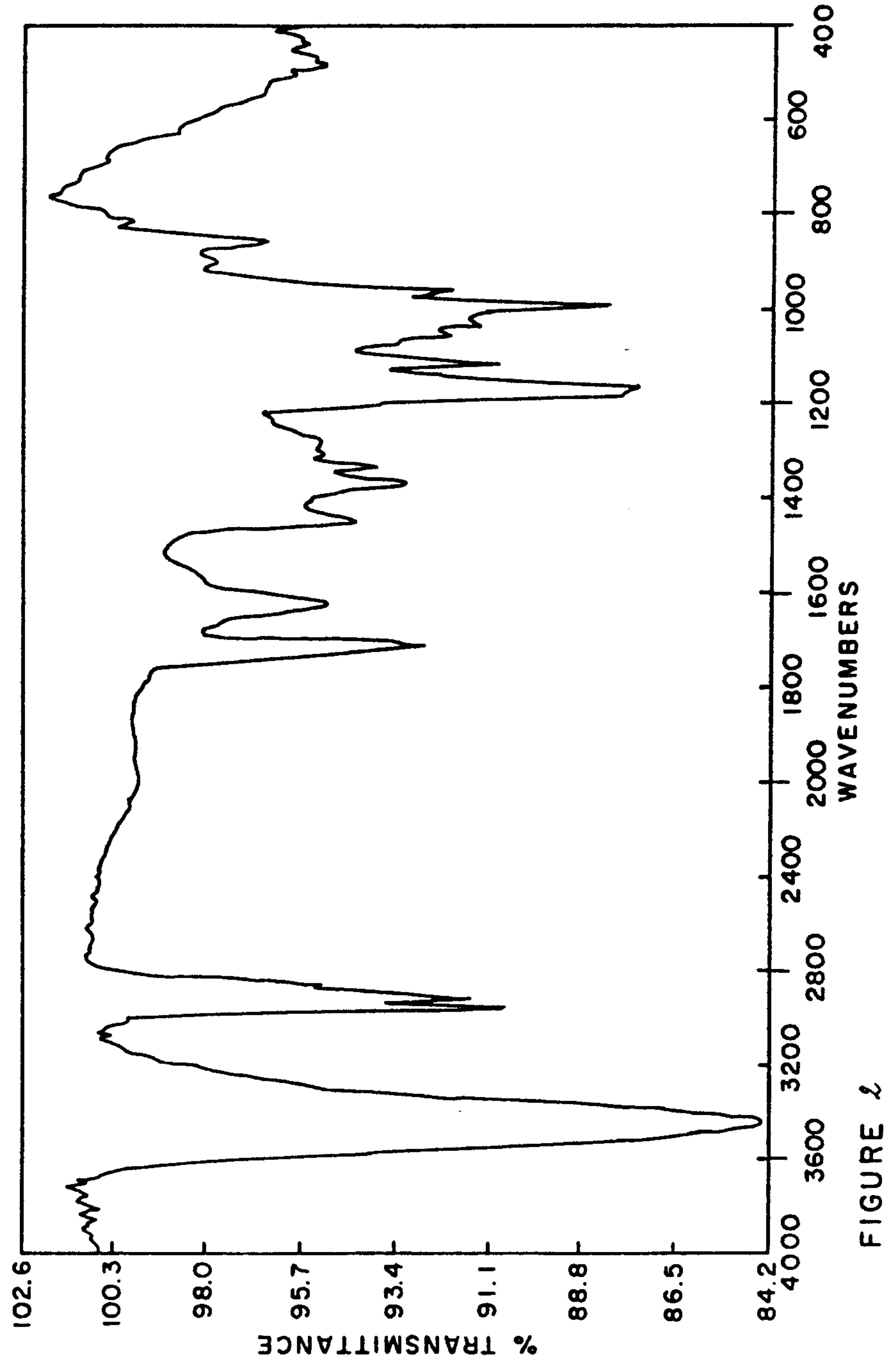


FIGURE 2

PROTON NUCLEAR MAGNETIC RESONANCE SPECTRUM
OF LL-F28249α IN $CDCl_3$ SOLUTION

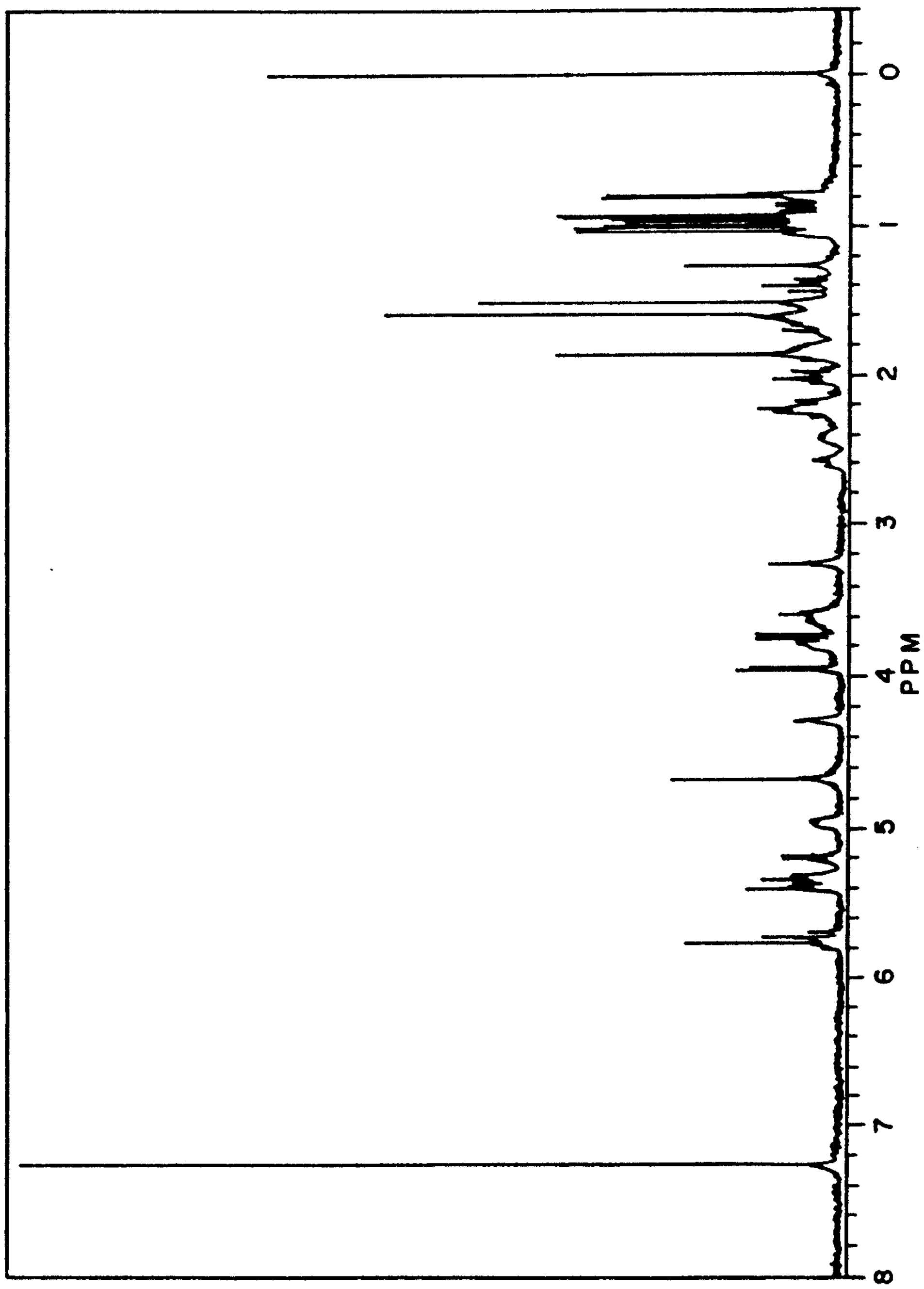


FIGURE 3

CARBON-13 NUCLEAR MAGNETIC RESONANCE SPECTRUM
OF LL-F28249α IN $CDCl_3$ SOLUTION

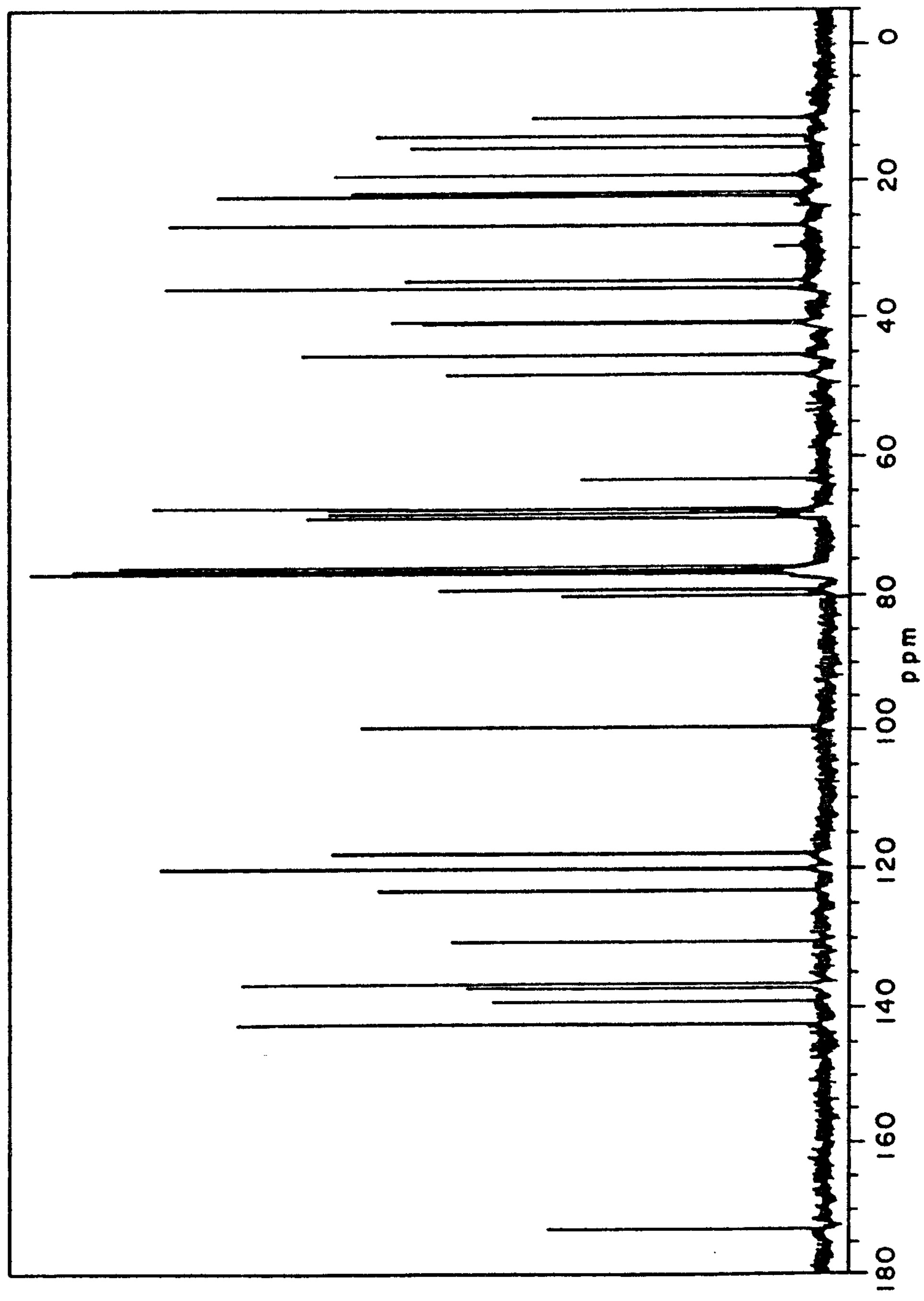


FIGURE 4

ELECTRON IMPACT MASS SPECTRUM
OF LL-F28249α

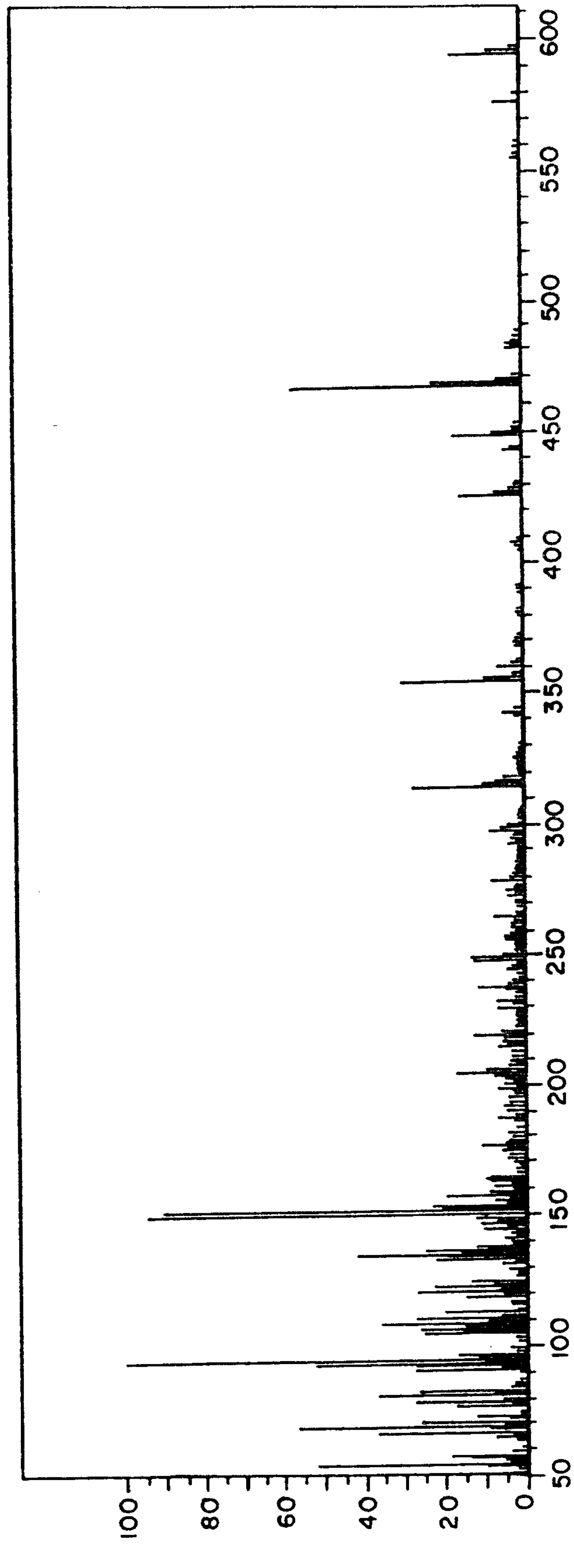


FIGURE 5

ULTRAVIOLET ABSORPTION SPECTRUM OF LL-F28249β
10 μg/ml in MeOH

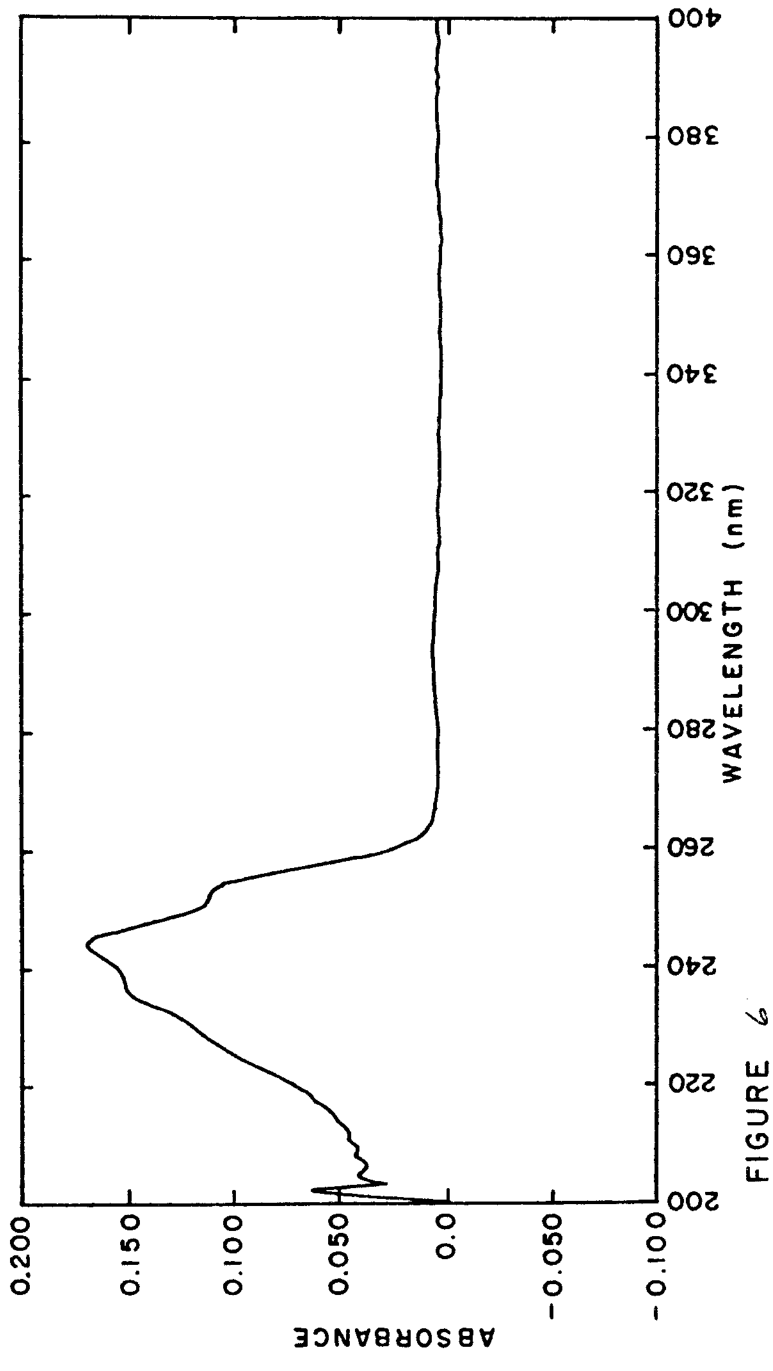


FIGURE 6

INFRARED ABSORPTION SPECTRUM
OF LL-28249β (KBr disc)

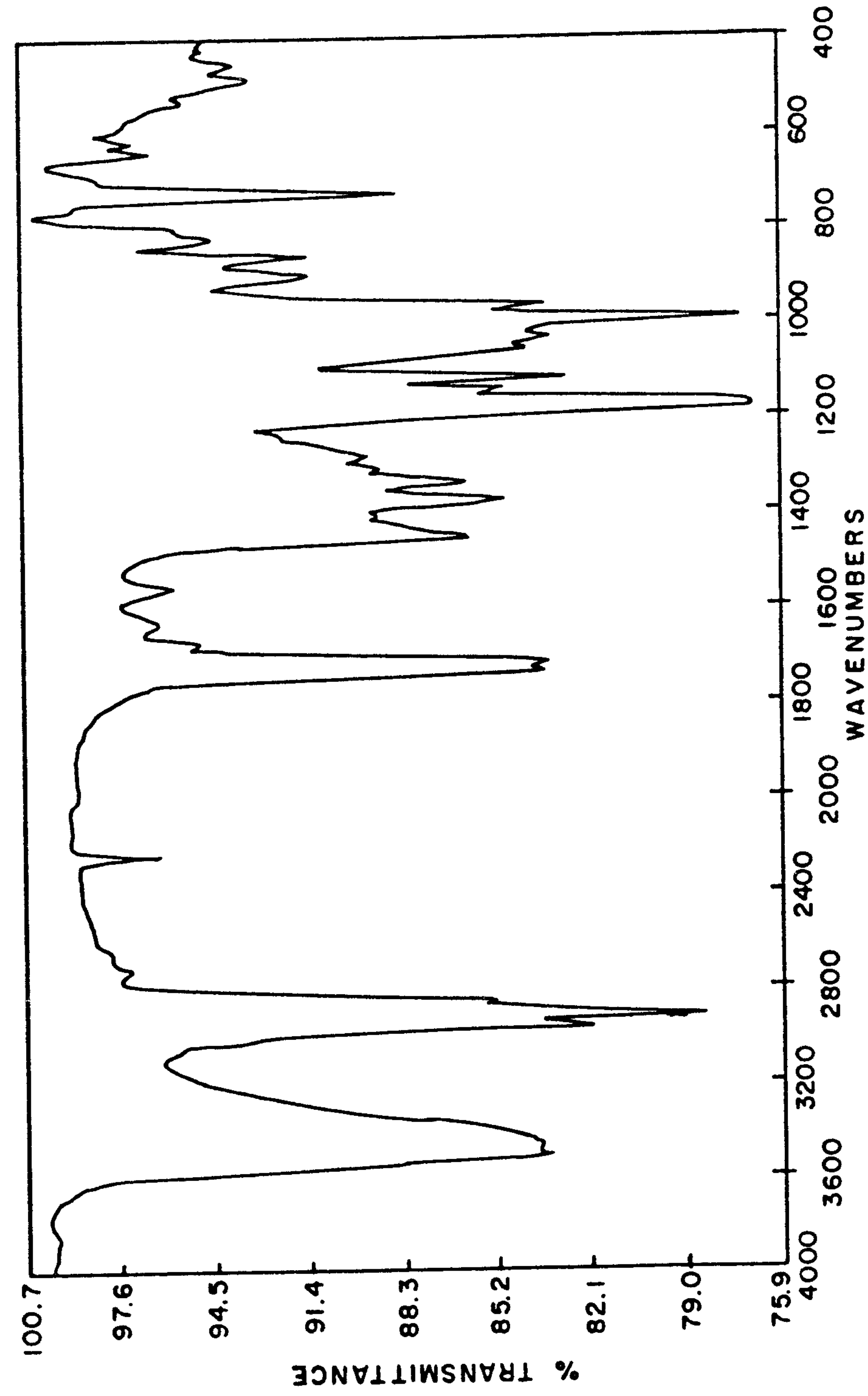


FIGURE 7

PROTON NUCLEAR MAGNETIC RESONANCE SPECTRUM
OF LL-F28249β IN $CDCl_3$ SOLUTION

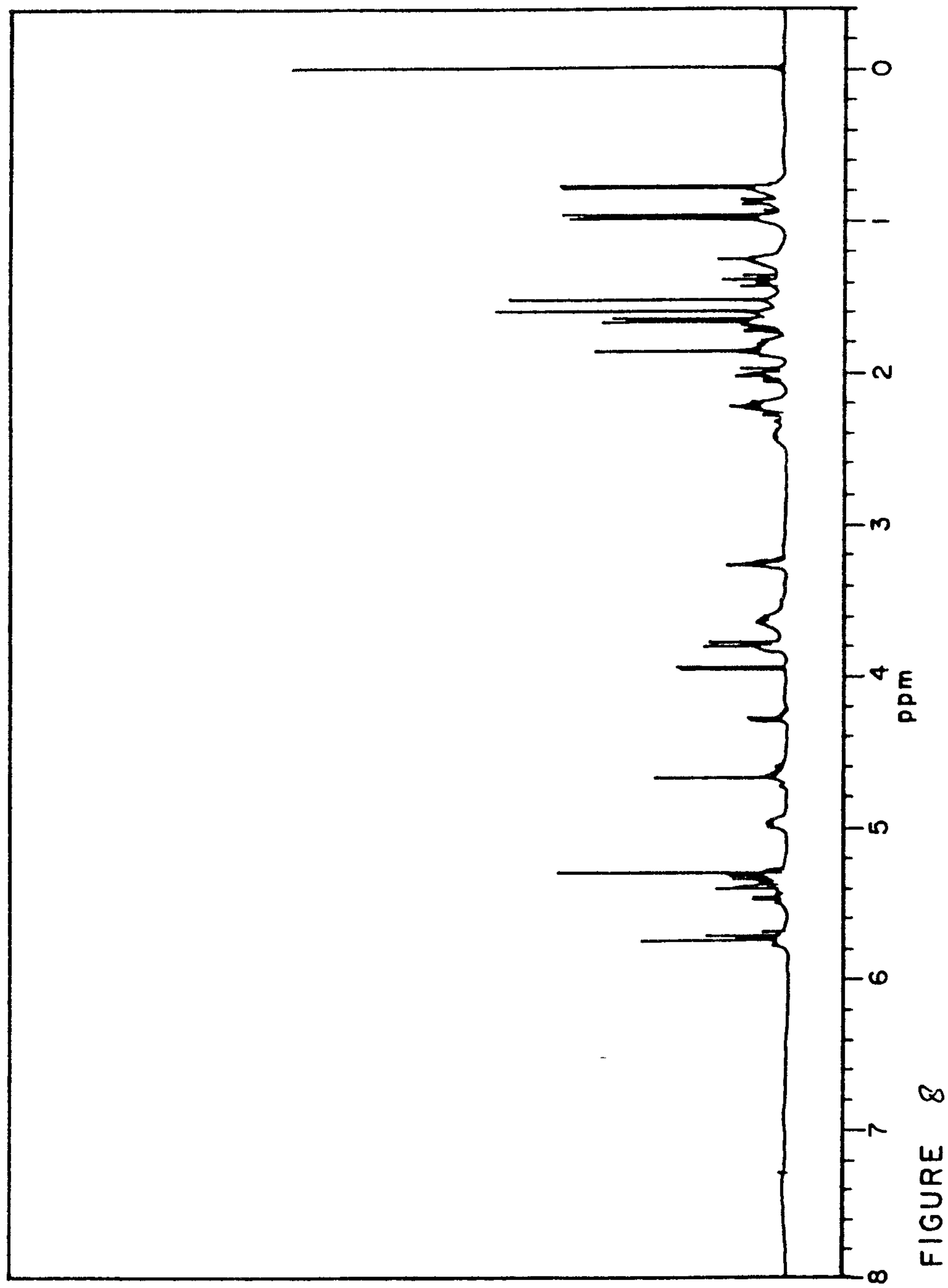


FIGURE 8

ELECTRON IMPACT MASS SPECTRUM OF LL-F28249β

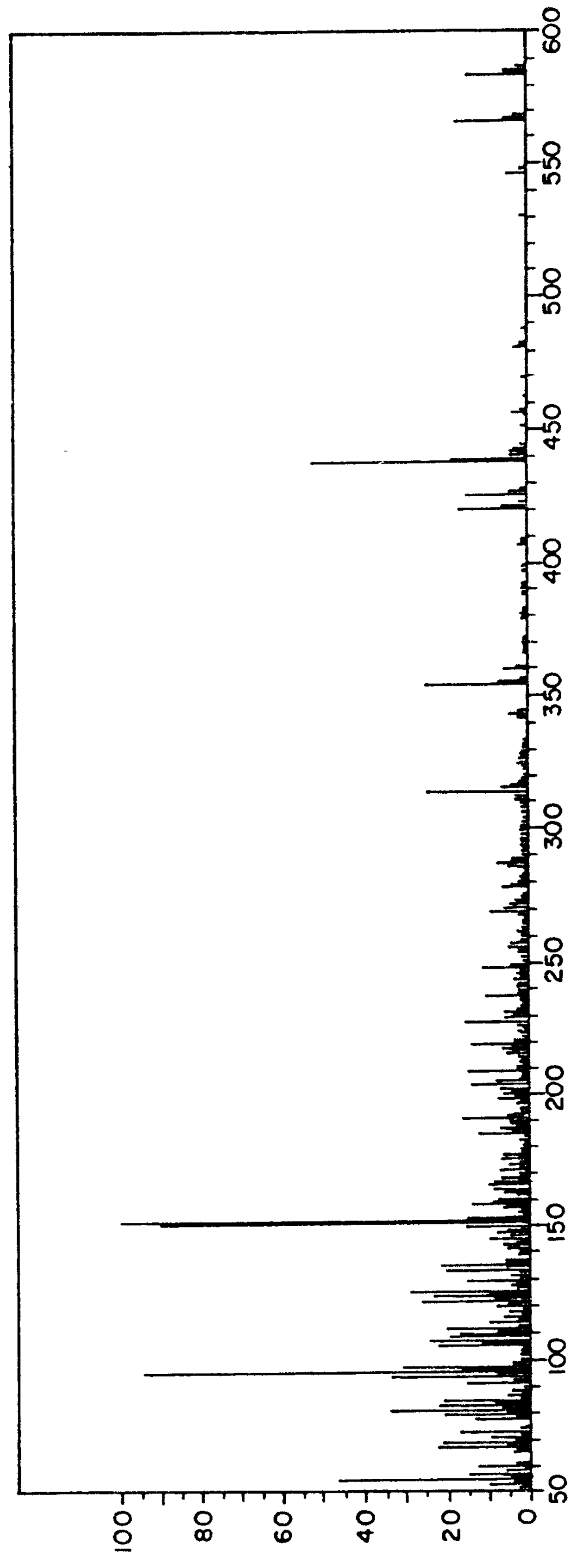


FIGURE 9

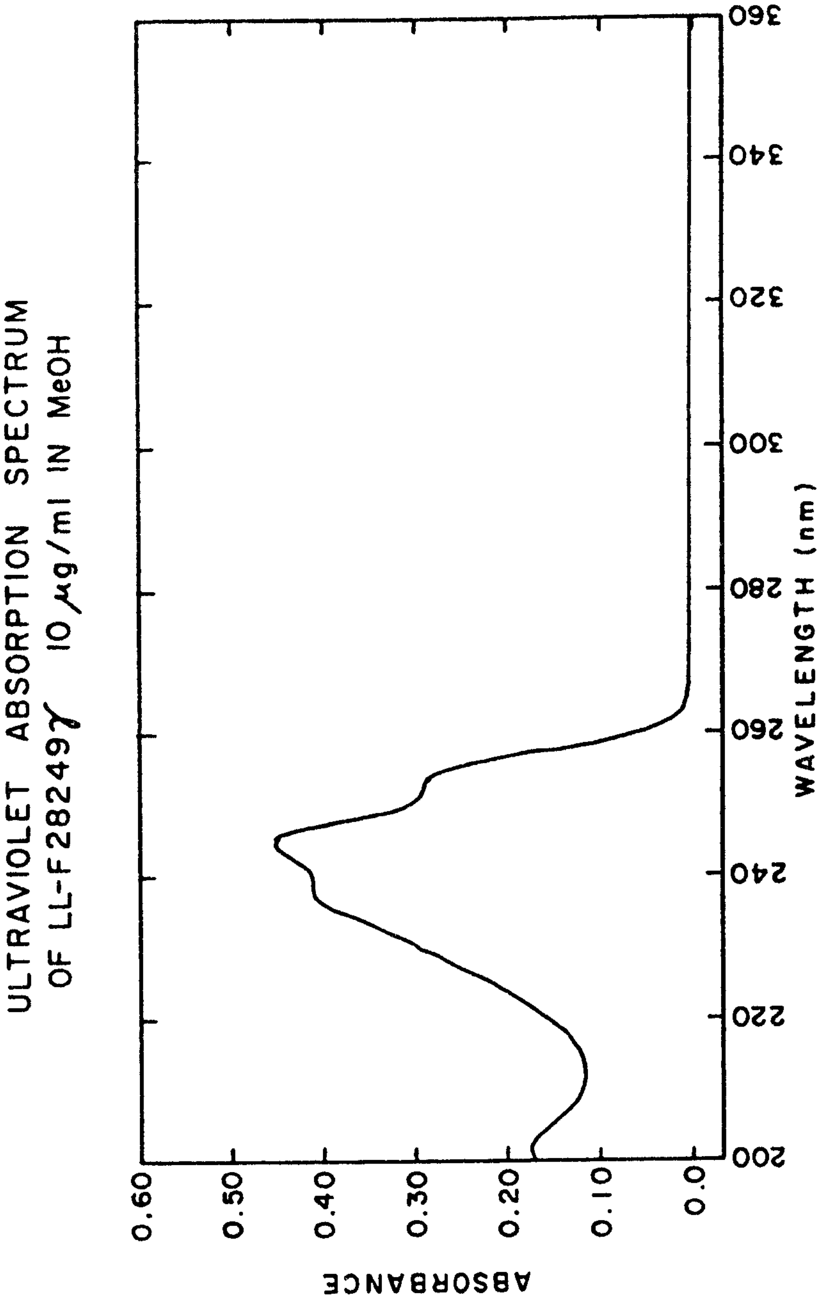
ULTRAVIOLET ABSORPTION SPECTRUM
OF LL-F28249γ 10 μg/ml IN MeOH
ABSORBANCE
0.60
0.50
0.40
0.30
0.20
0.10
0.0
200
220
240
260
280
300
320
340
360
WAVELENGTH (nm)

FIGURE 10

INFRARED ABSORPTION SPECTRUM OF LL-F28249γ (KBr disc)

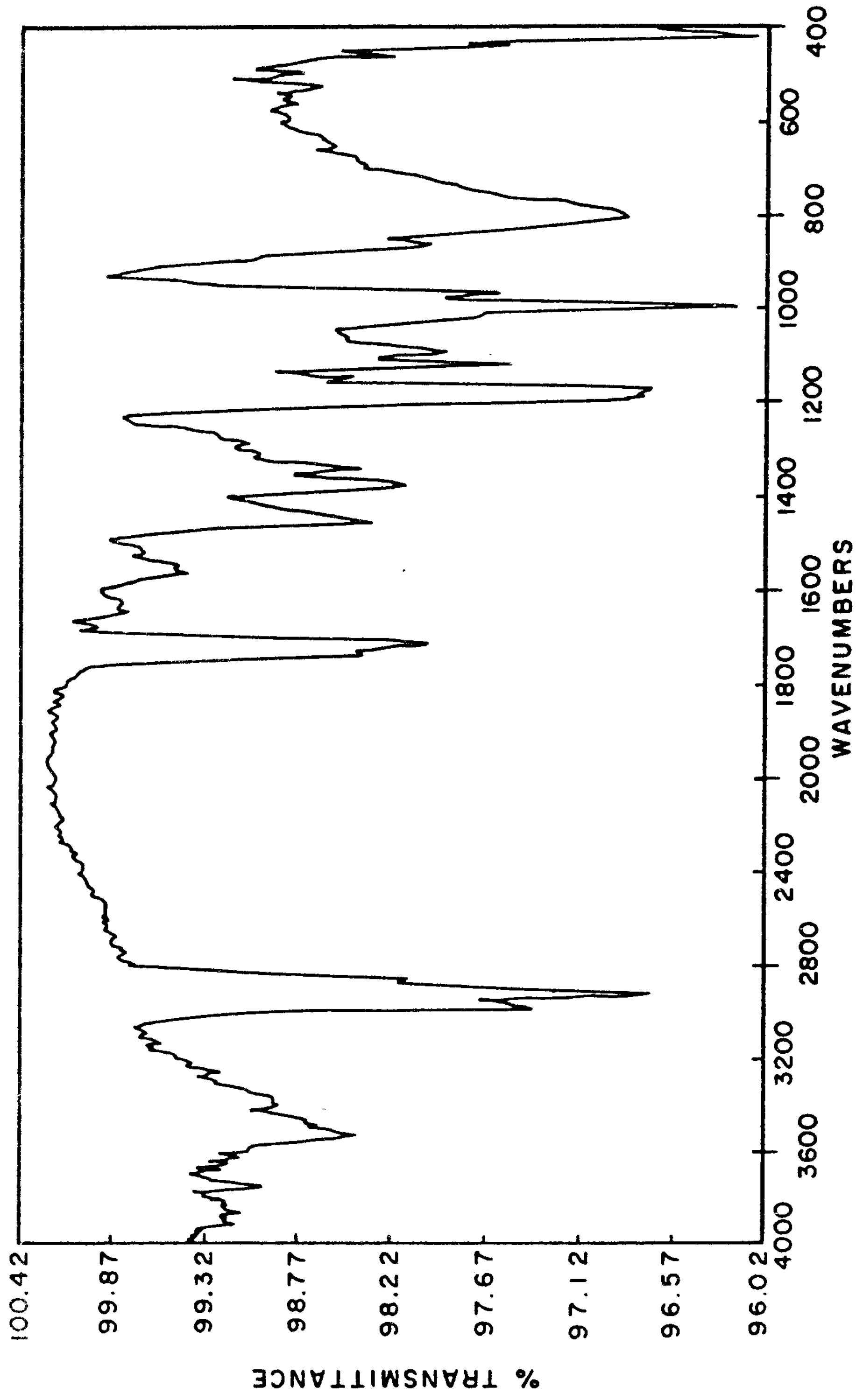


FIGURE 11

PROTON NUCLEAR MAGNETIC RESONANCE SPECTRUM
OF LL-F28249γ IN $CDCl_3$ SOLUTION

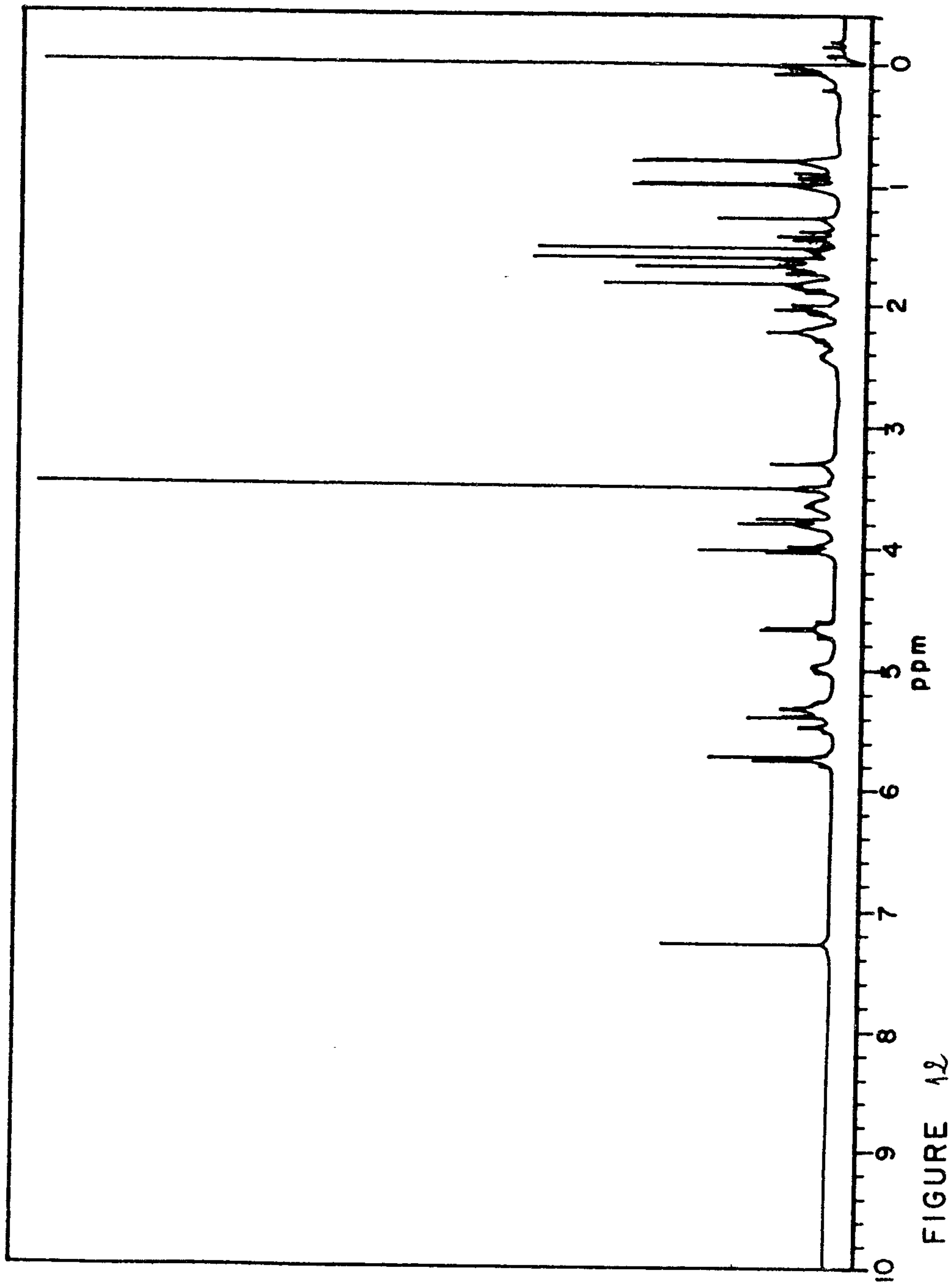


FIGURE 12

CARBON-13 NUCLEAR MAGNETIC RESONANCE SPECTRUM
OF LL-F28249γ IN $CDCl_3$ SOLUTION

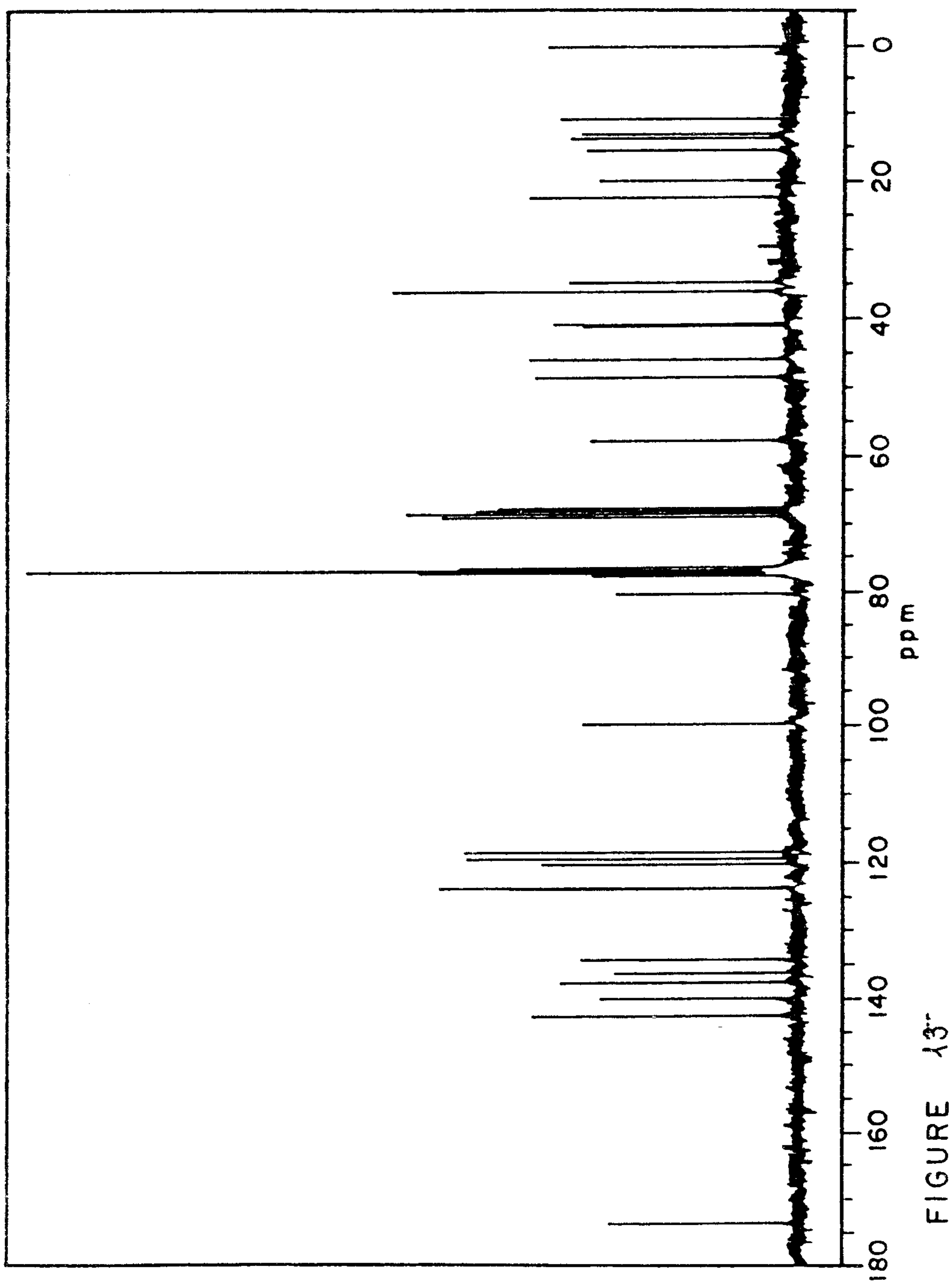


FIGURE 13

ELECTRON IMPACT MASS SPECTRUM OF LL-F28249γ

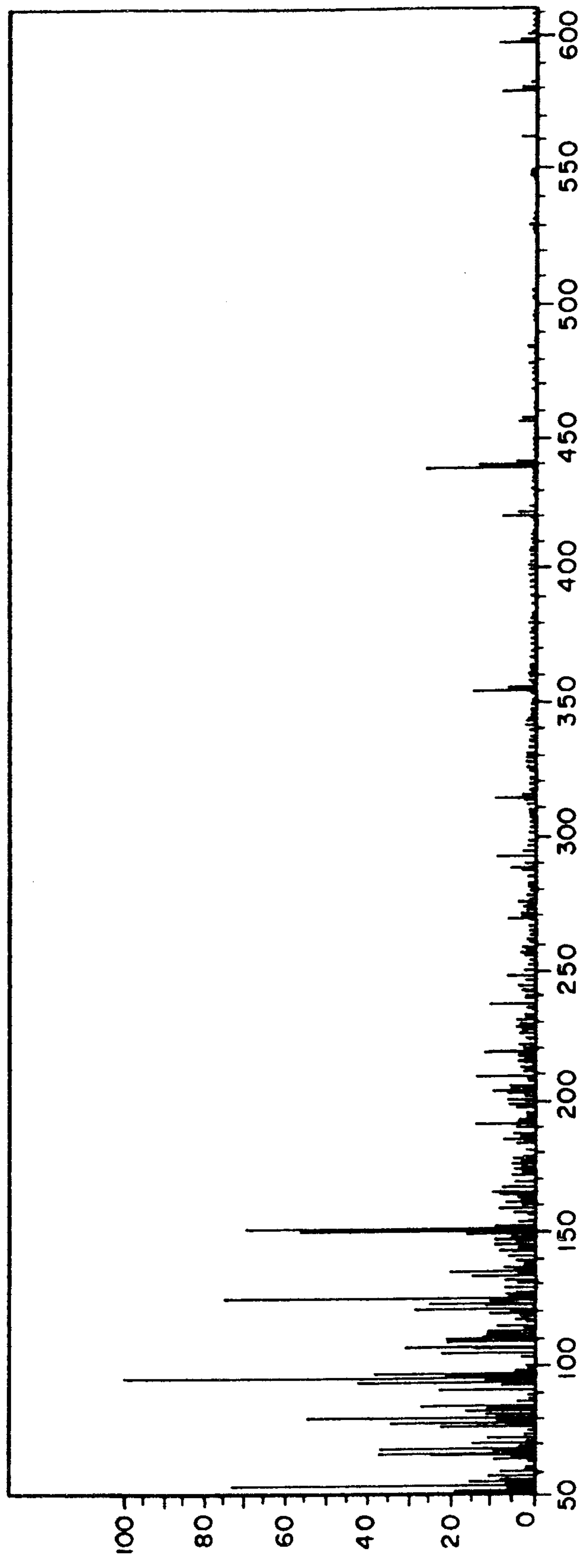


FIGURE 14

ULTRAVIOLET ABSORPTION SPECTRUM OF LL-28249ω

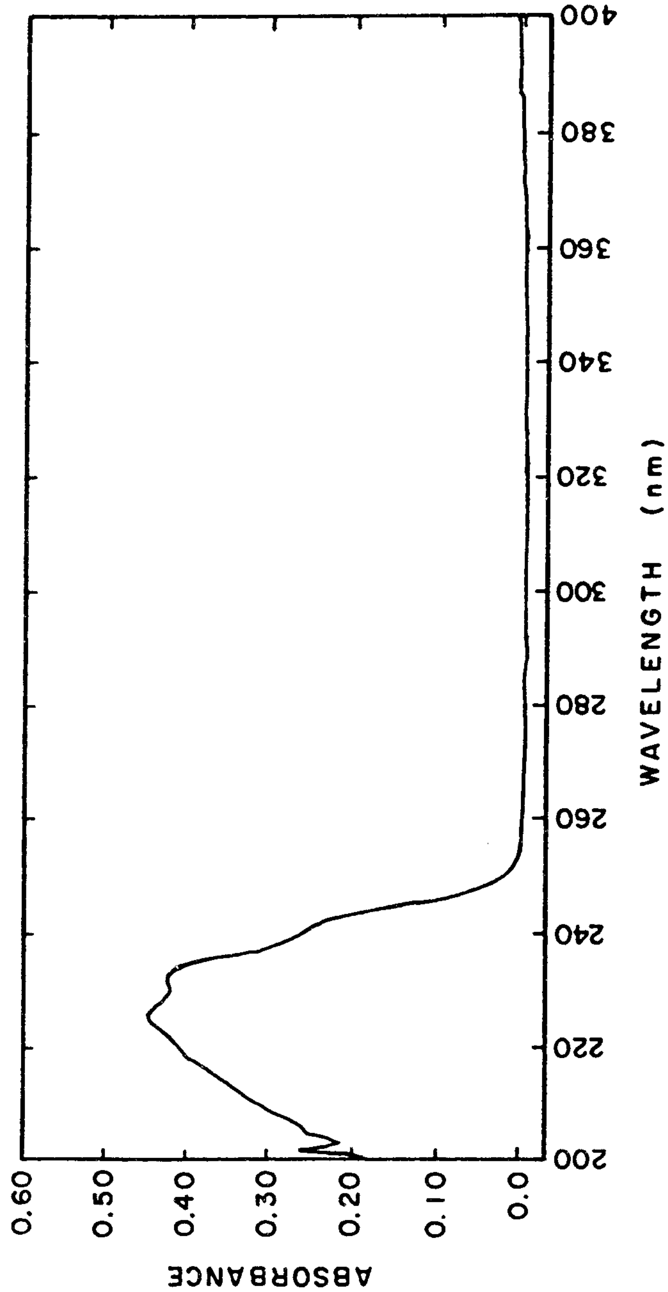


FIGURE 15

INFRARED ABSORPTION SPECTRUM
OF LL-F28249ω (KBr disc)

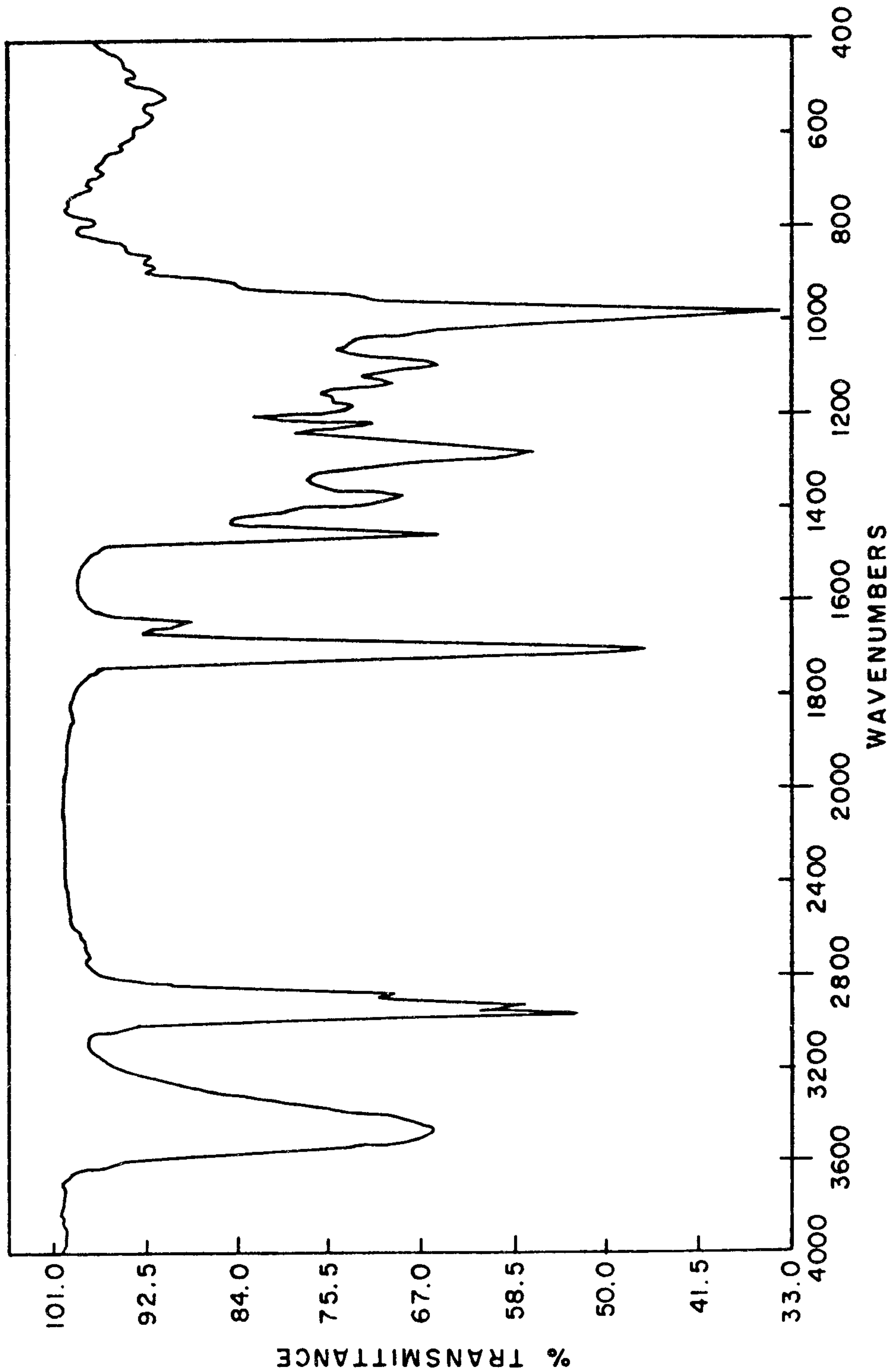


FIGURE 16

PROTON NUCLEAR MAGNETIC RESONANCE SPECTRUM
OF LL-F28249ω IN $CDCl_3$ SOLUTION

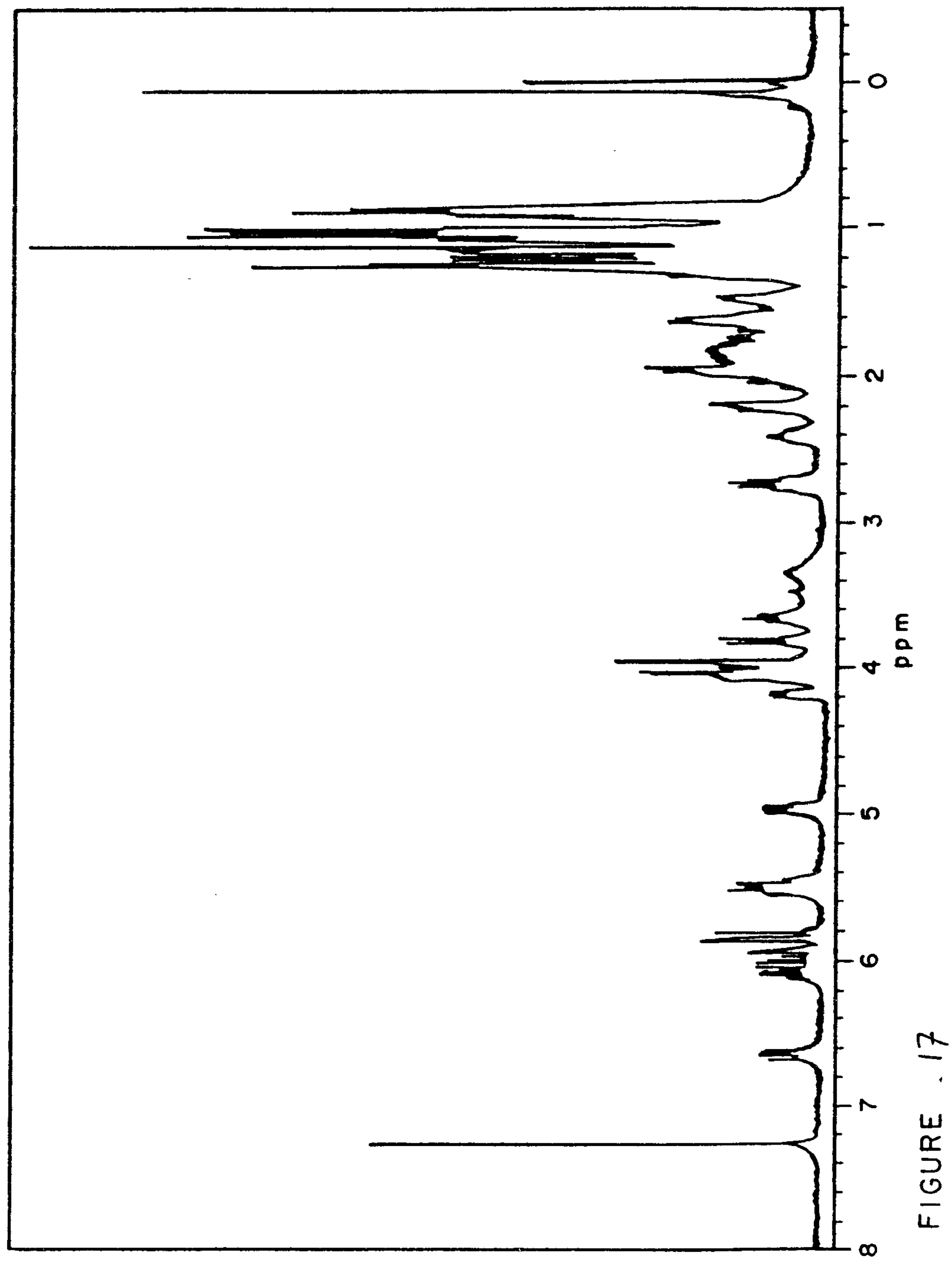


FIGURE . 17

CARBON-13 NUCLEAR MAGNETIC RESONANCE SPECTRUM OF LL-F28249ω IN $CDCl_3$ SOLUTION

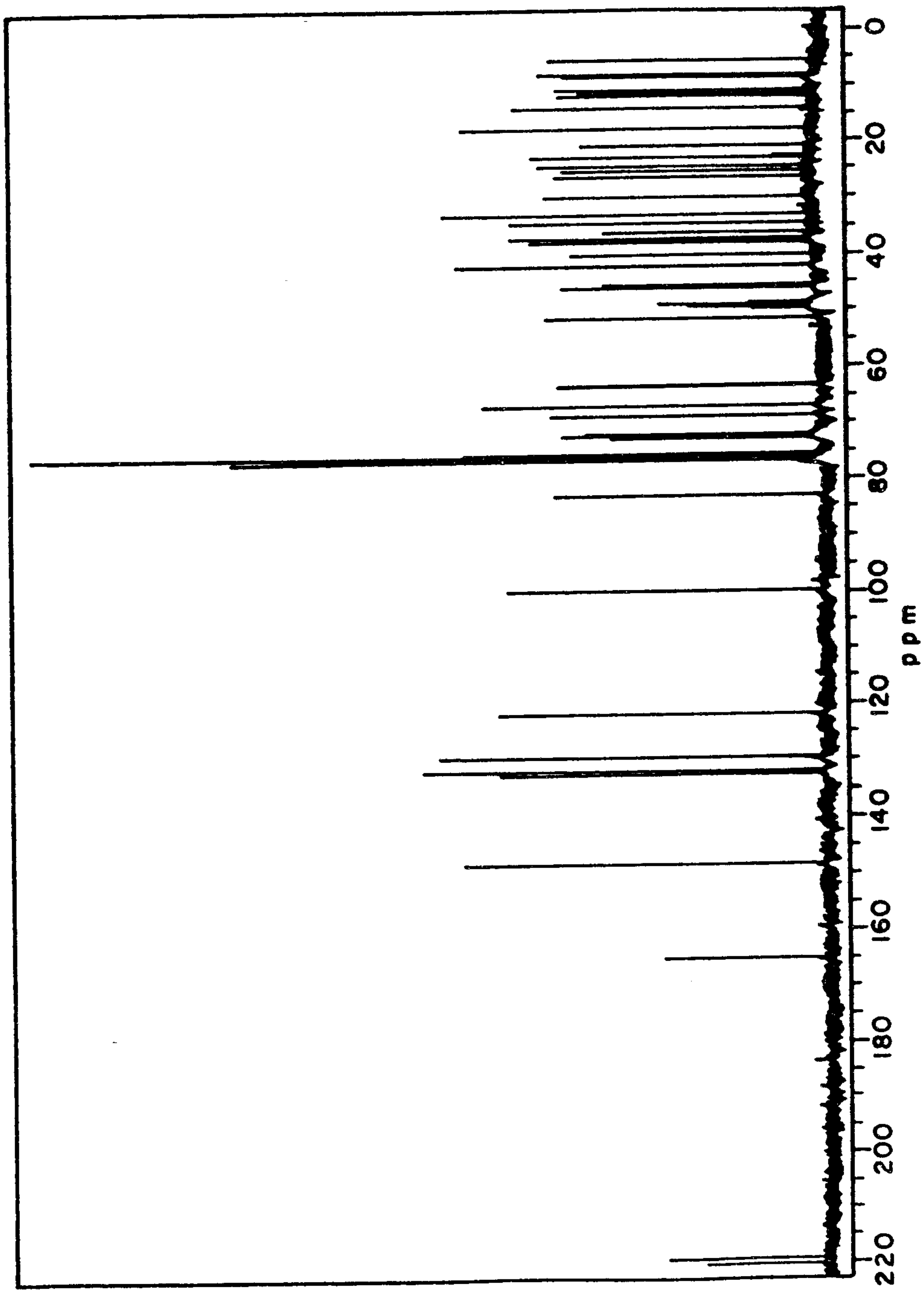


FIGURE 18

ELECTRON IMPACT MASS SPECTRUM
OF LL-F28249ω

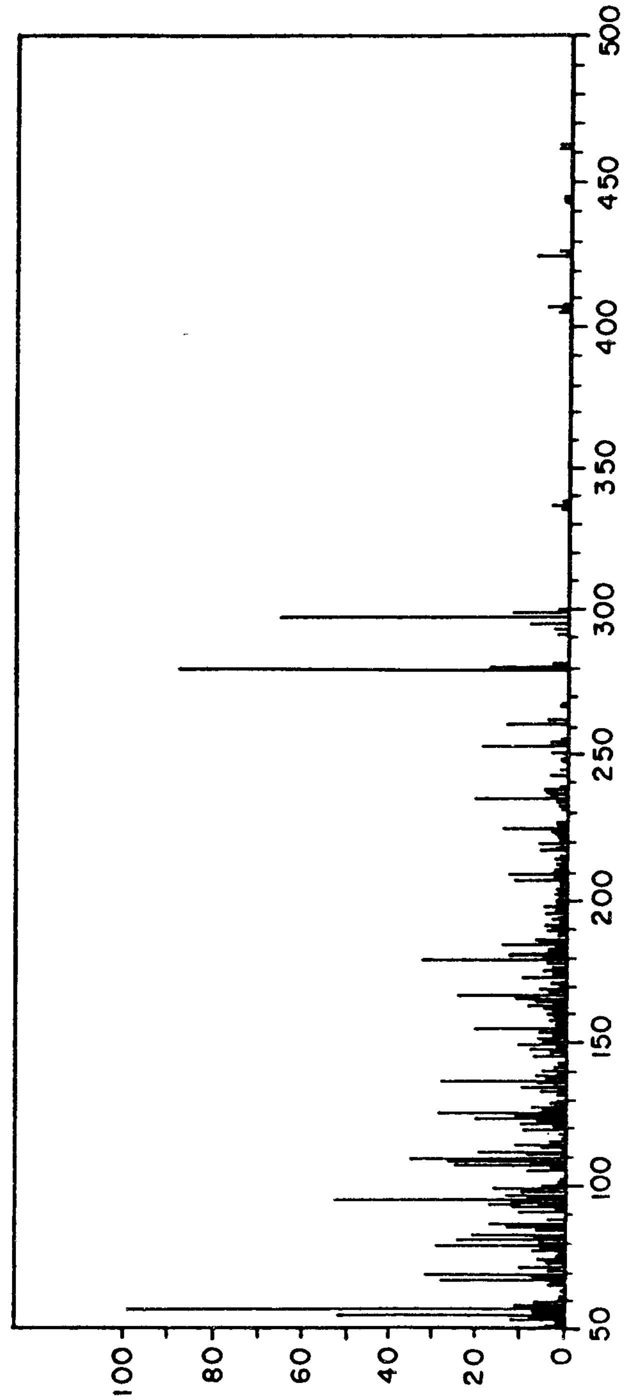


FIGURE 19

ULTRAVIOLET ABSORPTION SPECTRUM
OF LL-F28249δ

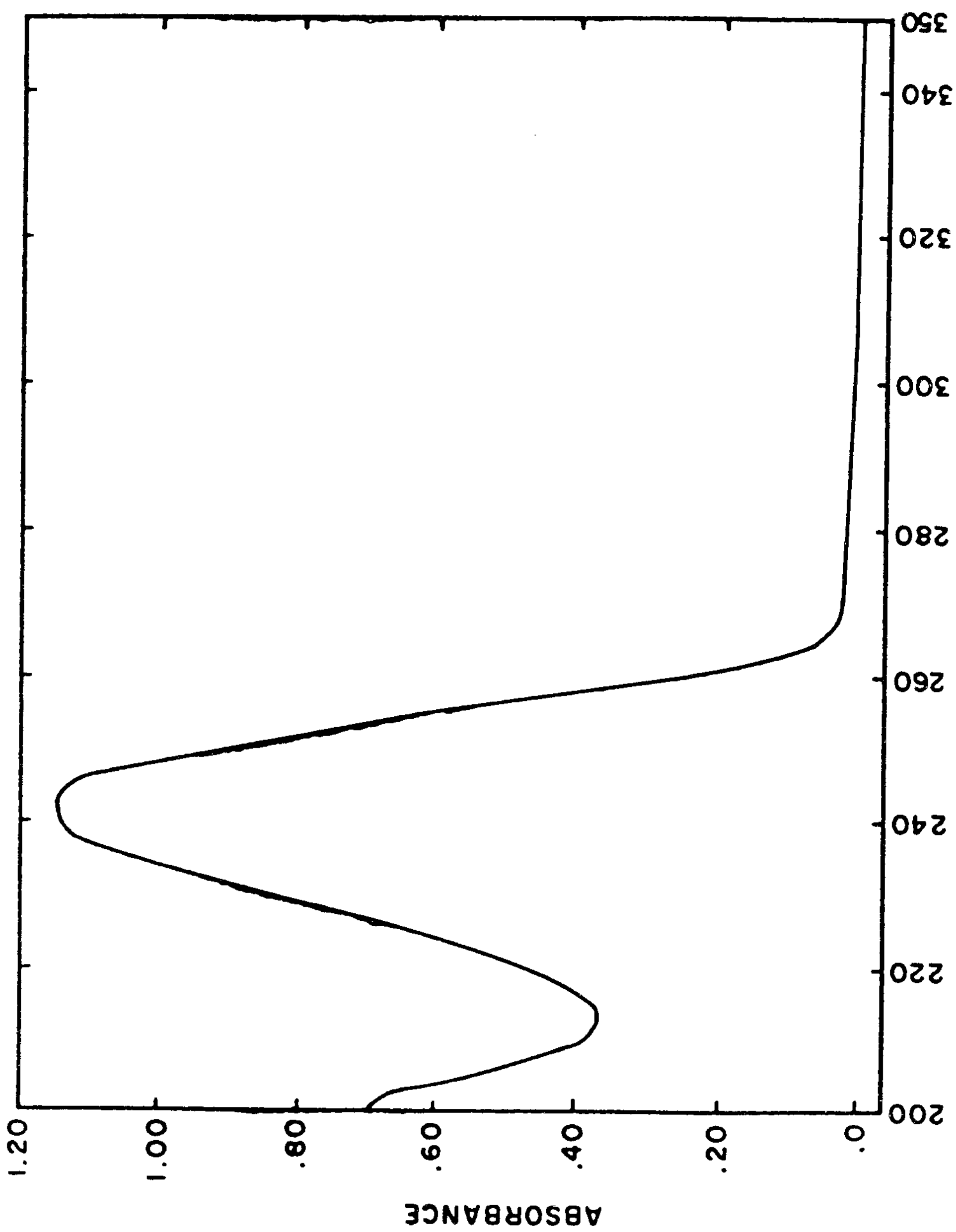


WAVELENGTH (nm)

FIGURE 2D

PROTON NUCLEAR MAGNETIC RESONANCE SPECTRUM
OF LL-F28249δ IN $CDCl_3$ SOLUTION

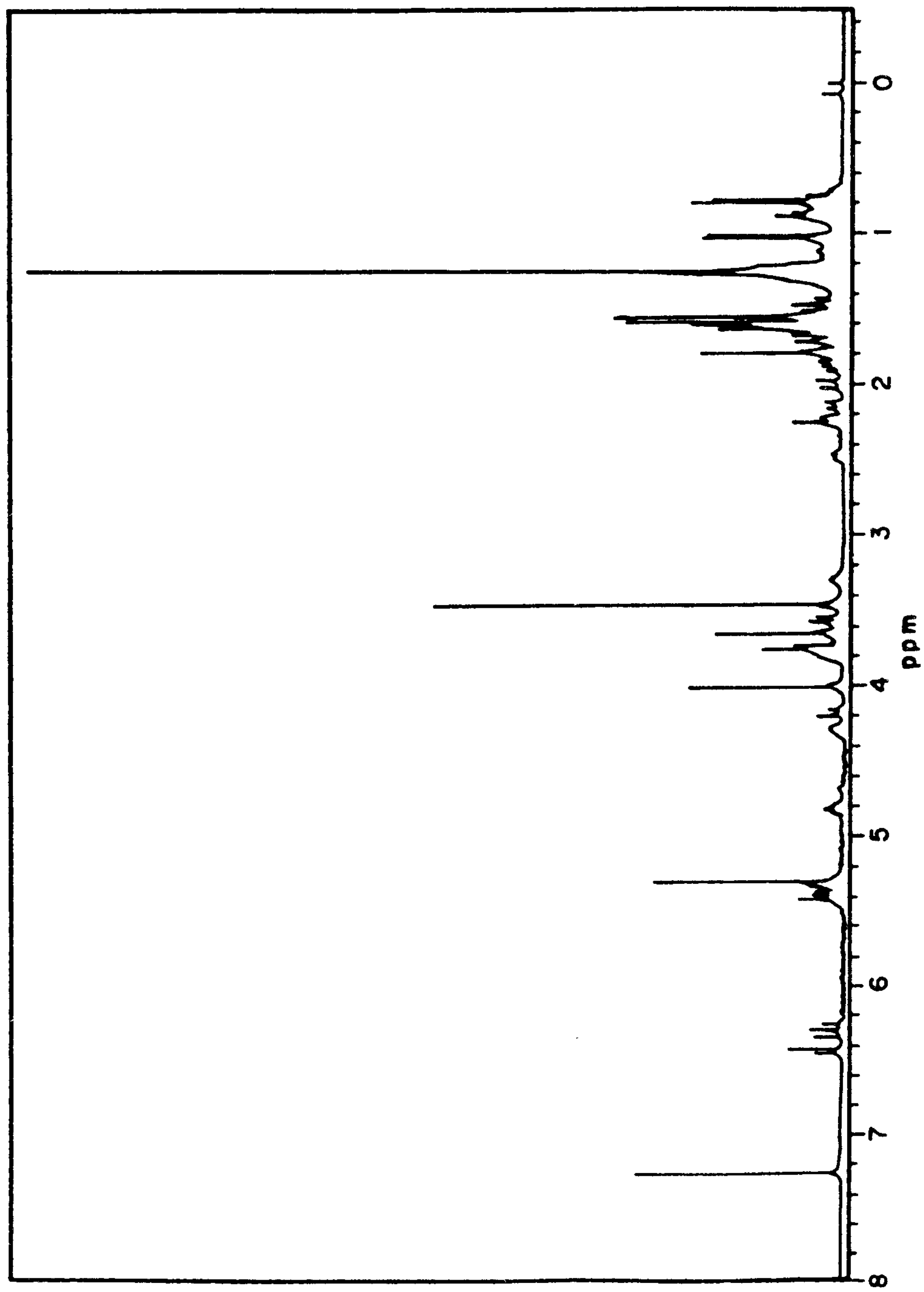


FIGURE 21

ELECTRON IMPACT MASS SPECTRUM OF LL-F28249δ

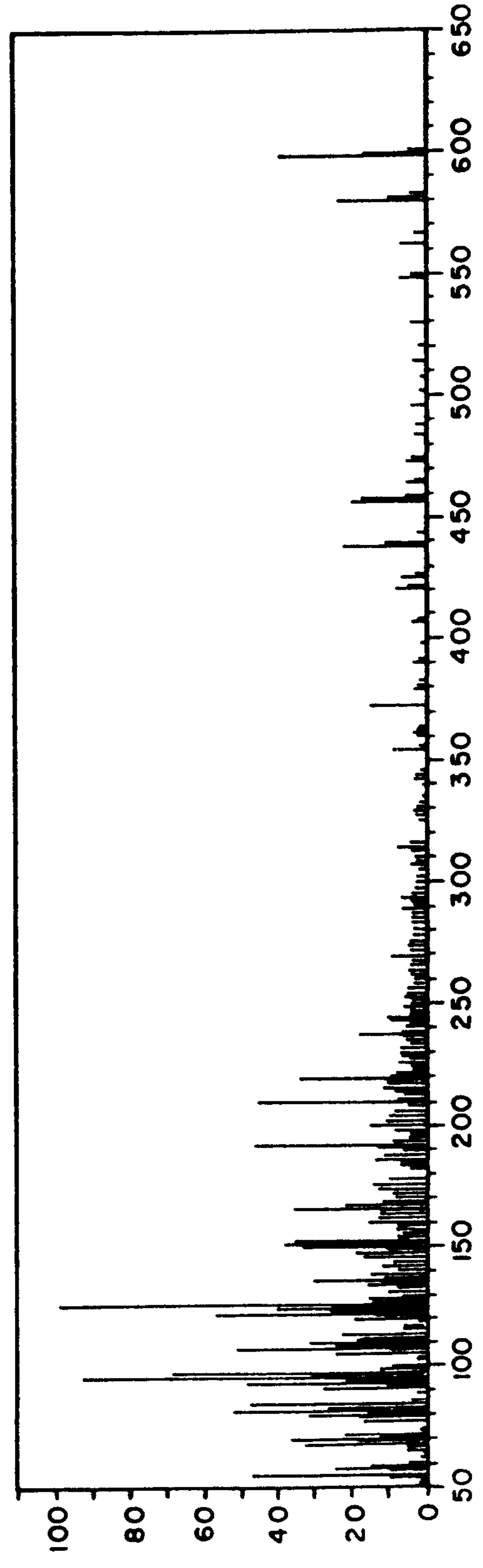


FIGURE 12

ULTRAVIOLET ABSORPTION SPECTRUM
OF LL-F28249ε

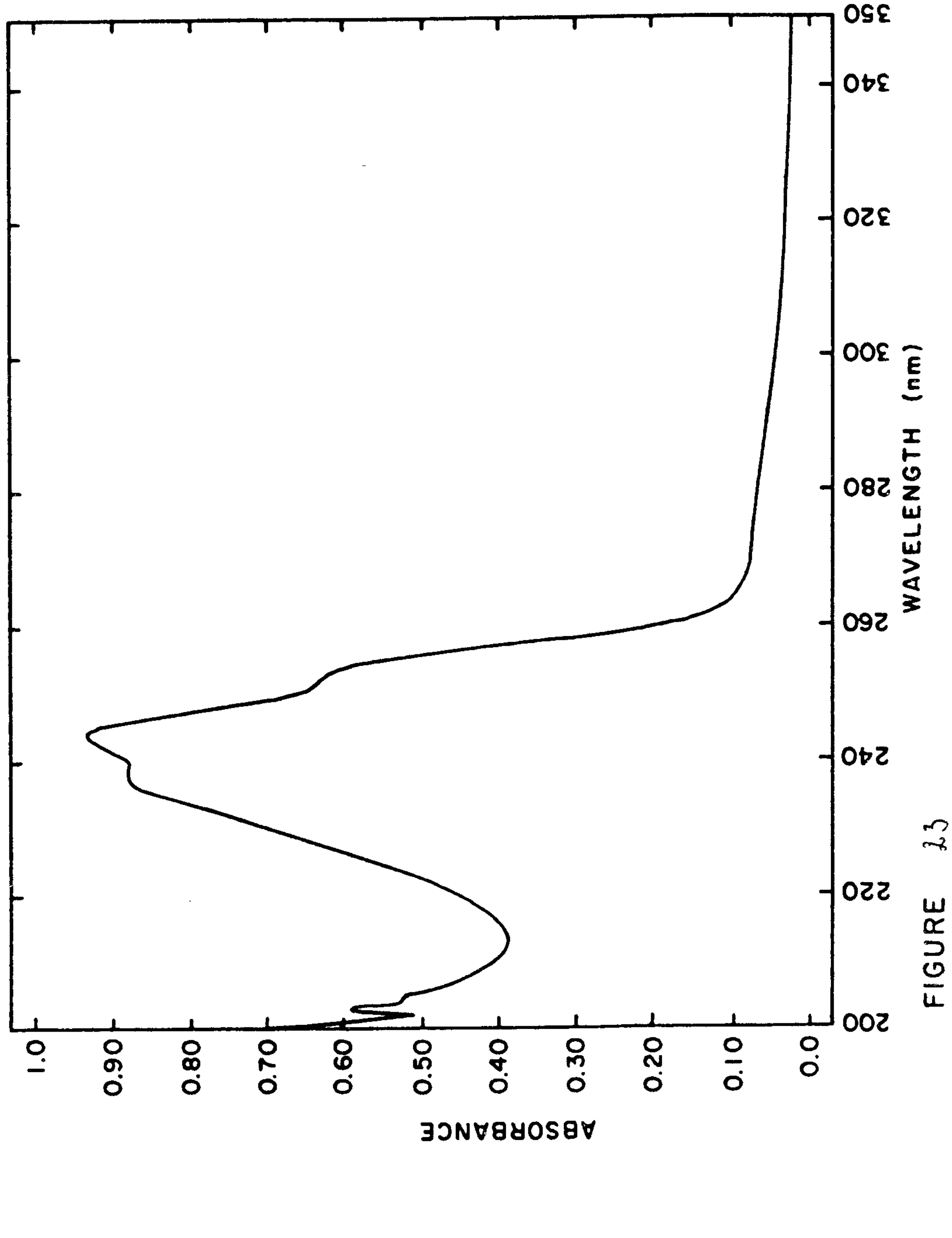


FIGURE 23

PROTON NUCLEAR MAGNETIC RESONANCE SPECTRUM
OF LL-F28249ε IN $CDCl_3$ SOLUTION

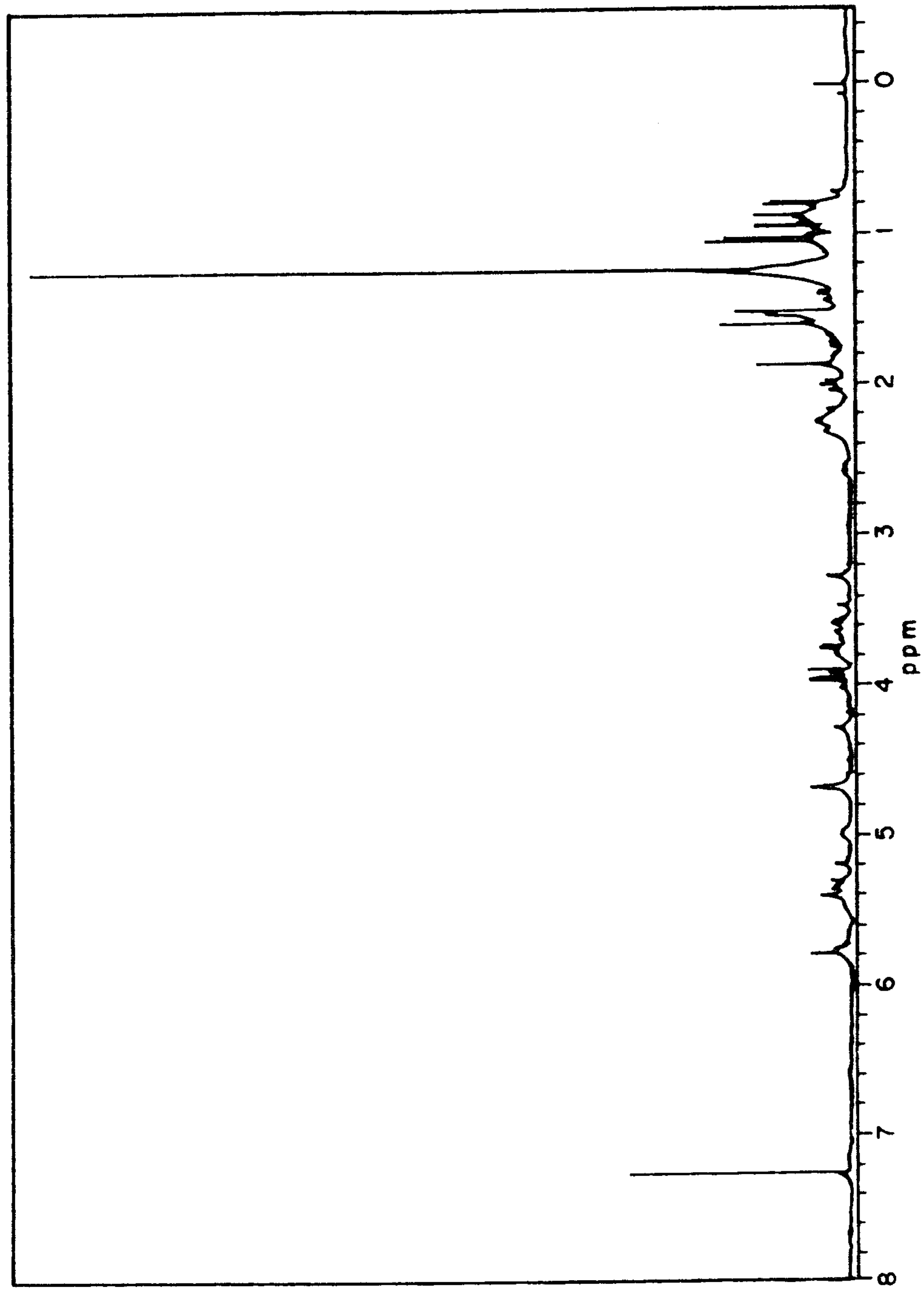


FIGURE 24

ELECTRON IMPACT MASS SPECTRUM OF LL-F28249ε

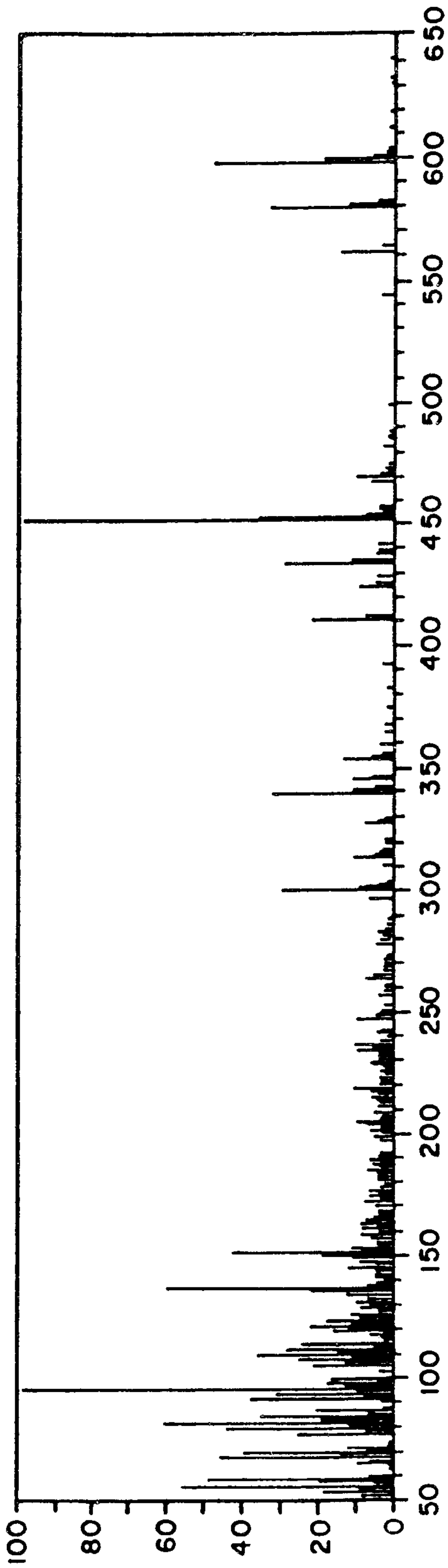


FIGURE 25

ULTRAVIOLET ABSORPTION SPECTRUM OF LL-F28249ζ

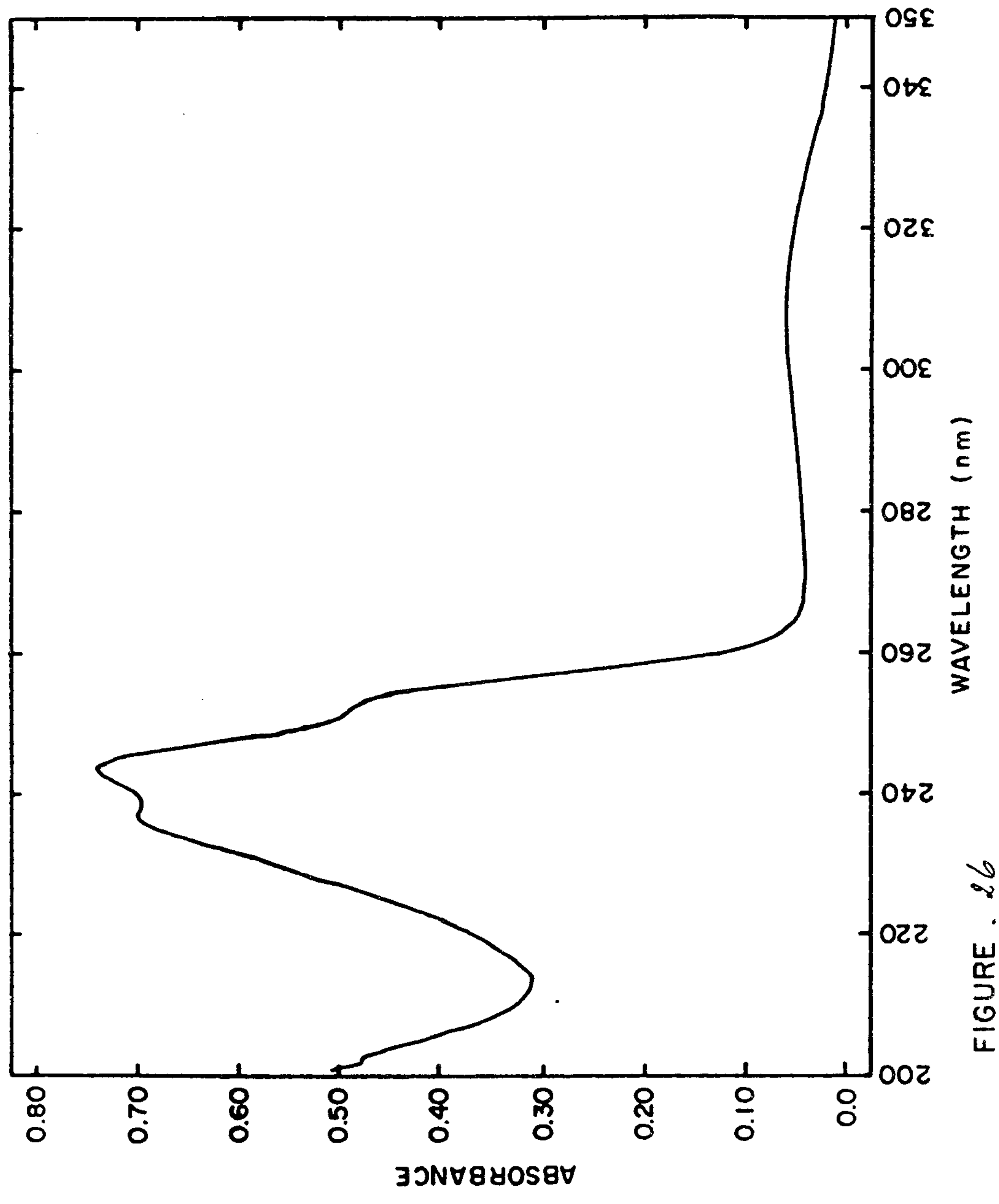


FIGURE . 26

PROTON NUCLEAR MAGNETIC RESONANCE SPECTRUM OF LL-F28249ζ IN $CDCl_3$ SOLUTION

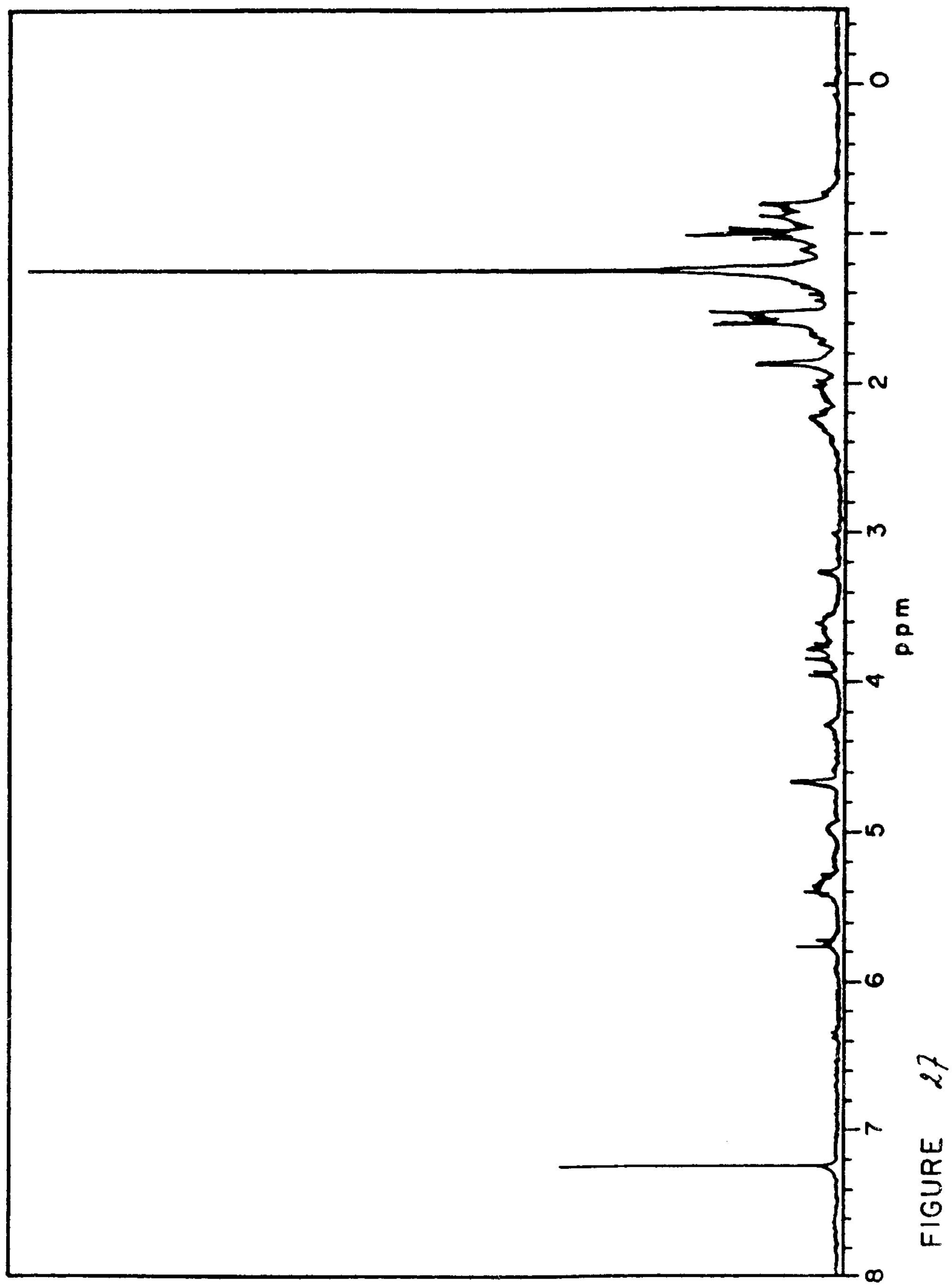


FIGURE 27

ELECTRON IMPACT MASS SPECTRUM OF LL-F28249ε

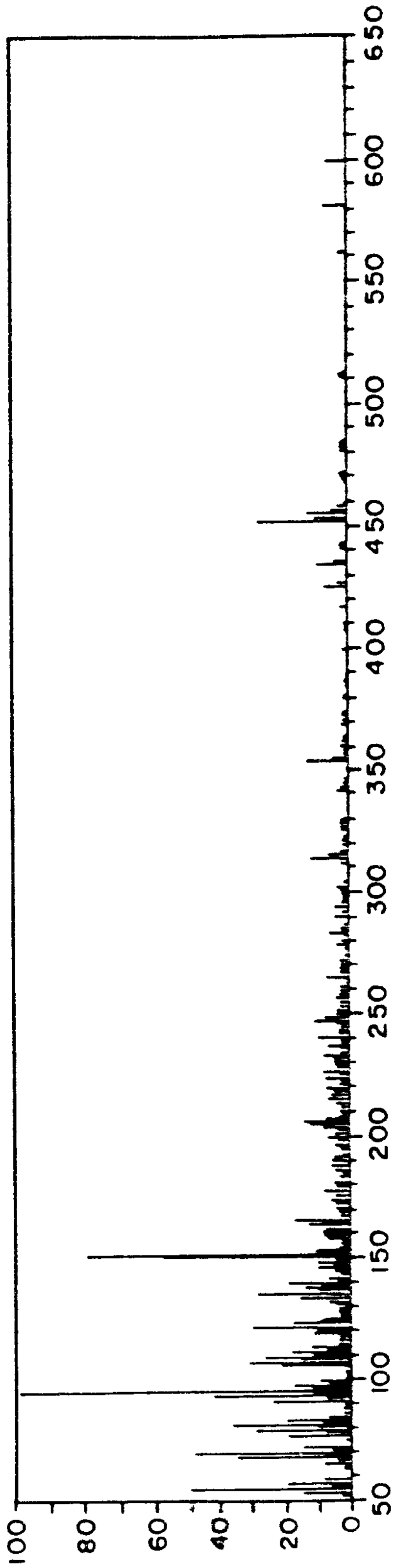

FIGURE 28

ULTRAVIOLET ABSORPTION SPECTRUM OF LL-F28249η

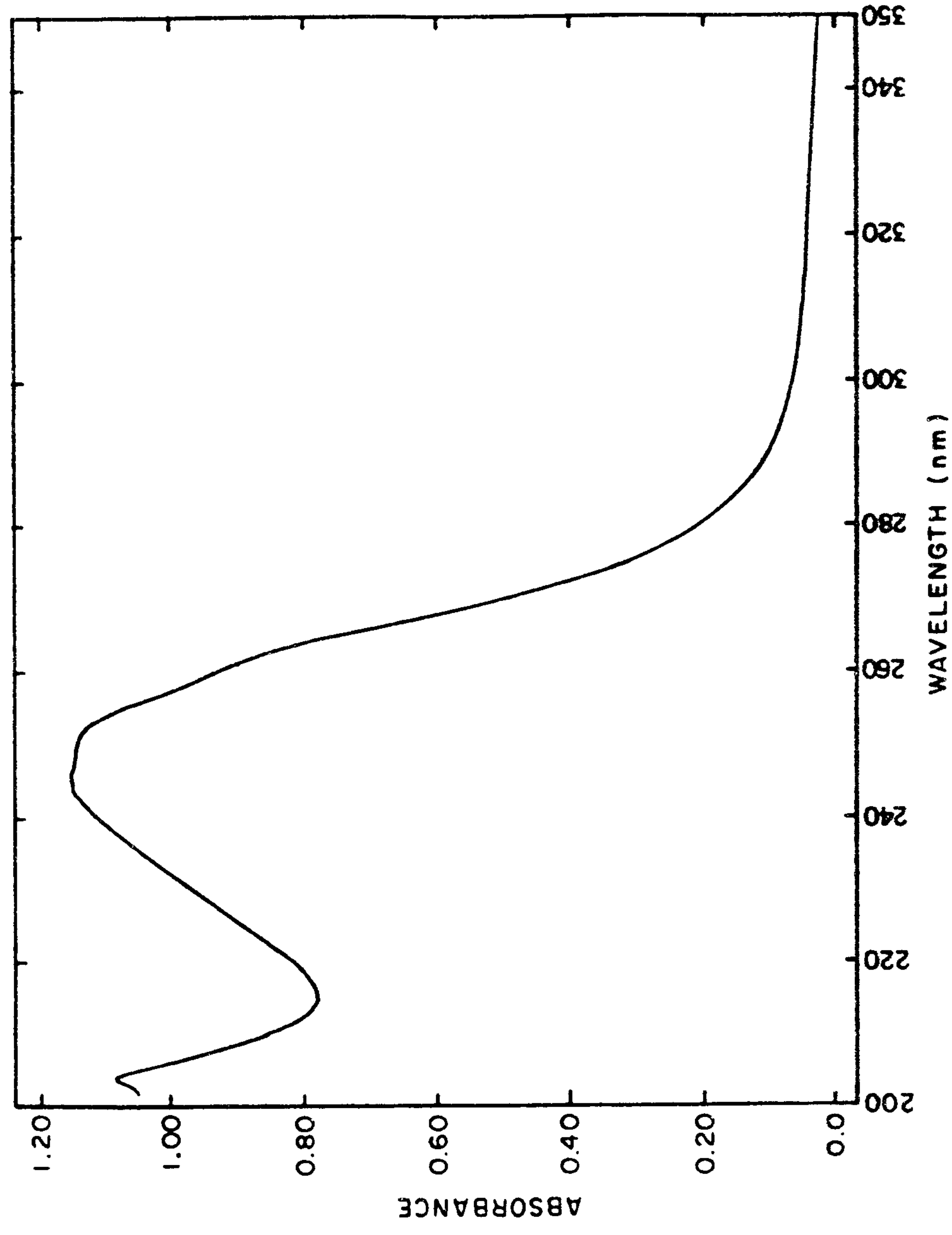


FIGURE . 19

PROTON NUCLEAR MAGNETIC RESONANCE SPECTRUM OF
LL-F28249η IN $CDCl_3$ SOLUTION

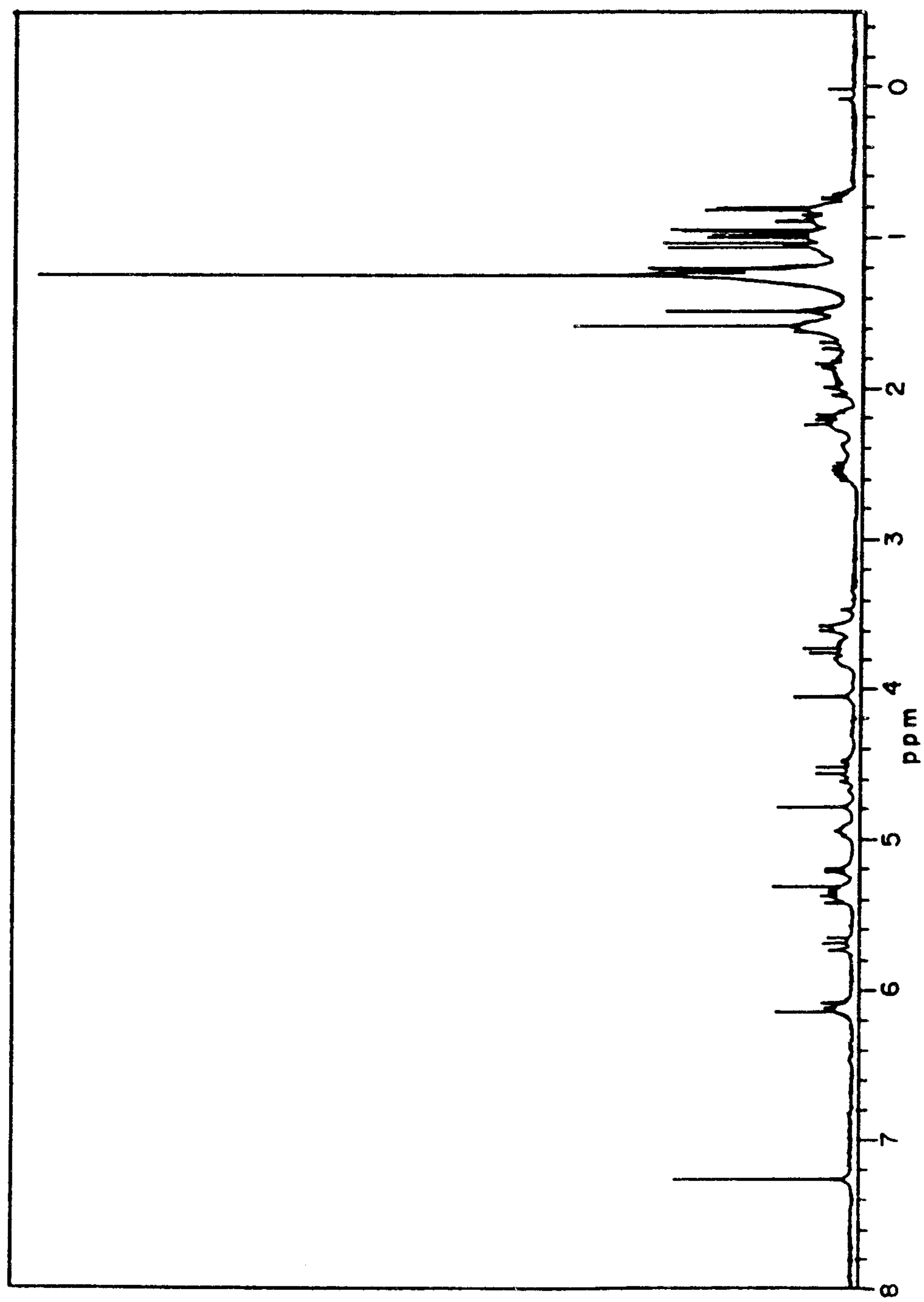


FIGURE 30

ELECTRON IMPACT MASS SPECTRUM OF LL-F28249η

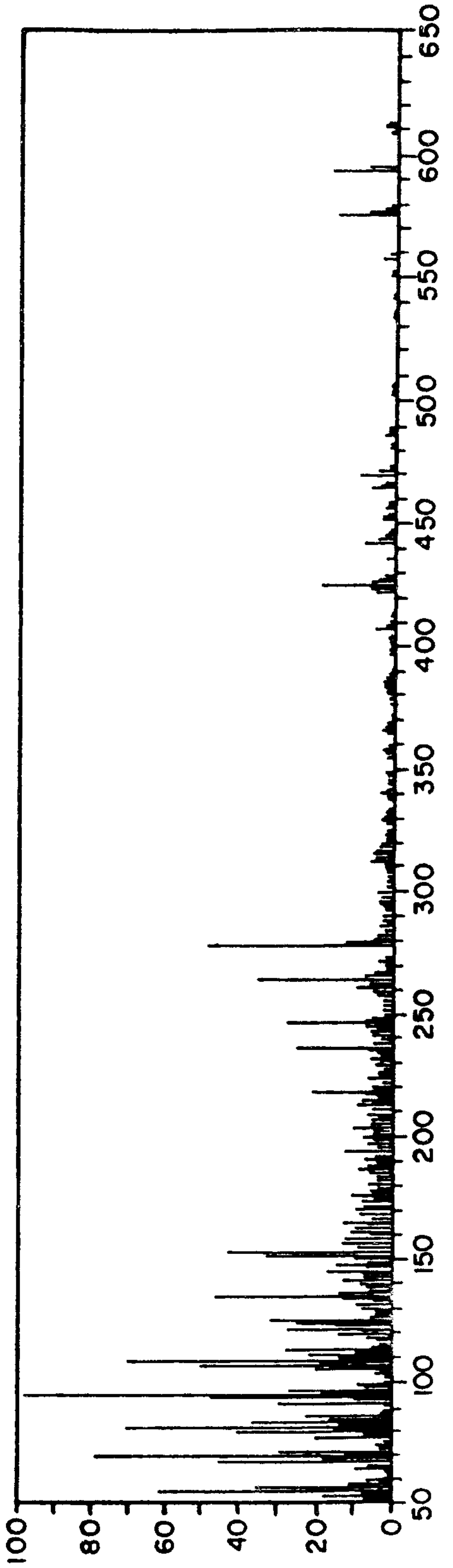


FIGURE 31

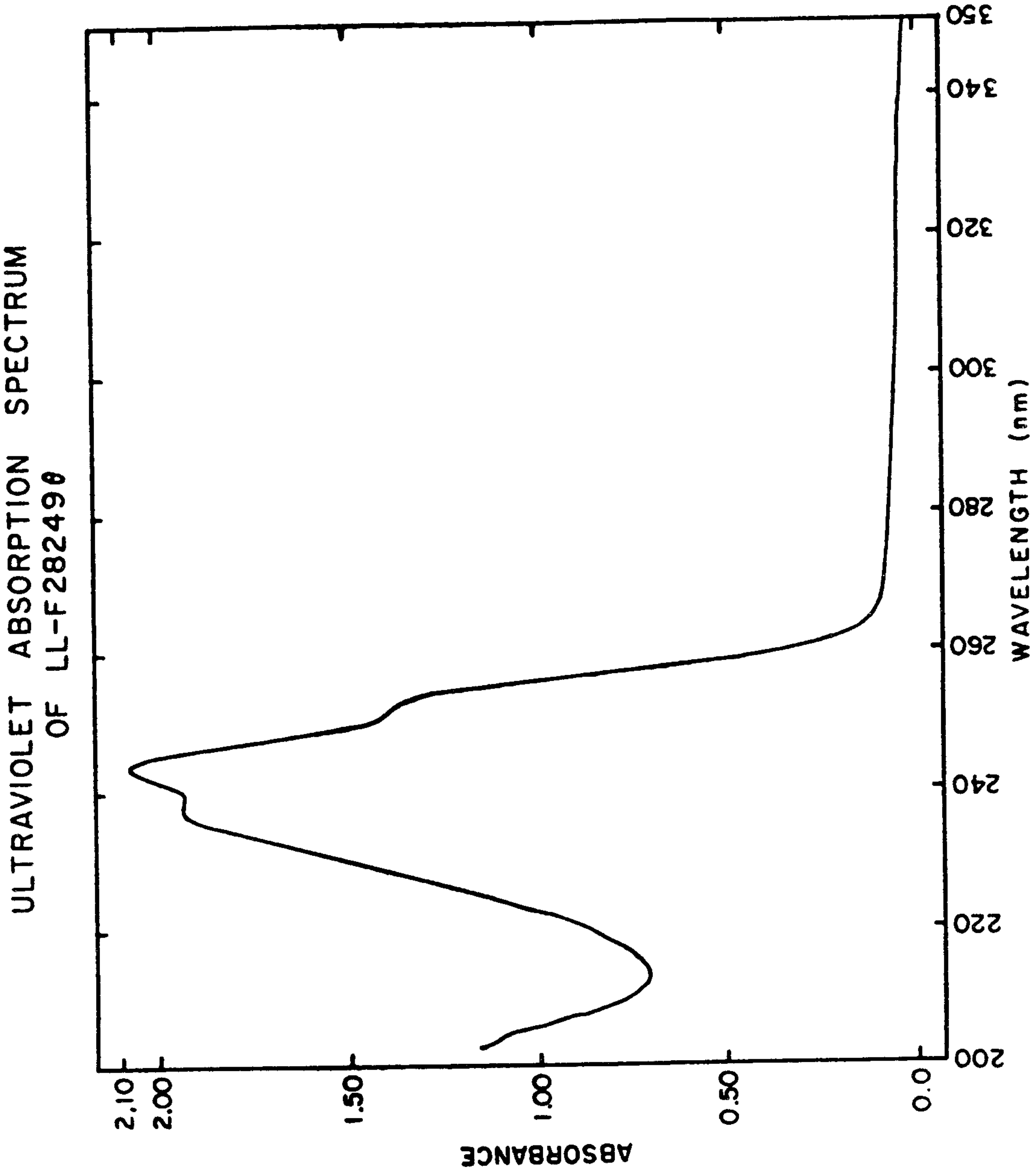
ULTRAVIOLET ABSORPTION SPECTRUM
OF LL-F28249θ
ABSORBANCE
2.10
2.00
1.50
1.00
0.50
0.0
200
220
240
260
280
300
320
340
350
WAVELENGTH (nm)

FIGURE 32

PROTON NUCLEAR MAGNETIC RESONANCE SPECTRUM OF LL-F28249θ IN $CDCl_3$ SOLUTION

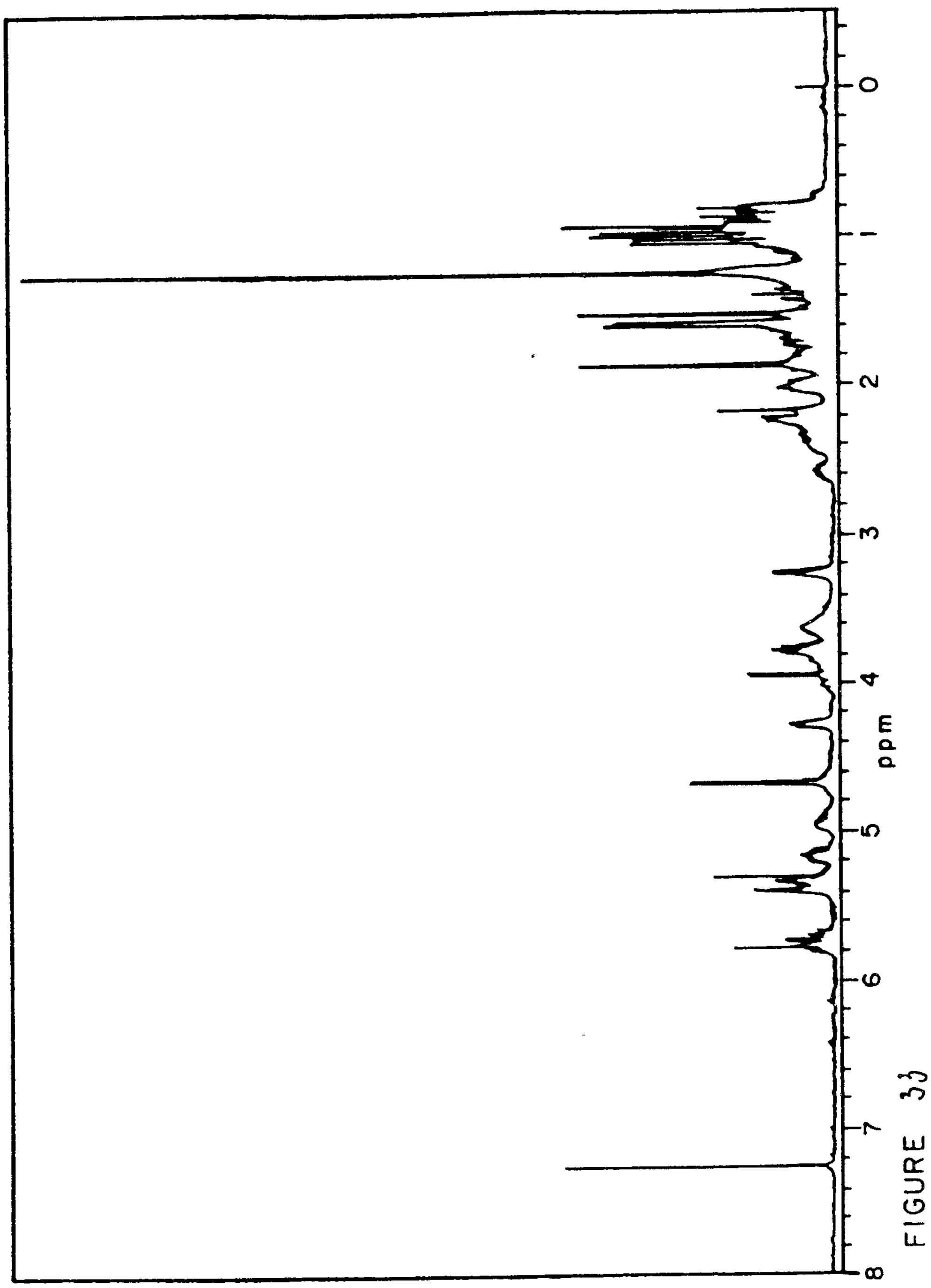


FIGURE 33

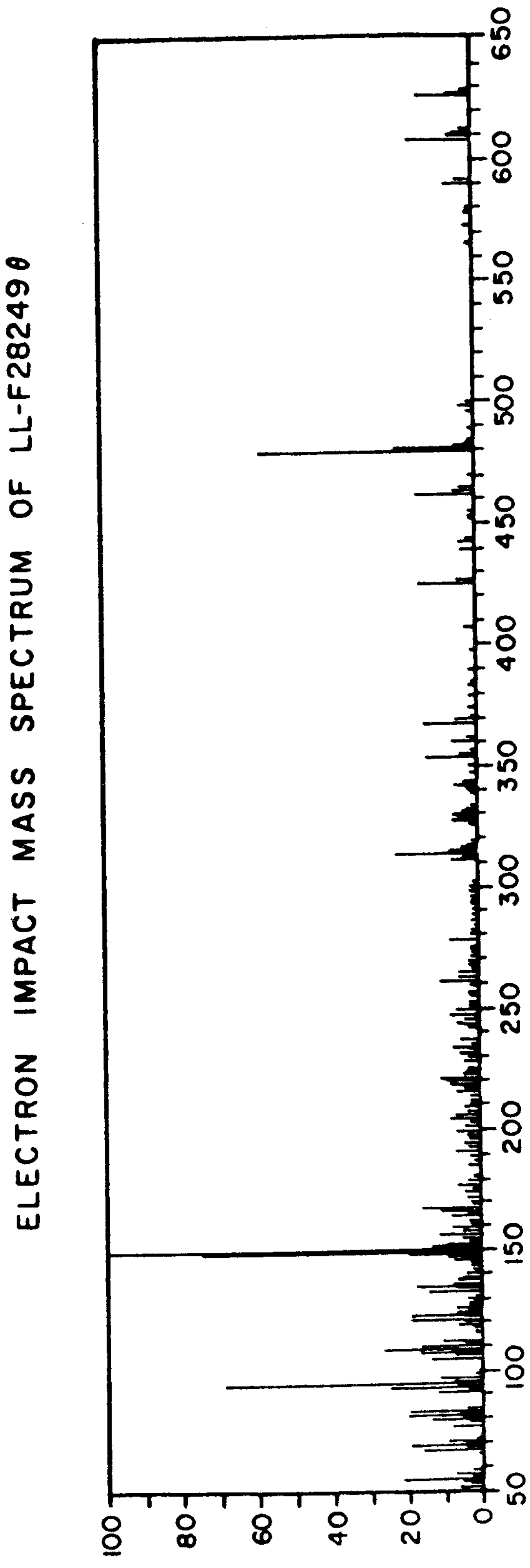
ELECTRON IMPACT MASS SPECTRUM OF LL-F28249θ
100
80
60
40
20
0
50
100
150
200
250
300
350
400
450
500
550
600
650

FIGURE 34

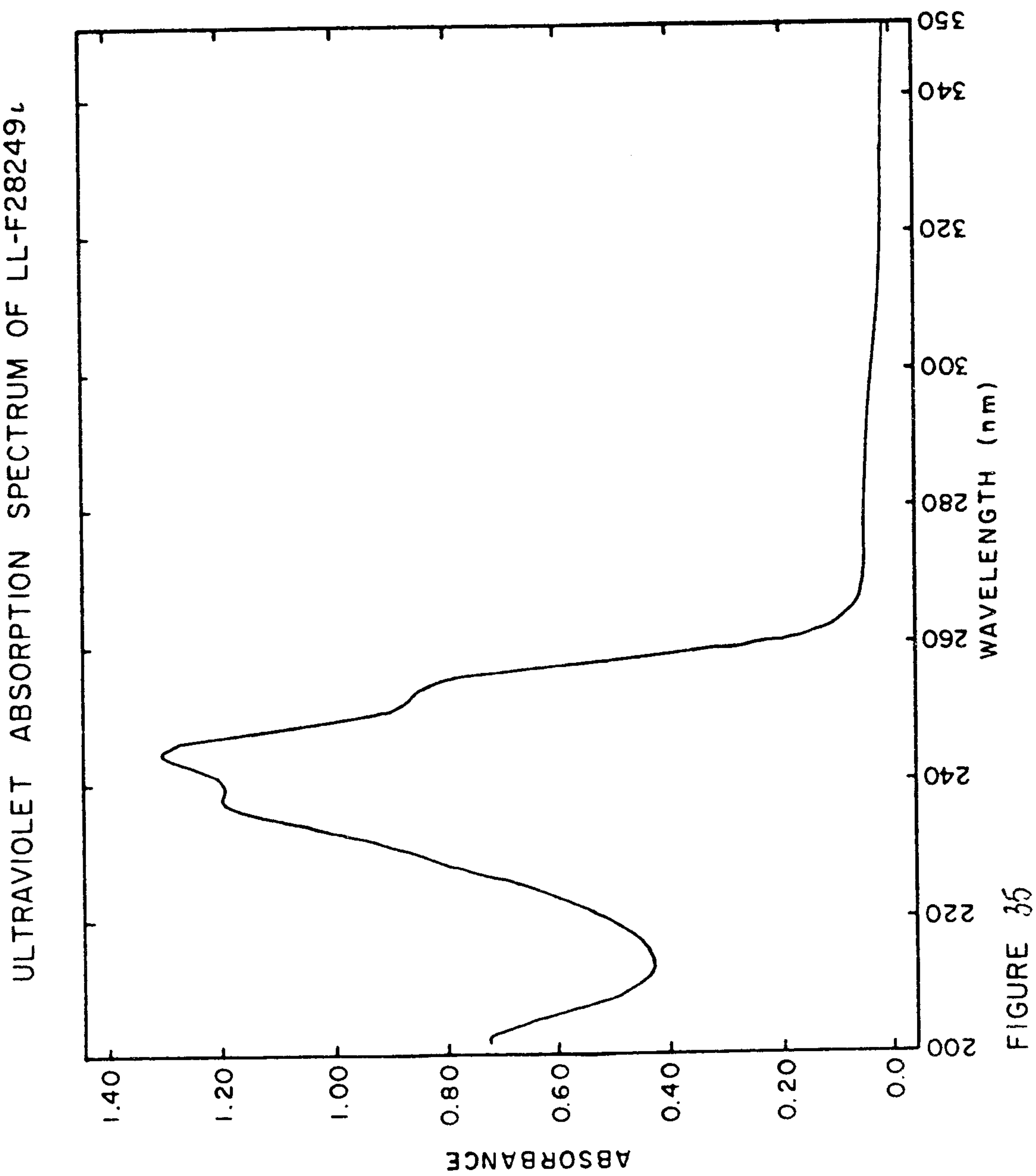
ULTRAVIOLET ABSORPTION SPECTRUM OF LL-F28249ι
ABSORBANCE
1.40
1.20
1.00
0.80
0.60
0.40
0.20
0.0
200
220
240
260
280
300
320
340
350
WAVELENGTH (nm)

FIGURE 35

PROTON NUCLEAR MAGNETIC RESONANCE SPECTRUM
OF LL-F28249ι IN $CDCl_3$ SOLUTION

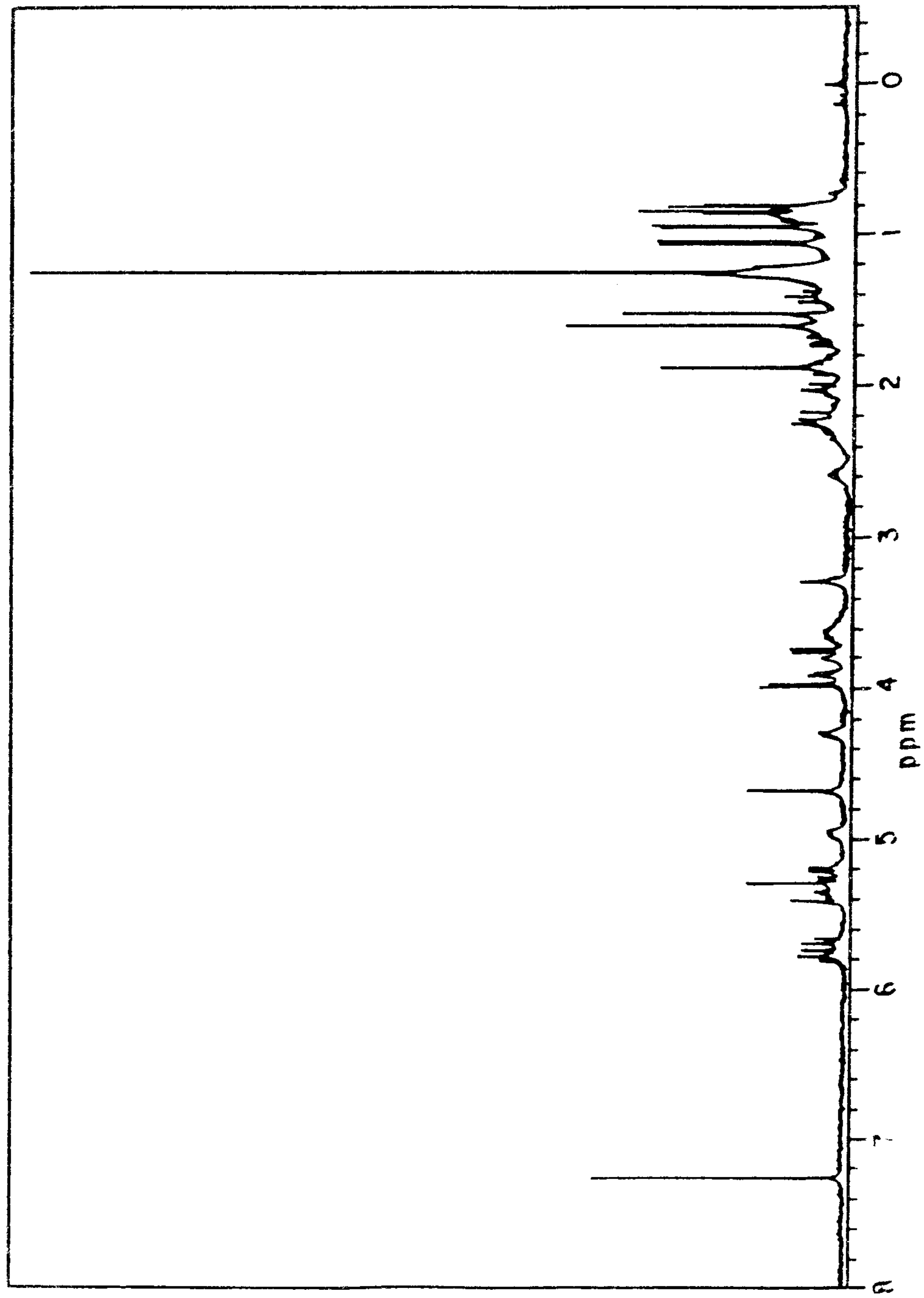


FIGURE 36

ELECTRON IMPACT MASS SPECTRUM OF LL-F28249ω

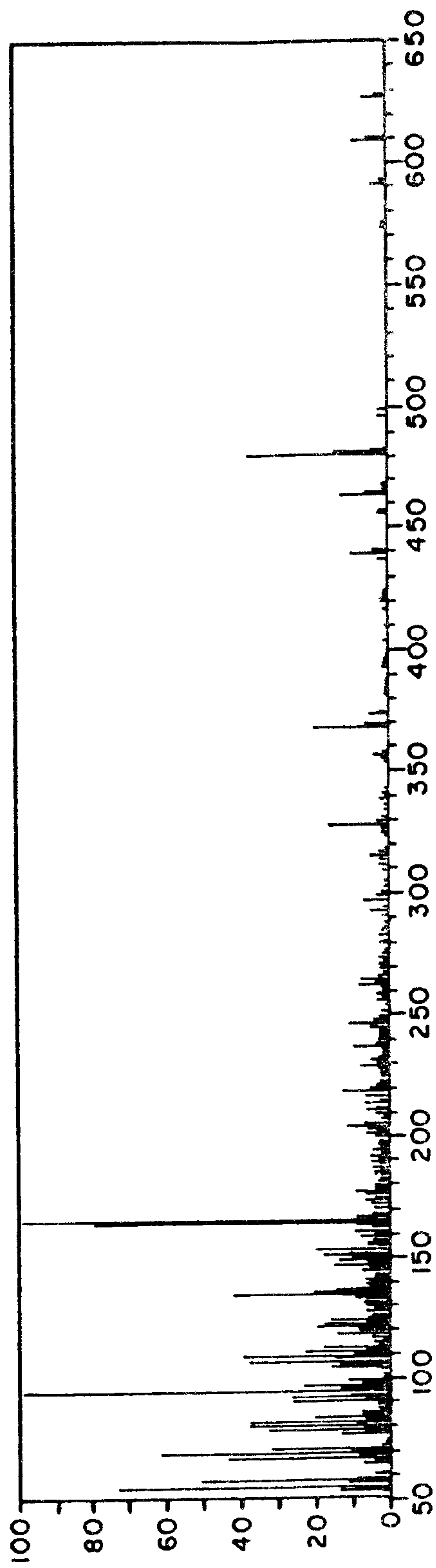


FIGURE 37

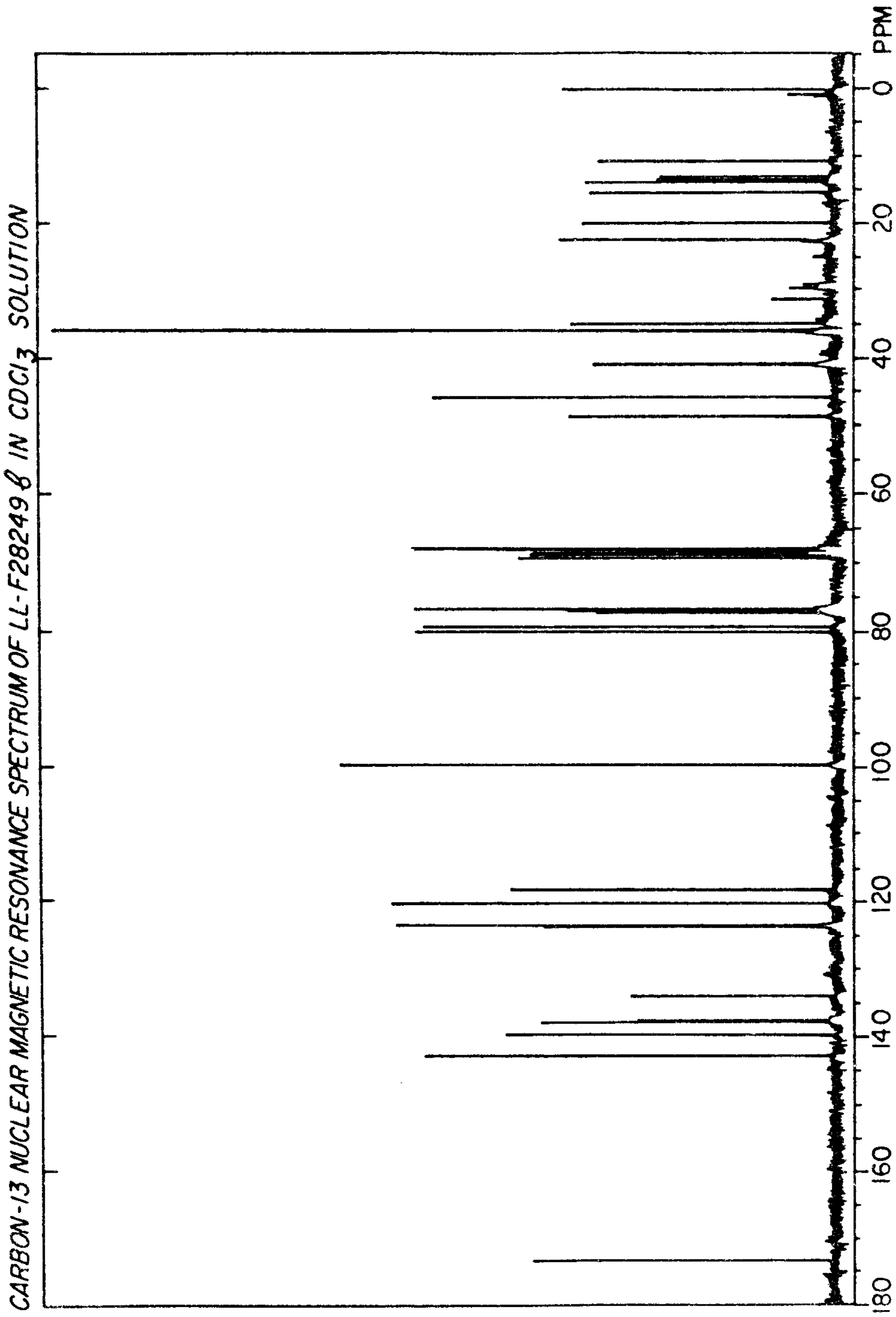
CARBON-13 NUCLEAR MAGNETIC RESONANCE SPECTRUM OF LL-F28249 δ IN CDCl3 SOLUTION
PPM
0
20
40
60
80
100
120
140
160
180

FIGURE 38

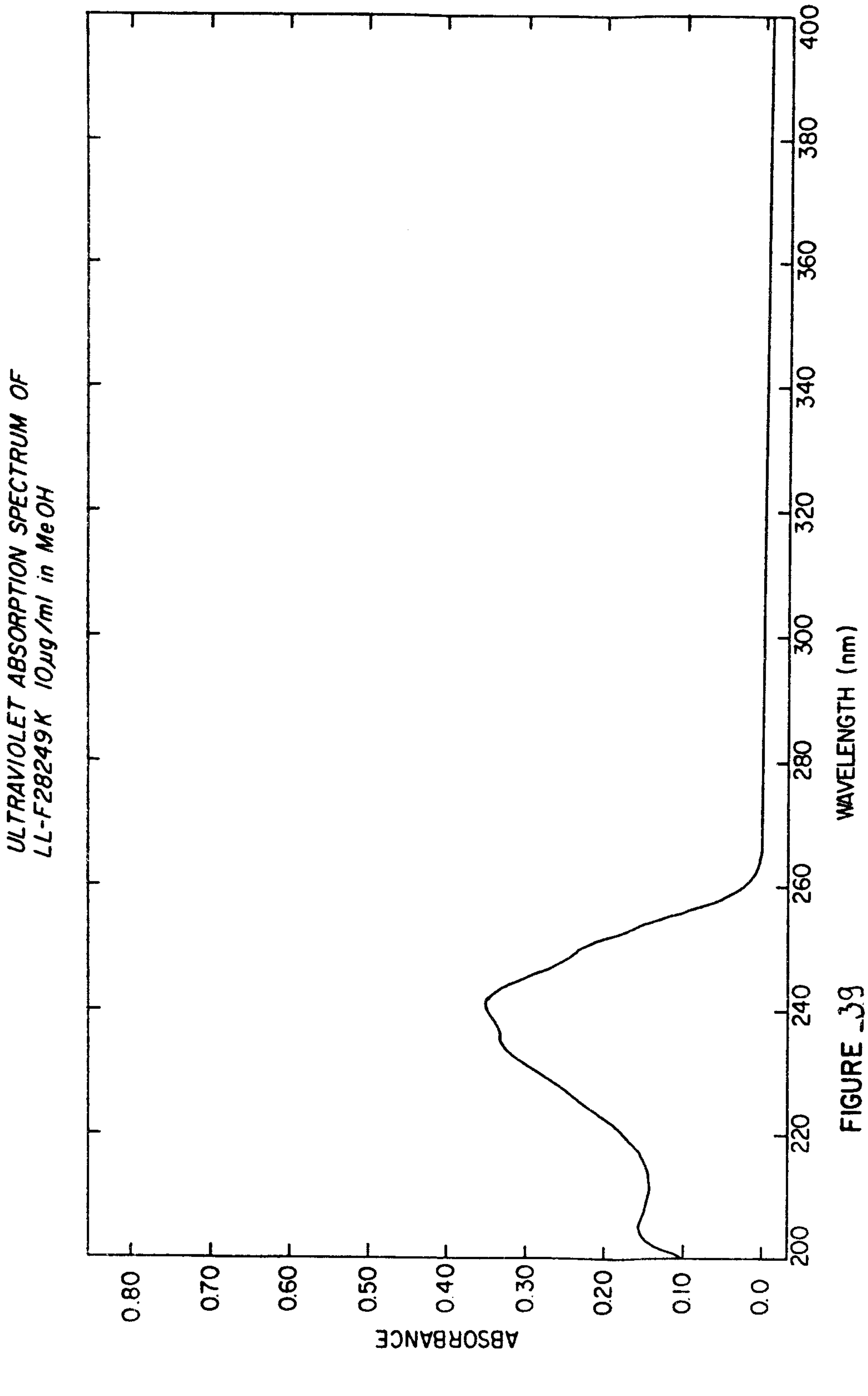
ULTRAVIOLET ABSORPTION SPECTRUM OF
LL-F28249K 10μg/ml in MeOH
ABSORBANCE
0.80
0.70
0.60
0.50
0.40
0.30
0.20
0.10
0.0
200
220
240
260
280
300
320
340
360
380
400
WAVELENGTH (nm)

FIGURE 39

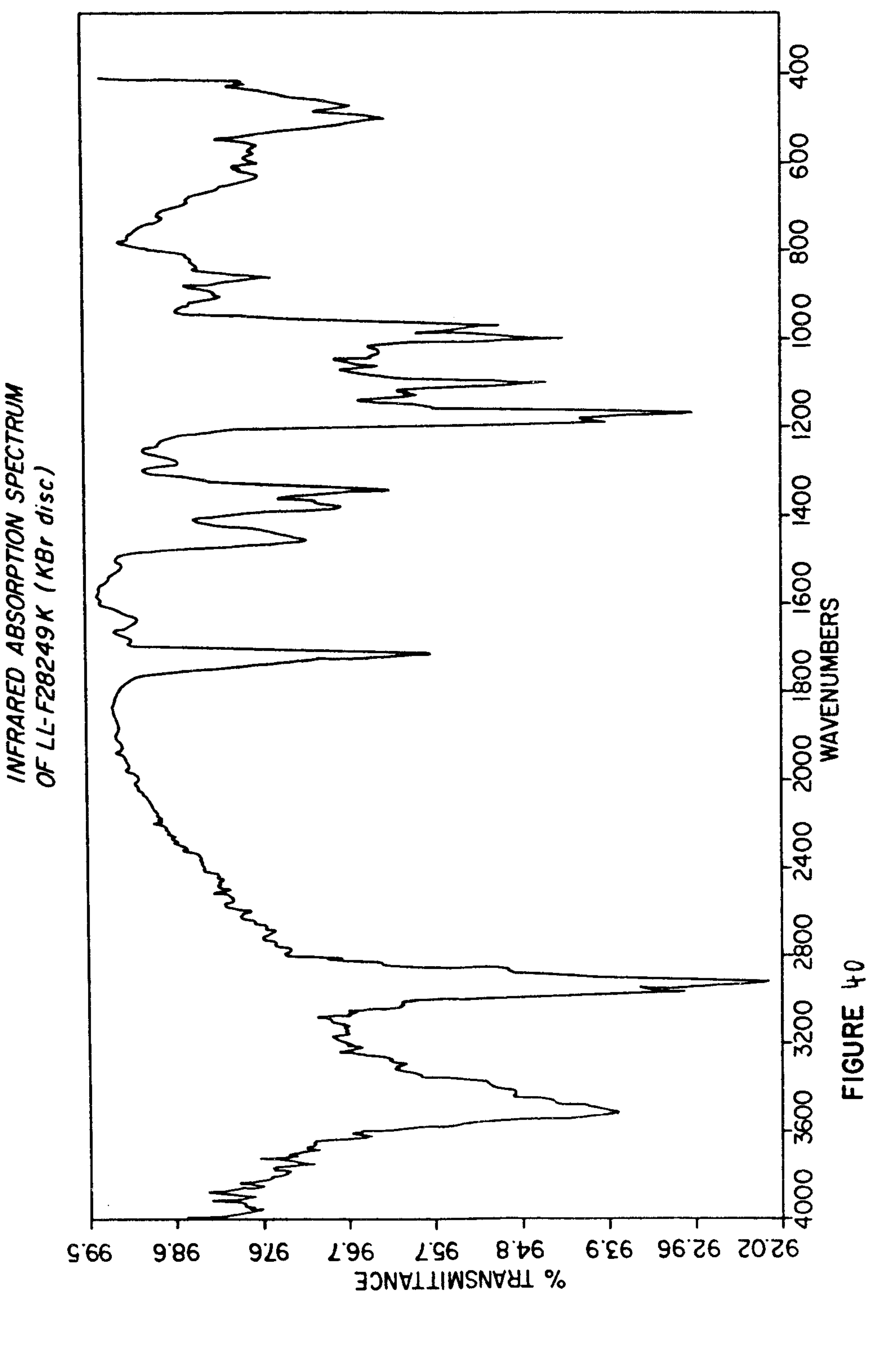
INFRARED ABSORPTION SPECTRUM
OF LL-F28249K (KBr disc)
% TRANSMITTANCE
99.5
98.6
97.6
96.7
95.7
94.8
93.9
92.96
92.02
WAVENUMBERS
4000
3600
3200
2800
2400
2000
1800
1600
1400
1200
1000
800
600
400

FIGURE 40

ELECTRON IMPACT MASS SPECTRUM OF
LL-F28249K

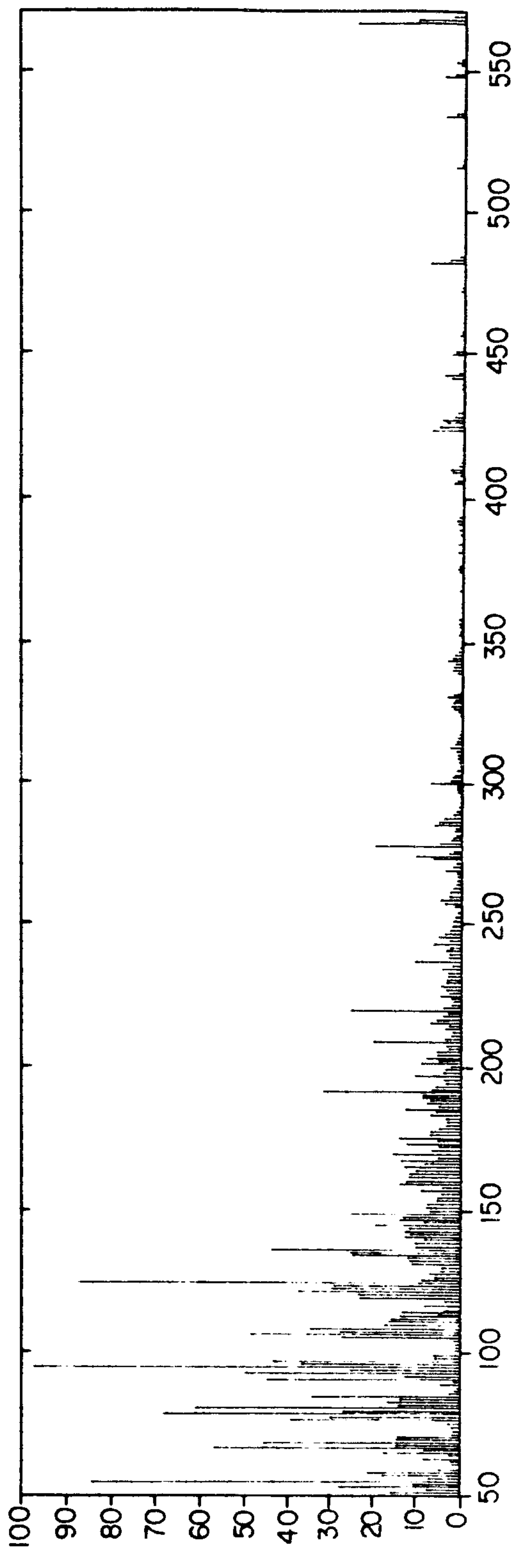


FIGURE 41

PROTON NUCLEAR MAGNETIC RESONANCE SPECTRUM
OF LL-F28249K IN $CDCl_3$ SOLUTION

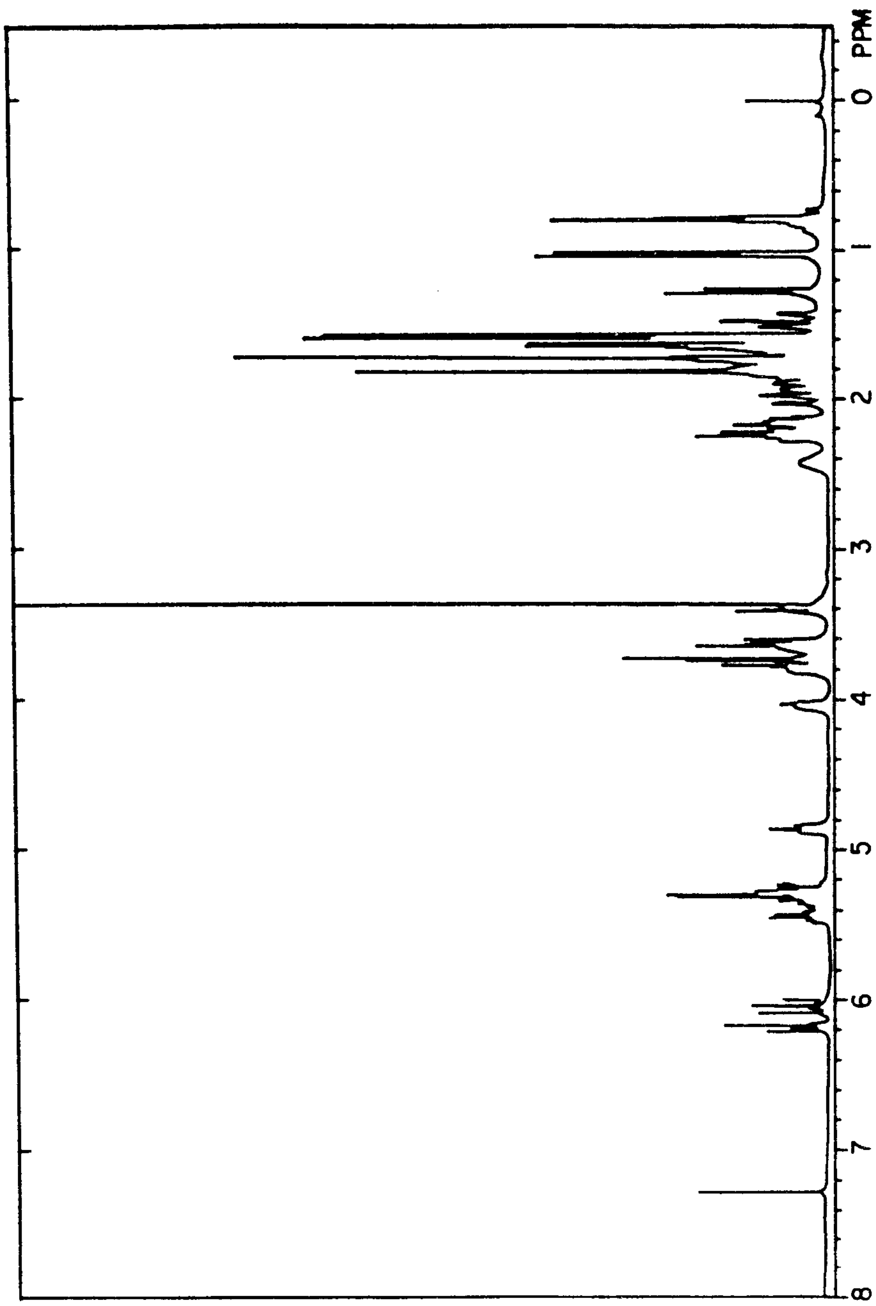


FIGURE 42

CARBON-13 NUCLEAR MAGNETIC RESONANCE SPECTRUM
OF LL-F28249K IN $CDCl_3$ SOLUTION

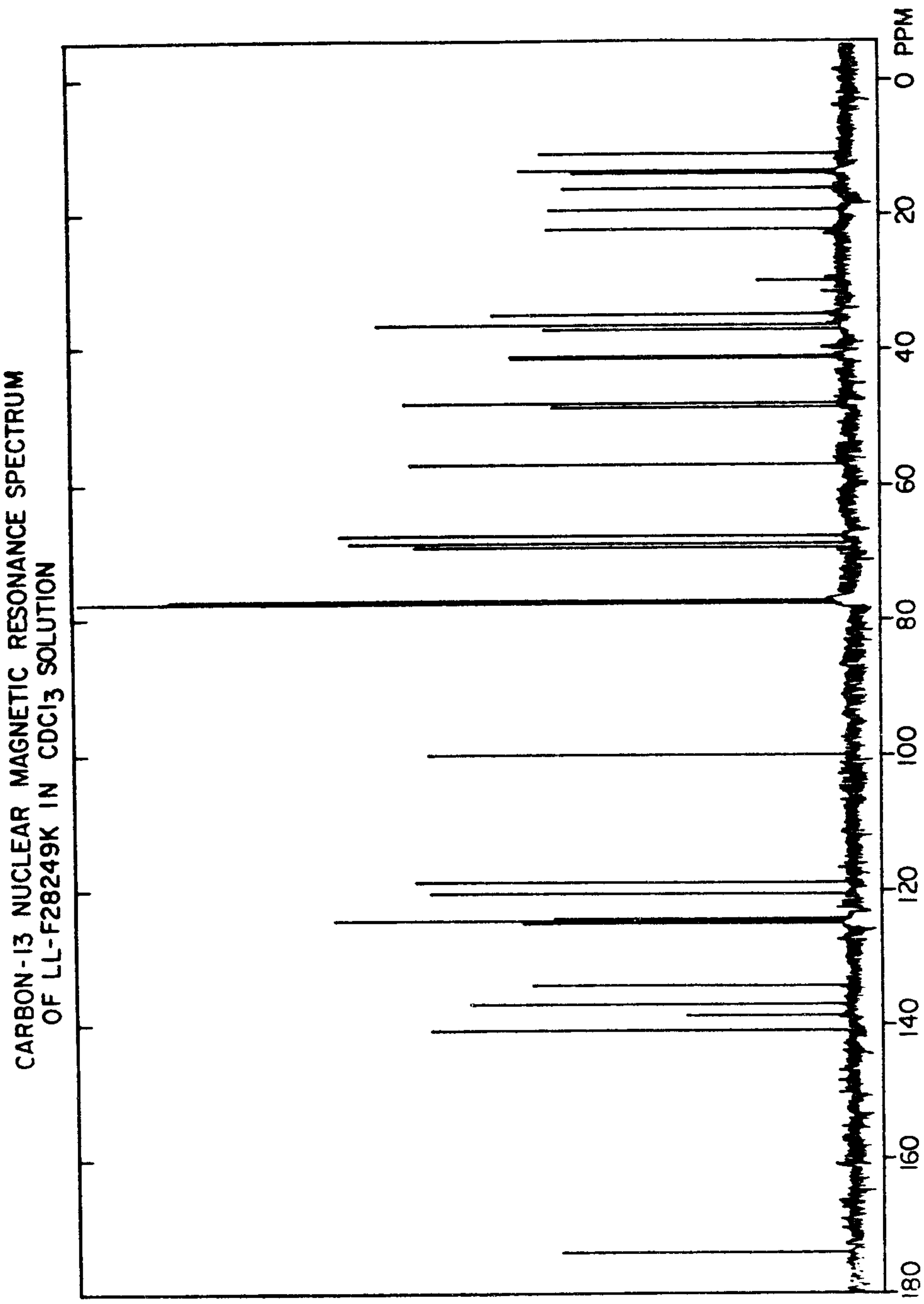


FIGURE 43

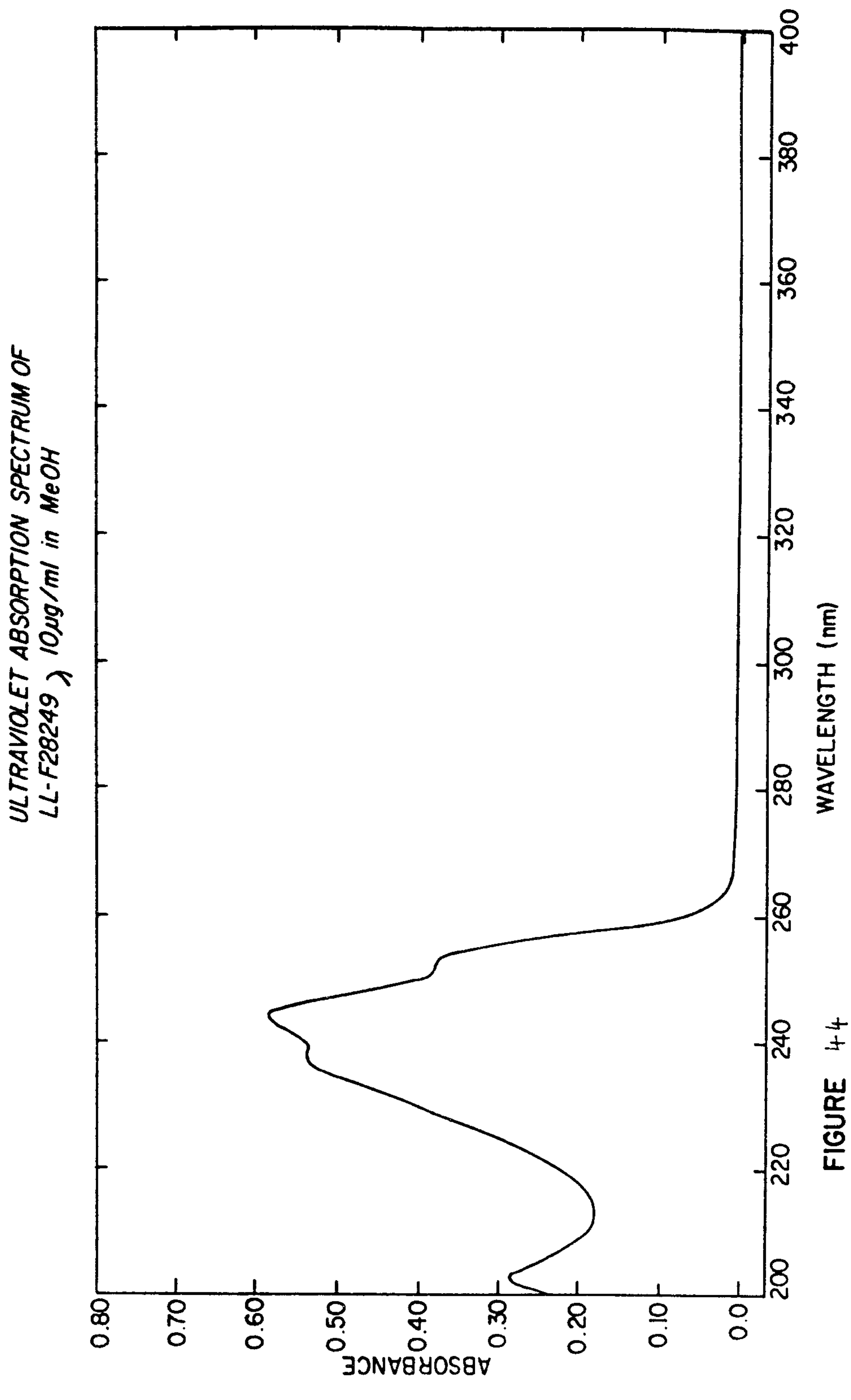
ULTRAVIOLET ABSORPTION SPECTRUM OF
LL-F28249 λ 10μg/ml in MeOH
ABSORBANCE
0.80
0.70
0.60
0.50
0.40
0.30
0.20
0.10
0.0
200
220
240
260
280
300
320
340
360
380
400
WAVELENGTH (nm)

FIGURE 44

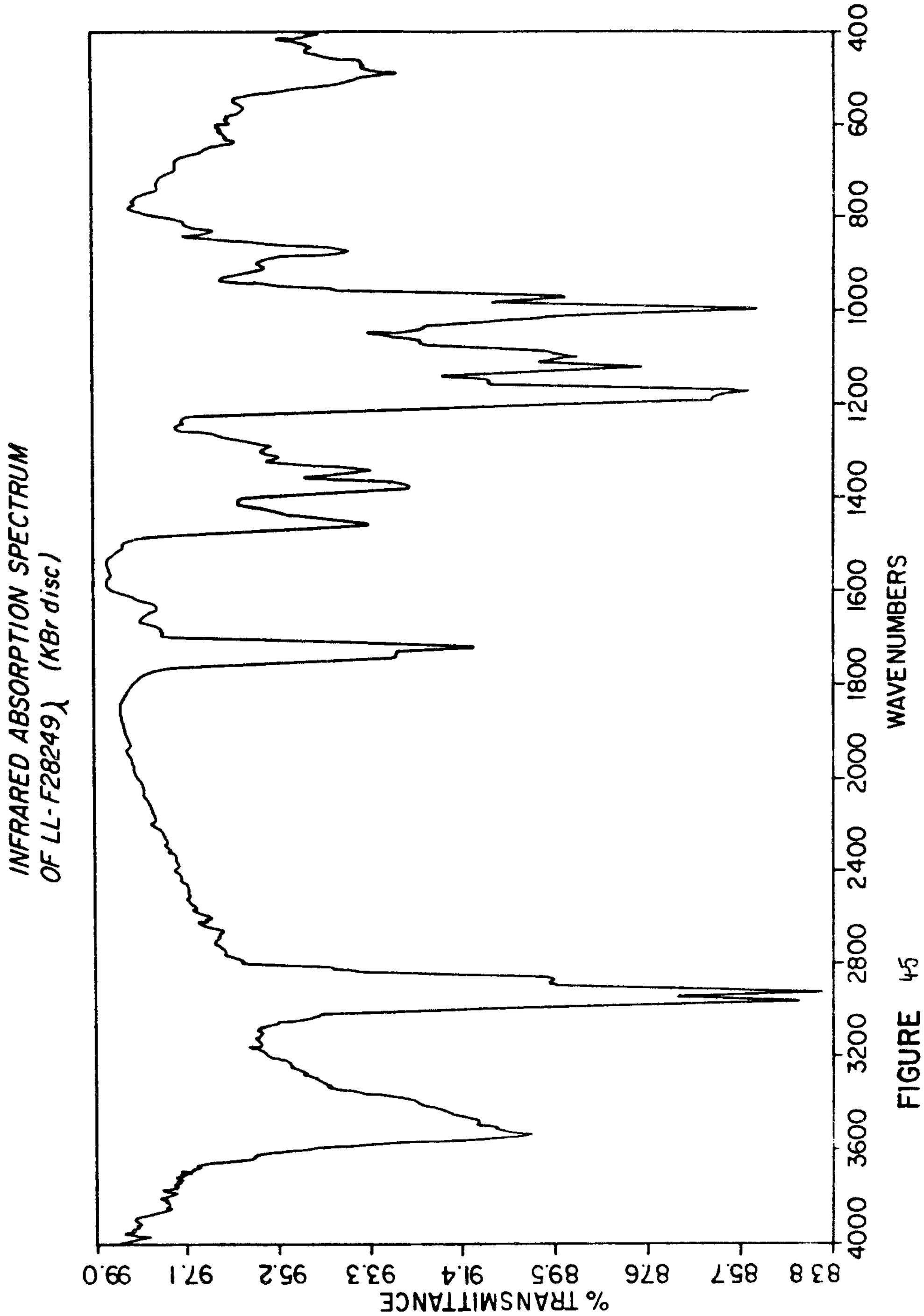
INFRARED ABSORPTION SPECTRUM
OF LL-F28249λ (KBr disc)
% TRANSMITTANCE
99.0 97.1 95.2 93.3 91.4 89.5 87.6 85.7 83.8
WAVENUMBERS
4000 3600 3200 2800 2400 2000 1800 1600 1400 1200 1000 800 600 400

FIGURE 45

ELECTRON IMPACT MASS SPECTRUM
OF LL-F28249 λ

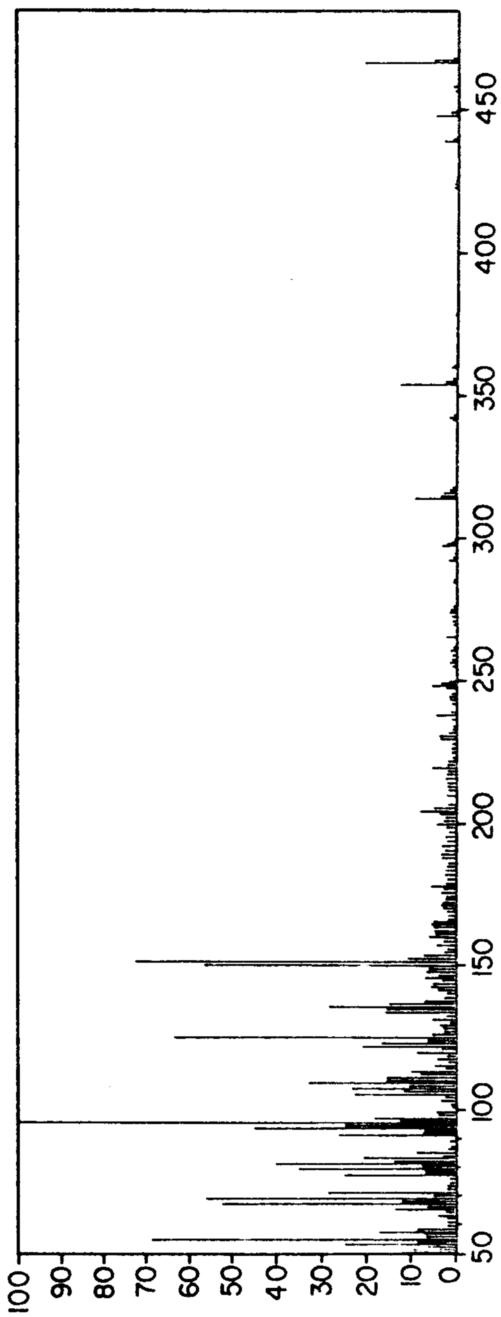


FIGURE 46

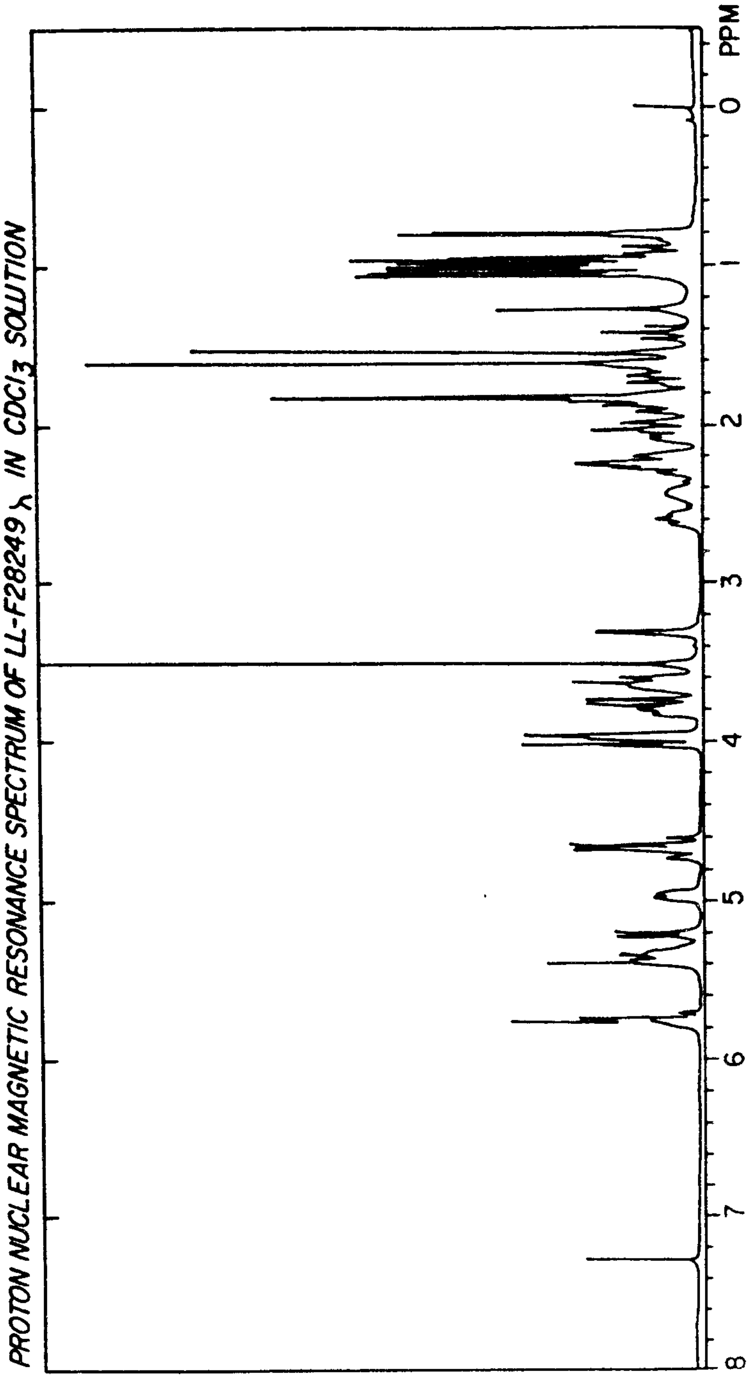


PROTON NUCLEAR MAGNETIC RESONANCE SPECTRUM OF LL-F28249λ IN $CDCl_3$ SOLUTION

FIGURE 47

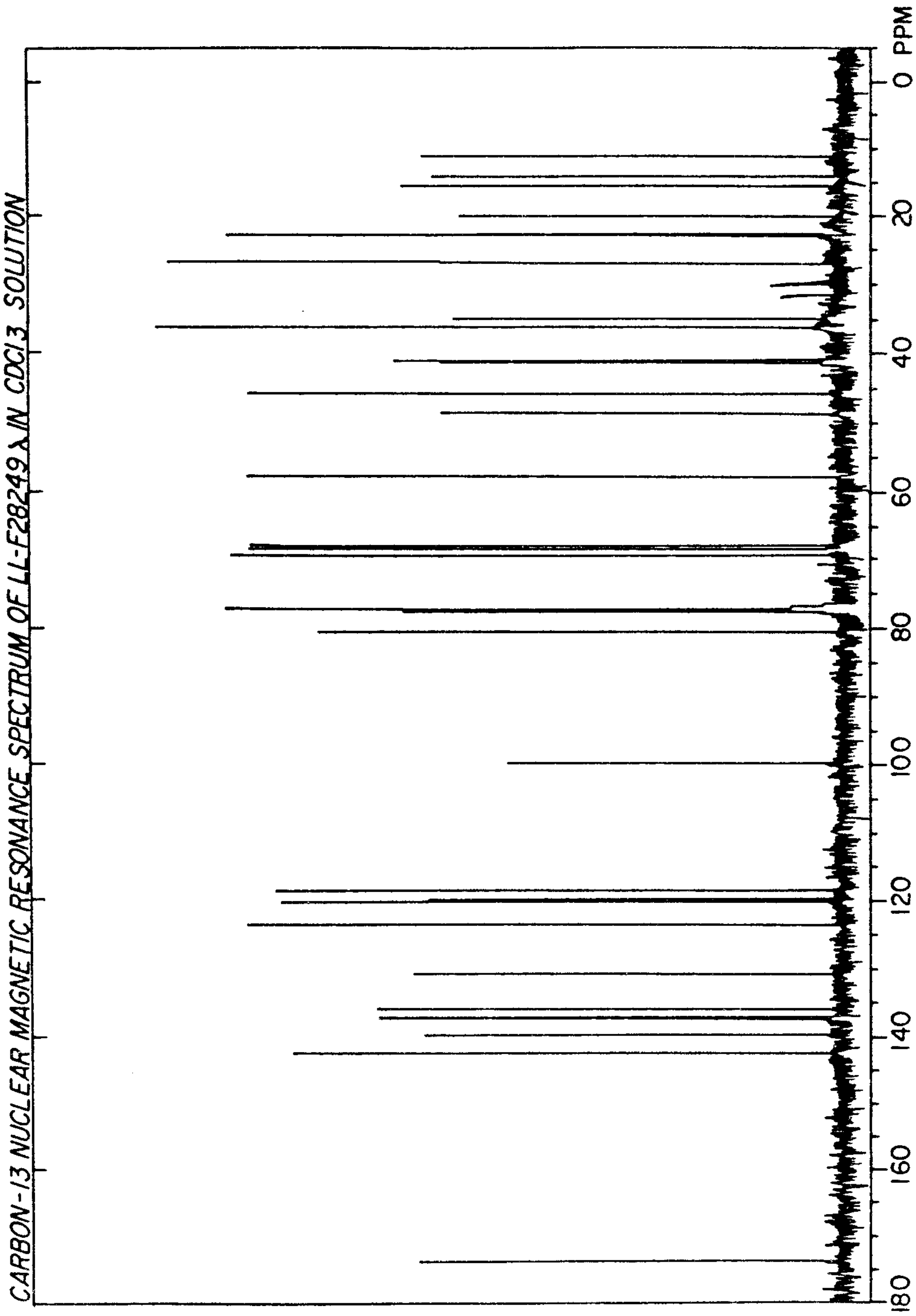
CARBON-13 NUCLEAR MAGNETIC RESONANCE SPECTRUM OF LL-F28249 λ IN CDCl3 SOLUTION
180
160
140
120
100
80
60
40
20
0 PPM

FIGURE 48

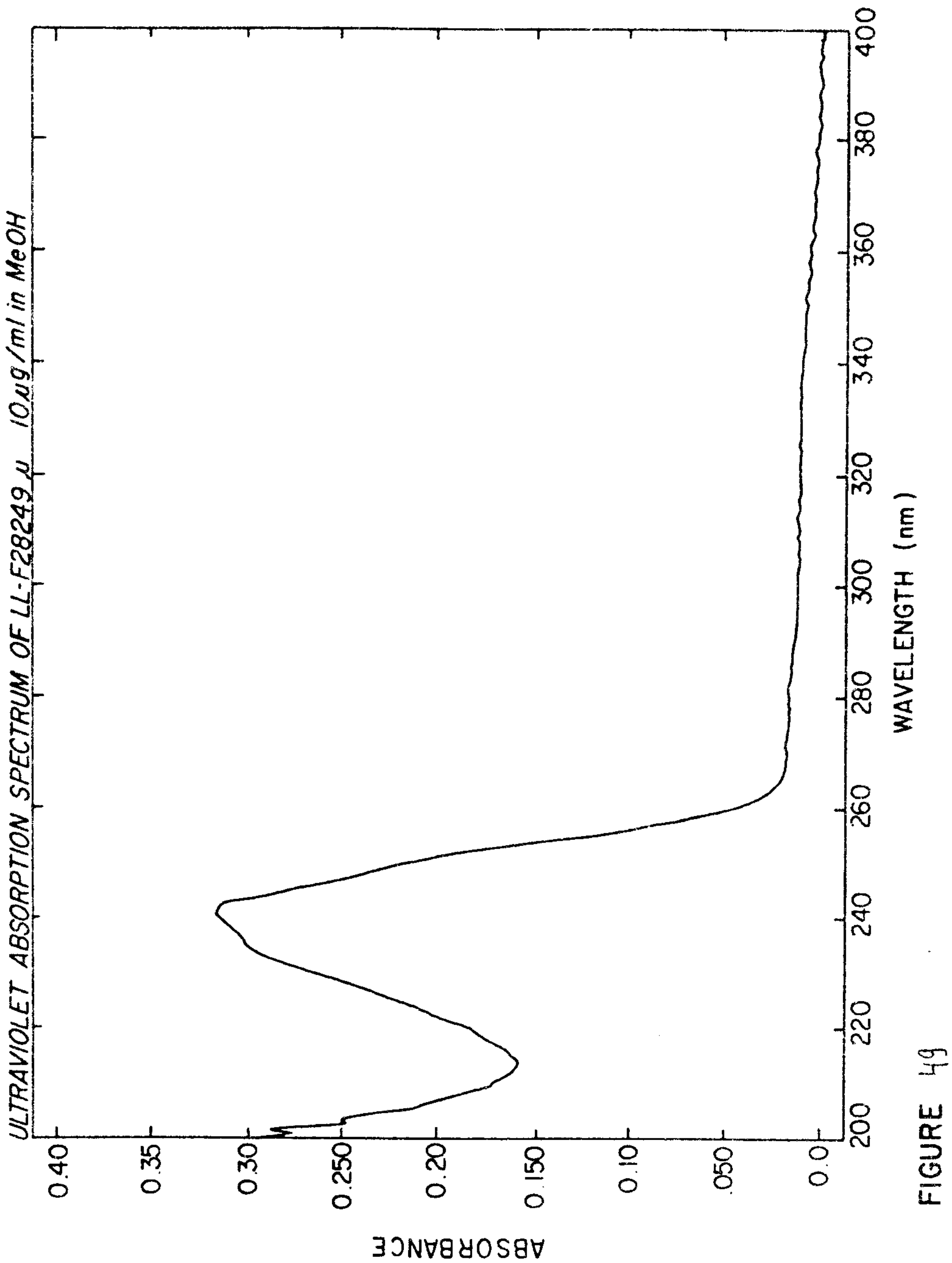
ULTRAVIOLET ABSORPTION SPECTRUM OF LL-F28249 μ 10μg/ml in MeOH
ABSORBANCE
0.40
0.35
0.30
0.250
0.20
0.150
0.10
.050
0.0
WAVELENGTH (nm)
200
220
240
260
280
300
320
340
360
380
400

FIGURE 49

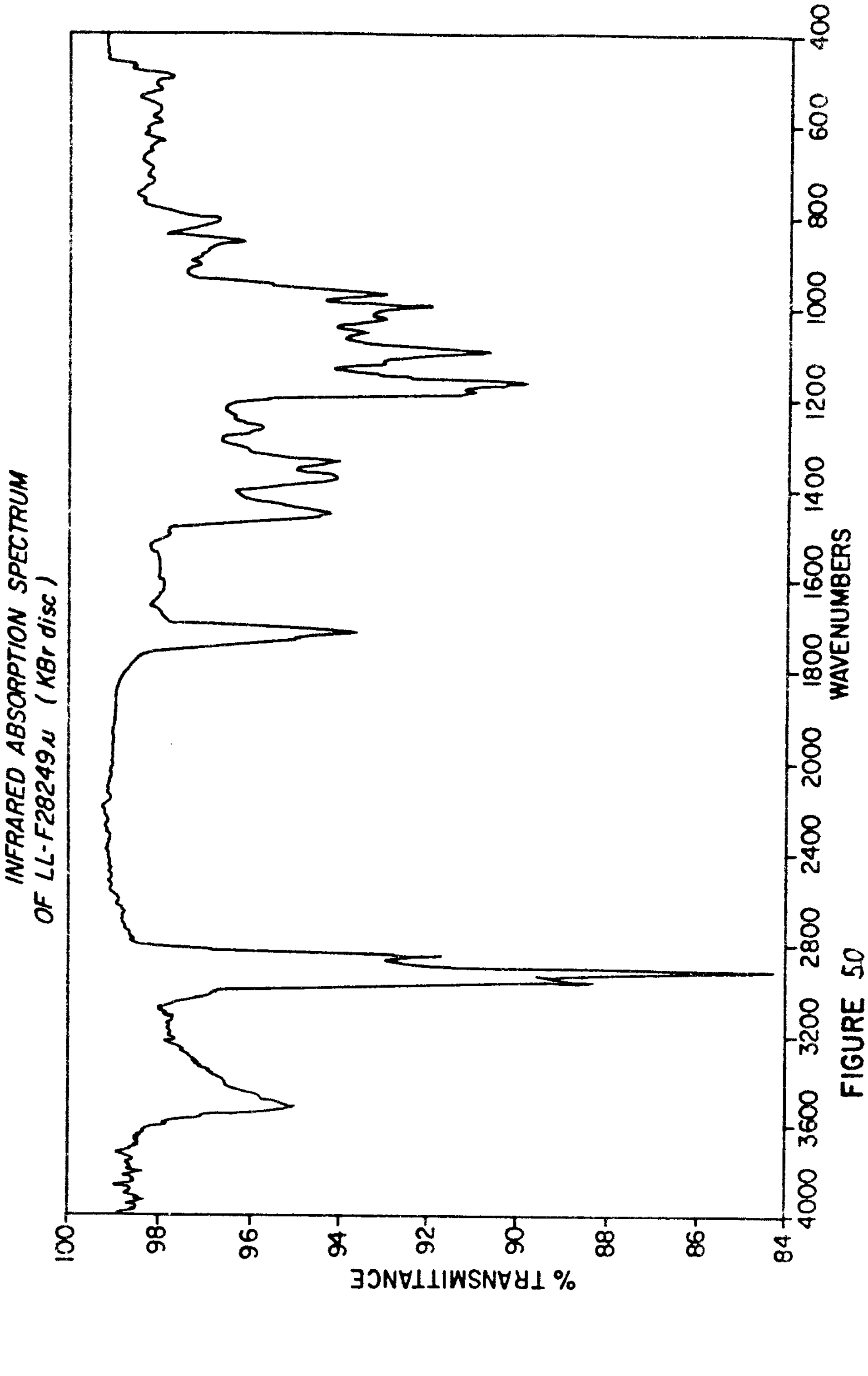
INFRARED ABSORPTION SPECTRUM
OF LL-F28249ν ( KBr disc )
% TRANSMITTANCE
100
98
96
94
92
90
88
86
84
4000
3600
3200
2800
2400
2000
1800
1600
1400
1200
1000
800
600
400
WAVENUMBERS

FIGURE 50

ELECTRON IMPACT MASS SPECTRUM
OF LL-F28249 μ

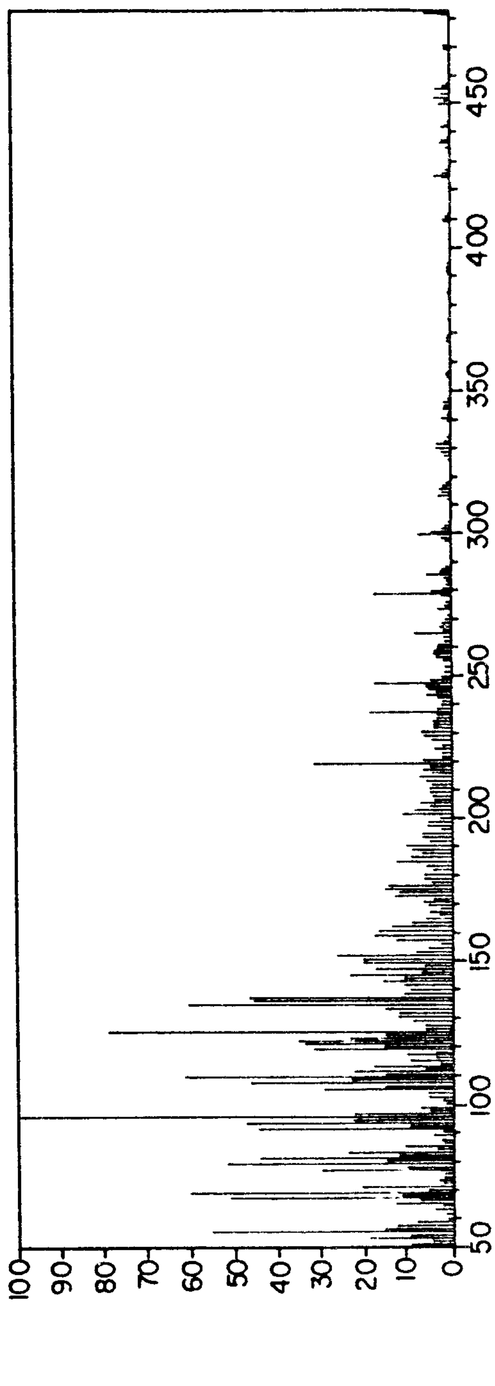


FIGURE 51

PROTON NUCLEAR MAGNETIC RESONANCE SPECTRUM
OF LL-F28249μ IN $CDCl_3$ SOLUTION

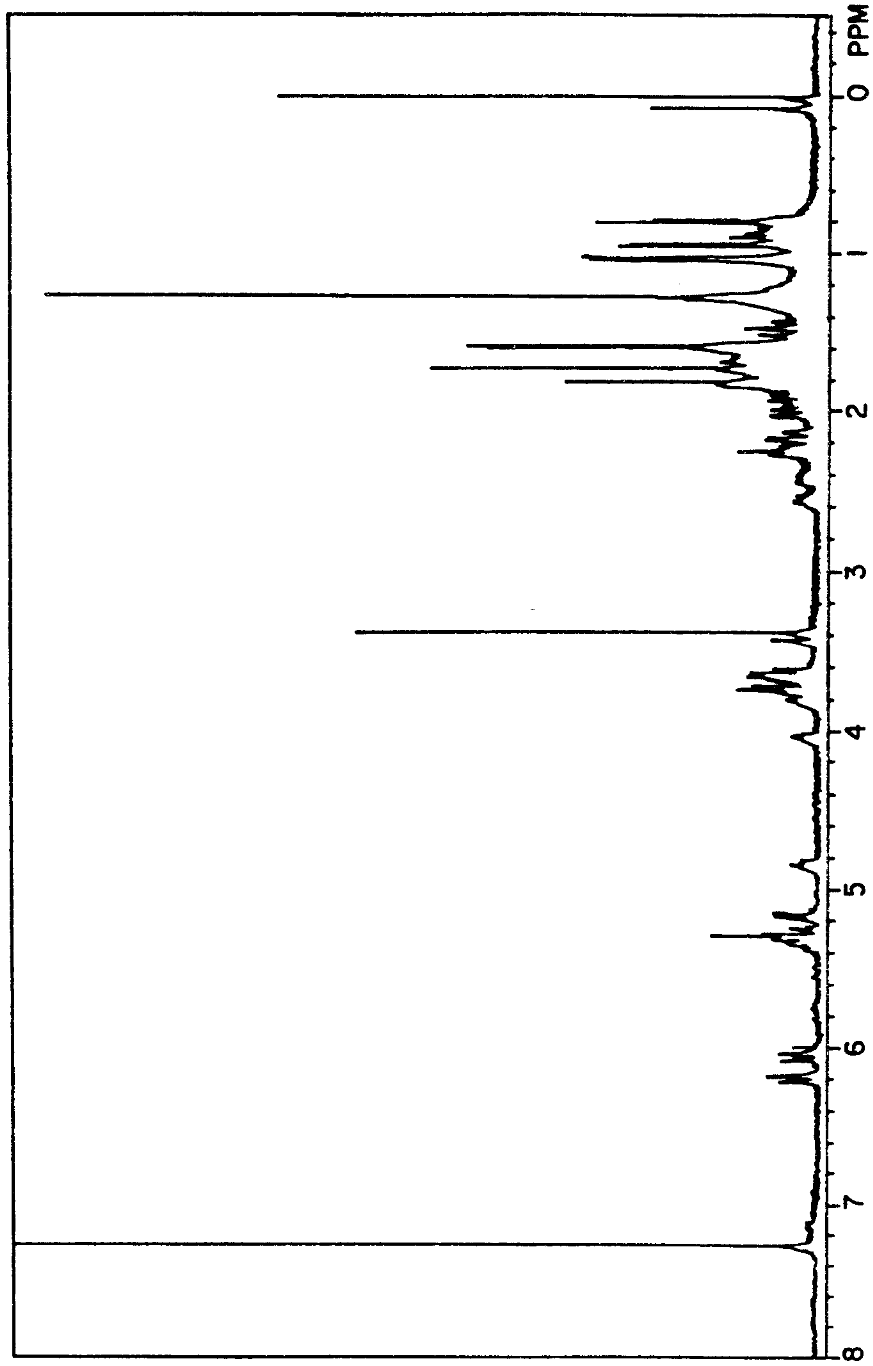


FIGURE 52

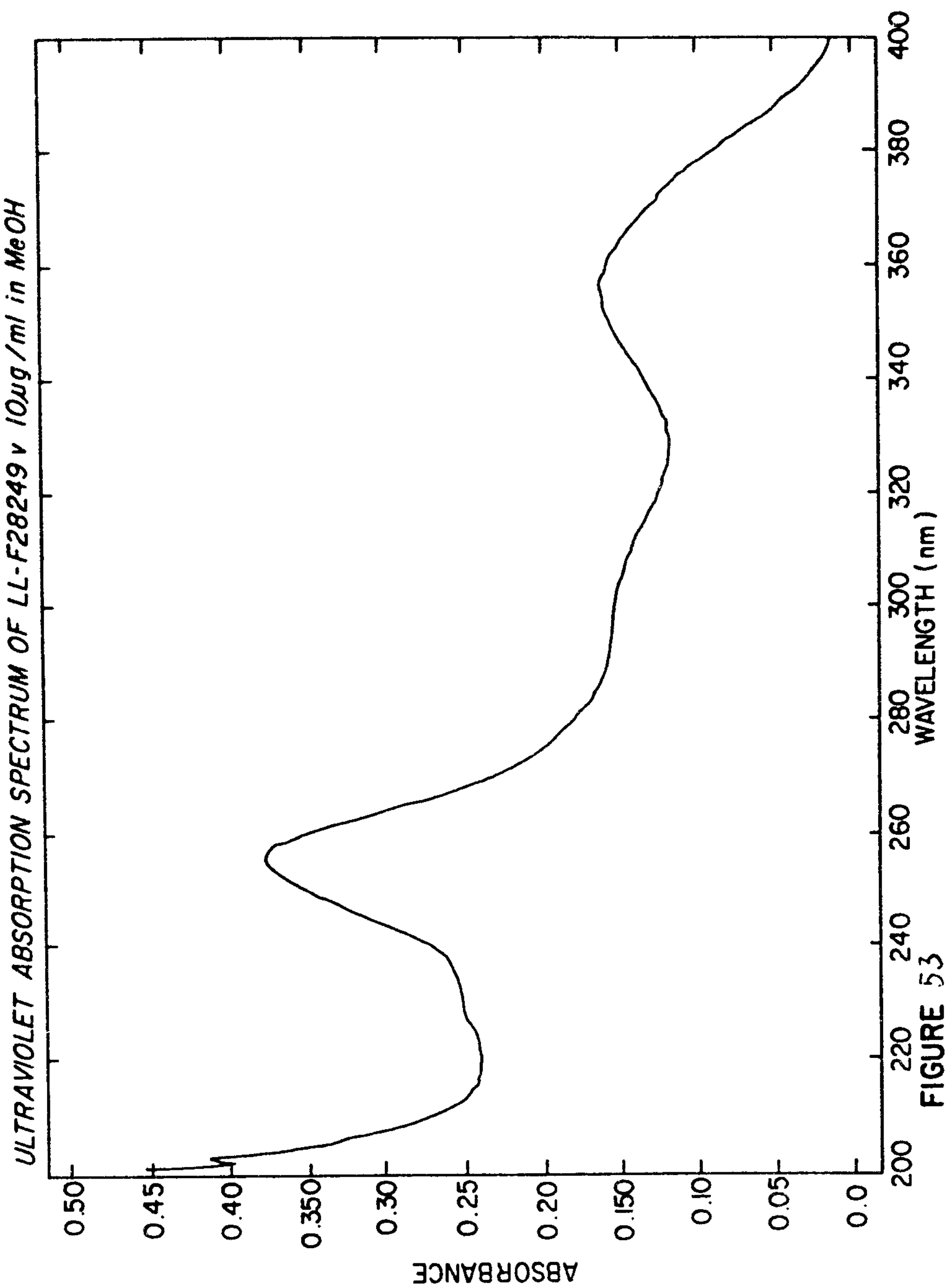

ULTRAVIOLET ABSORPTION SPECTRUM OF LL-F28249 ν 10μg/ml in MeOH
ABSORBANCE
0.50
0.45
0.40
0.350
0.30
0.25
0.20
0.150
0.10
0.05
0.0
200
220
240
260
280
300
320
340
360
380
400
WAVELENGTH (nm)


FIGURE 53

INFRARED ABSORPTION SPECTRUM
OF LL-F28249 ν (KBr disc)

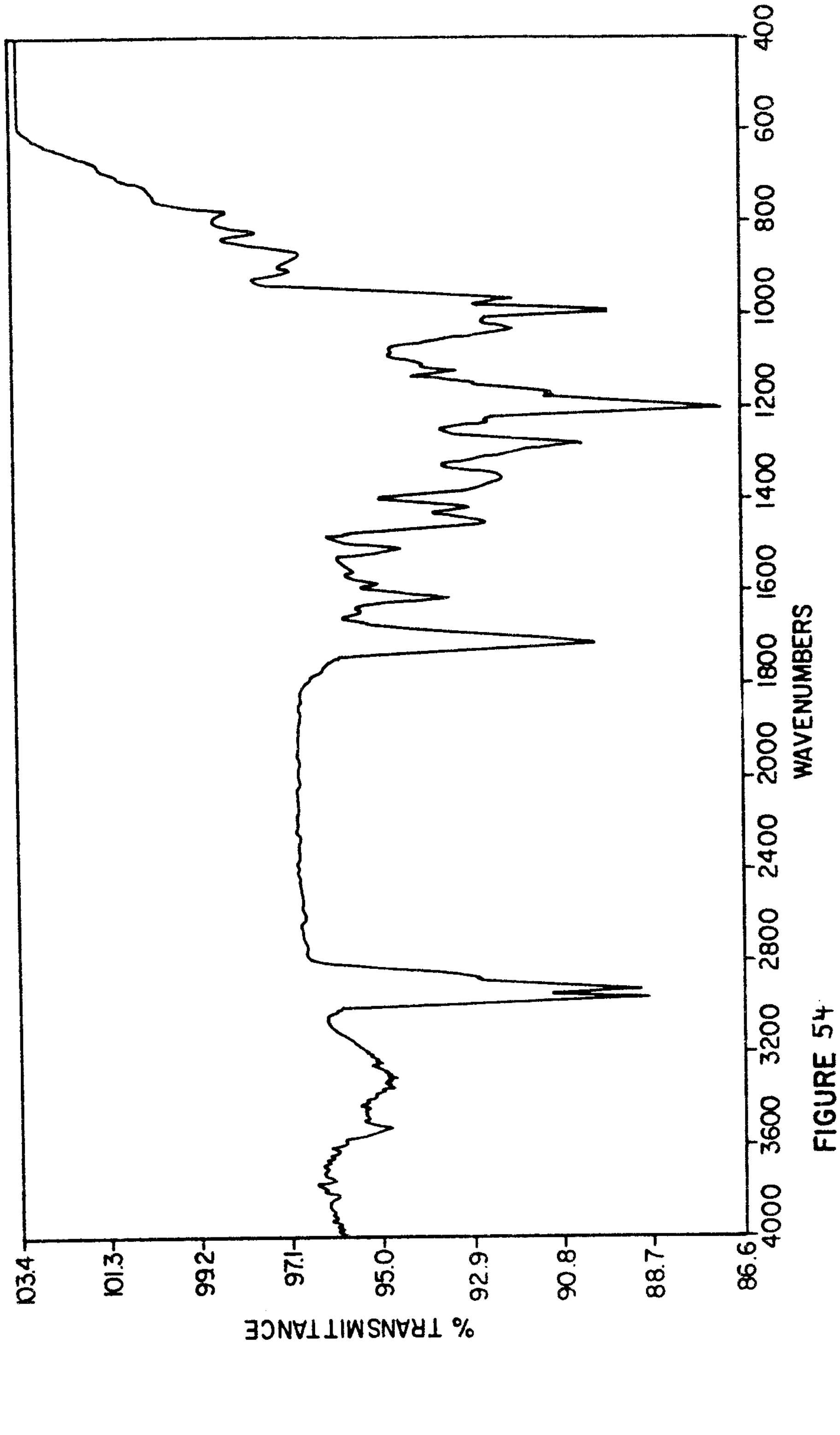


FIGURE 54

ELECTRON IMPACT MASS SPECTRUM
OF LL-F28249 ν

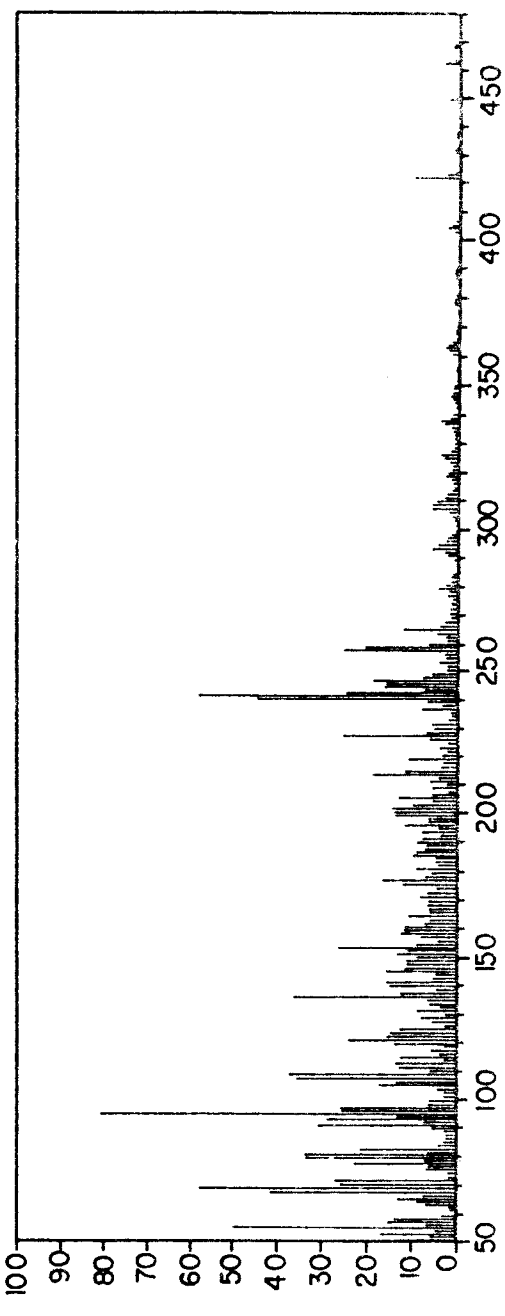


FIGURE 55

PROTON NUCLEAR MAGNETIC RESONANCE SPECTRUM
OF LL-F28249ν IN $CDCl_3$ SOLUTION

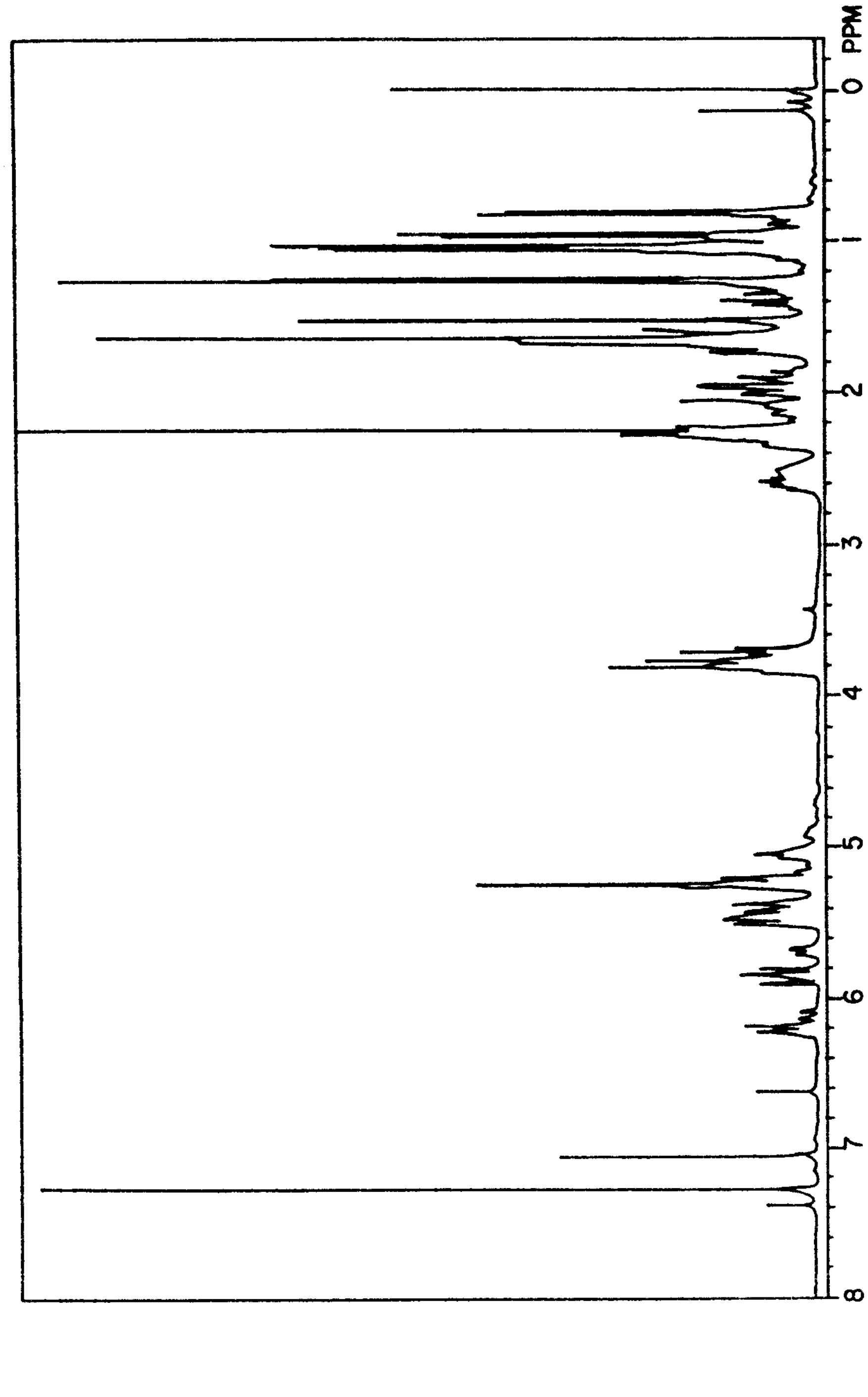


FIGURE 56

CARBON-13 NUCLEAR MAGNETIC RESONANCE SPECTRUM
OF LL-F28249ν IN $CDCl_3$ SOLUTION

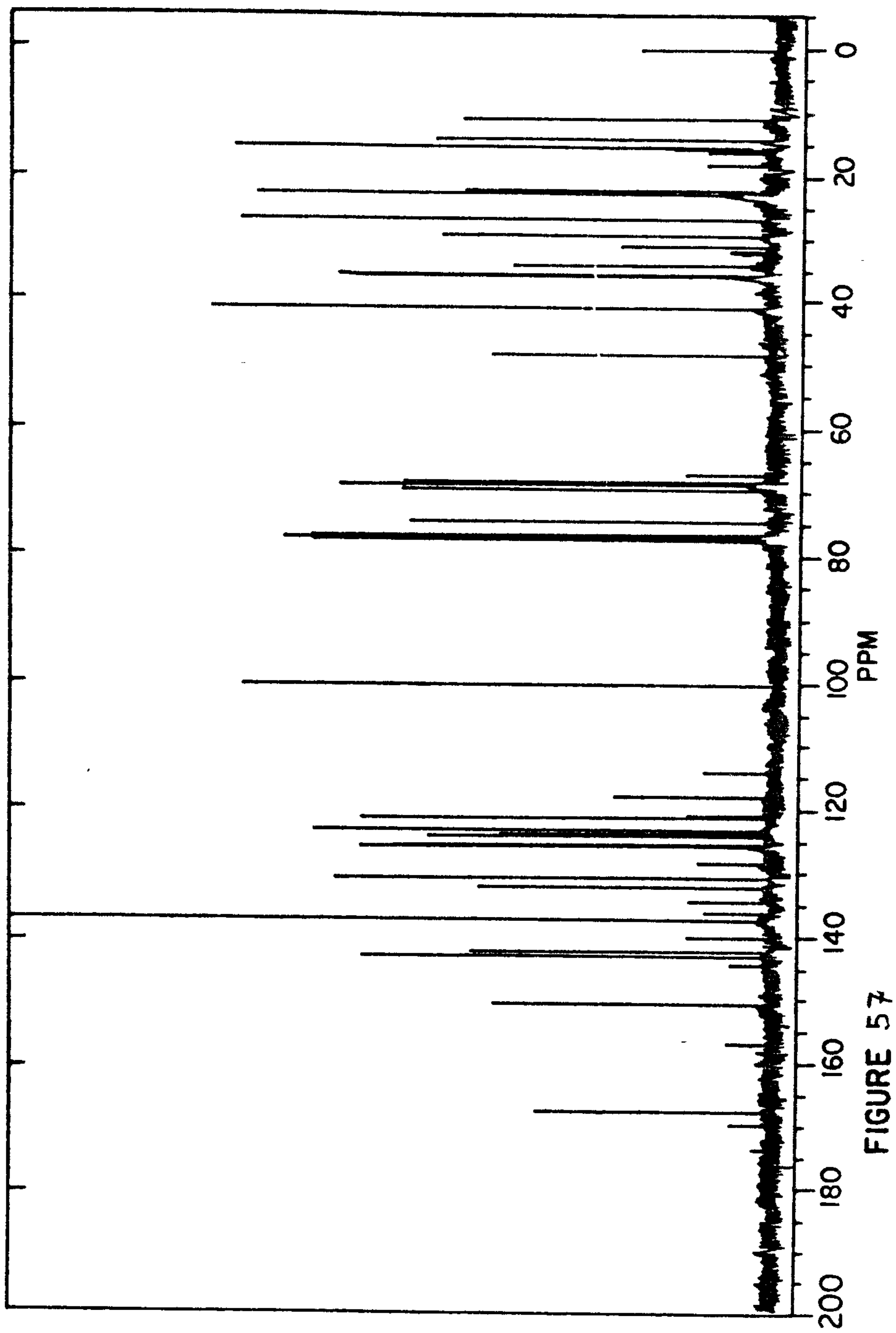


FIGURE 57